(19) Europäisches Patentamt
European Patent Office
Office européen des brevets

(11) Publication number: **0 232 912 B1**

# (12) EUROPEAN PATENT SPECIFICATION

(45) Date of publication of patent specification: **13.05.92** (51) Int. Cl.[5]: **C07D 493/08**, A61K 31/34

(21) Application number: **87101942.8**

(22) Date of filing: **12.02.87**

## (54) Hydroxamic acids of 7-oxabicycloheptane substituted ethers.

(30) Priority: **14.02.86 US 829257**
**14.02.86 US 829639**

(43) Date of publication of application:
**19.08.87 Bulletin 87/34**

(45) Publication of the grant of the patent:
**13.05.92 Bulletin 92/20**

(84) Designated Contracting States:
**BE CH DE ES FR GB GR IT LI LU NL SE**

(56) References cited:
**US-A- 4 550 120**
**US-A- 4 555 523**
**US-A- 4 607 049**

(73) Proprietor: **E.R. Squibb & Sons, Inc.**
**Lawrenceville-Princeton Road**
**Princeton, N.J. 08540-4000(US)**

(72) Inventor: **Varma, Ravi Kannadikovilakom**
**293 Deer Run Drive**
**Belle Mead, N.J.(US)**
Inventor: **Das, Jagabandhu**
**47 Joni Avenue**
**Hamilton Square, N.J.(US)**

(74) Representative: **Vossius & Partner**
**Siebertstrasse 4 P.O. Box 86 07 67**
**W-8000 München 86(DE)**

**EP 0 232 912 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

In US-A-4 550 120 and 4 555 523 are described 7-oxabicycloheptane substituted ether and thio prostaglandin analogs which are cardiovascular agents.

The present invention relates to hydroxamic acids of 7-oxabicycloheptane substituted ethers which are inhibitors of $\Delta^5$-lipoxygenase and inhibitors of prostaglandin and leukotriene biosynthesis and as such are useful, for example, as anti-allergy and antiinflammatory agents and also as antipsoriatic agents. These compounds have the general formula I

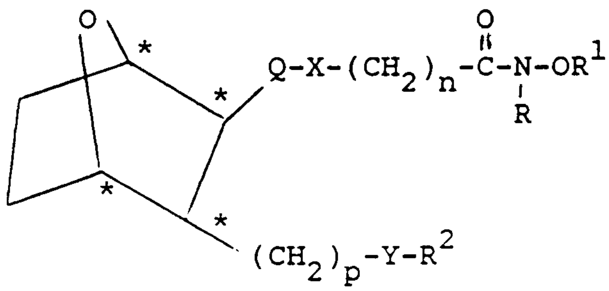

(I)

and including all stereoisomers thereof, wherein

Q is $(CH_2)_t$ wherein t is 1 or 2, or $CH_2$-A-$(CH_2)_m$ wherein A is -CH=CH- or -$(CH_2)_2$, m is 1 to 6 wherein A is -CH=CH- and m is 0 to 6 wherein A is $(CH_2)_2$; X is O or $S(O)_q$, wherein q' is 0, 1 or 2; n is 1 to 8; R is H, lower alkyl, aryl, aralkyl or cycloalkyl; $R^1$ is H, lower alkyl, aryl, aralkyl, cycloalkyl, alkanoyl or aroyl; p is 1 to 5; Y is O or $S(O)_q$ wherein q is 0, 1 or 2, and wherein q is 0, 1 or 2 where X is O (oxygen), and q is 0 (zero) when X is $S(O)_{q'}$; and $R^2$ is lower alkyl, aryl, aralkyl, cycloalkyl, cycloalkylalkyl, lower alkenyl or lower alkynyl containing 2 to 12 carbons, wherein lower alkyl or alkyl alone or as part of another group contains 1 to 12 carbons and is unsubstituted or is substituted with halo, $CF_3$, alkoxy, aryl, alkyl-aryl, haloaryl, cycloalkyl, or alkylcycloalkyl;

aryl alone or as part of another group is phenyl or naphthyl which is unsubstituted or is substituted with 1 or 2 lower alkyl groups, 1 or 2 halogens and/or 1 or 2 lower alkoxy groups;

cycloalkyl alone or as part of another group contains 3 to 12 carbons and is unsubstituted or is substituted with 1 or 2 halogens, 1 or 2 lower alkyl groups and/or 1 or 2 lower alkoxy groups; and

$(CH_2)_m$, $(CH_2)_n$ and $(CH_2)_p$ may independently contain 1 or 2 lower alkyl and/or halo substituents.

Thus, the compounds of the invention include the following types of compounds:

IA

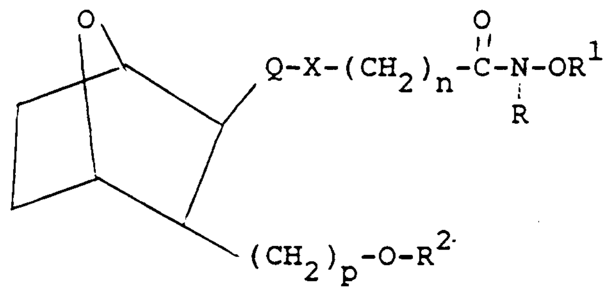

IB

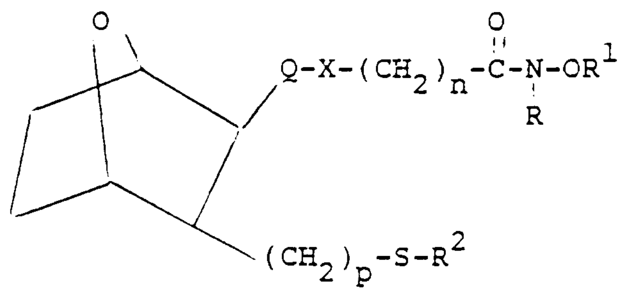

IC

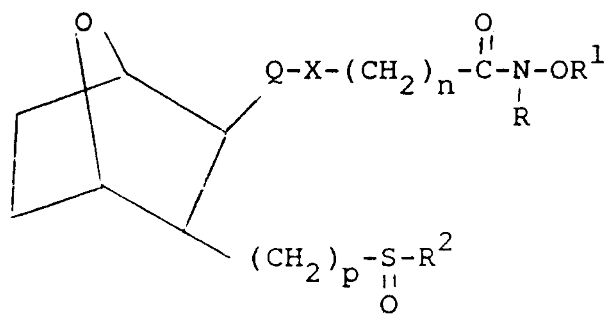

ID

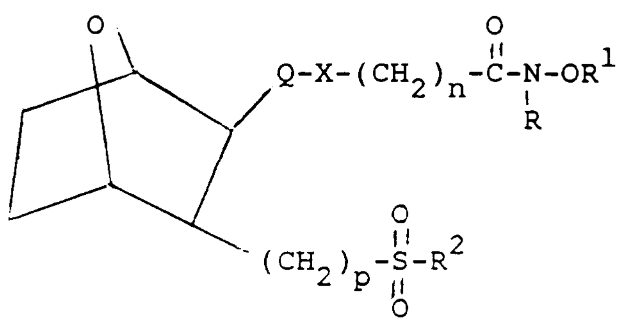

The term "lower alkyl" or "alkyl" as employed herein by itself or as part of another group includes both straight and branched chain radicals of up to 12 carbons, preferably 1 to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as

well as such groups including a halo-substituent, such as F, Br, Cl or I or $CF_3$, an alkoxy substituent, an aryl substituent, an alkyl-aryl substituent, a haloaryl substituent, a cycloalkyl substituent or an alkylcycloalkyl substituent.

The term "cycloalkyl" by itself or as part of another group includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with 1 or 2 halogens, 1 or 2 lower alkyl groups and/or 1 or 2 lower alkoxy groups.

The term "aryl" or "Ar" as employed herein by itself or as part of another group refers to monocyclic or bicyclic aromatic groups containing from 6 to 10 carbons in the ring portion, such as phenyl, naphthyl, substituted phenyl or substituted naphthyl wherein the substituent on either the phenyl or naphthyl may be 1 or 2 lower alkyl groups, halogens (Cl, Br or F), and/or 1 or 2 lower alkoxy groups.

The term "aralkyl", "aryl-alkyl" or "aryl-lower alkyl" as used herein by itself or as part of another group refers to lower alkyl groups as discussed above having an aryl substituent, such as benzyl.

The term "lower alkenyl" as used herein by itself or as part of another group refers to straight or branched chain radicals of 2 to 12 carbons, preferably 2 to 6 carbons in the normal chain, which include one double bond in the normal chain, such as 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl and the like.

The term "lower alkynyl" as used herein by itself or as part of another group refers to straight or branched chain radicals of 2 to 12 carbons, preferably 2 to 6 carbons in the normal chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl, 3-undecynyl, 4-dodecynyl and the like.

The terms "alkanoyl" and "aroyl" refer to a lower alkyl group linked to a carbonyl group or an aryl group linked to a carbonyl group.

The term "halogen" or "halo" as used herein by itself or as part of another group refers to chlorine, bromine, fluorine or iodine, with chlorine being preferred.

The terms "$(CH_2)_t$", "$(CH_2)_m$", "$(CH_2)_n$" and "$(CH_2)_p$" include 1 or 2 carbons in the case of $(CH_2)_t$, a straight or branched chain radical having 1 to 8 carbons in the normal chain in the case of "$(CH_2)_n$", 1 to 6 carbons in the normal chain in the case of "$(CH_2)_m$", and 1 to 5 carbons in the normal chain in the case of "$(CH_2)_p$", and may contain one or more lower alkyl and/or halogen substituents. Examples of $(CH_2)_t$, $(CH_2)_m$, $(CH_2)_n$ and $(CH_2)_p$ groups include $CH_2$,

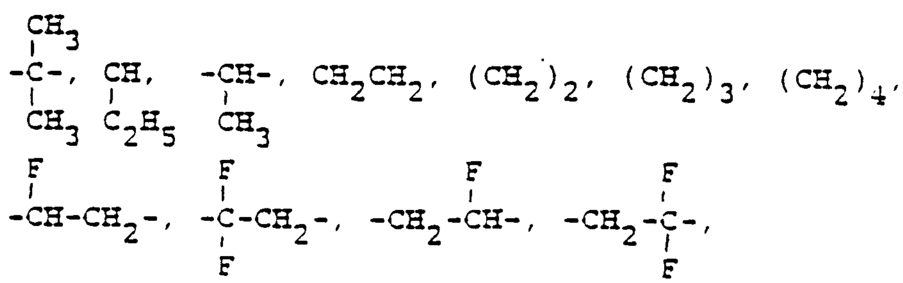

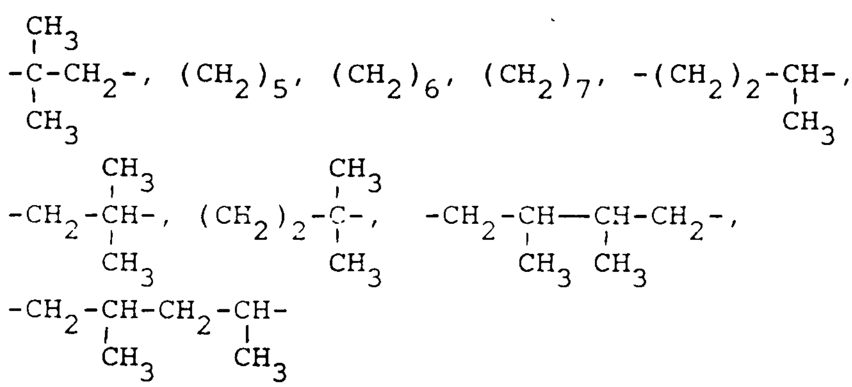

and the like.

Preferred are those compounds of formula I wherein Q is $-CH_2-A-(CH_2)_m-$, A is $-CH=CH-$ or $-CH_2-CH_2-$, m is 1 to 3, or Q is $(CH_2)_t$, X is O, n is 1 to 3, p is 1, Y is O or S, R is alkyl, $R^1$ is H, and $R^2$ is lower alkyl, such as hexyl, aryl, such as phenyl, or aralkyl such as benzyl.

The various compounds of the invention may be prepared as outlined below.

Compounds of the invention wherein Q is $-CH_2-CH=CH-(CH_2)_m$, m is 1 to 6, X is O, n is 1 to 4, p is 1 and Y is O may be prepared by reducing the mesoanhydride A

A

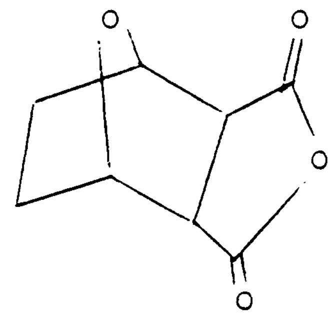

by treating A with lithium aluminum hydride in the presence of an inert solvent such as tetrahydrofuran to form the diol B

B

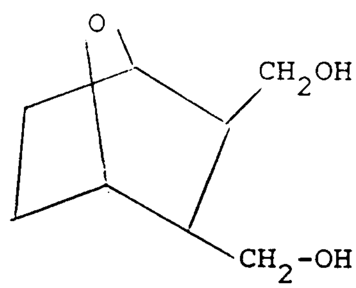

Diol B is then treated with a base such as sodium hydride in the presence of a solvent such as dimethylformamide and then is reacted with sulfonate reactants C, that is mesyl-$OR^2$ or C', that is, tosyl-$OR^2$ or halide C", that is $R^2Hal$ to form the ether alcohol II

II

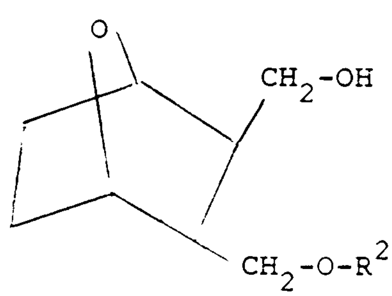

which is treated with tosyl chloride in the presence of methylene chloride, pyridine and lithium chloride to form the chloride III

III 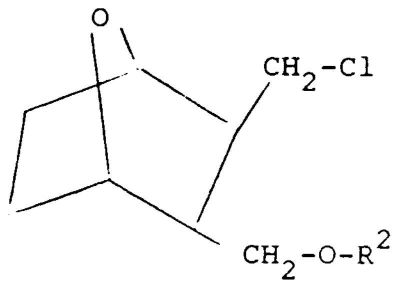

Chloride III is then treated with sodium cyanide in the presence of an inert solvent such as dimethyl sulfoxide at elevated temperatures of 90 to 95 ° C to form the cyano compound IV

IV 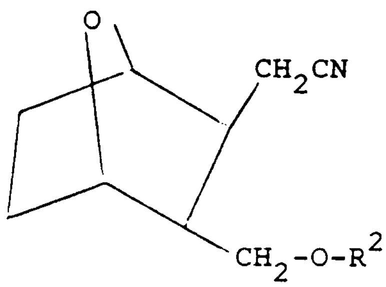

The cyano compound IV in toluene at reduced temperatures of -78 ° C to 0 ° C under argon is treated with diisobutylaluminum hydride to form the aldehyde V

V 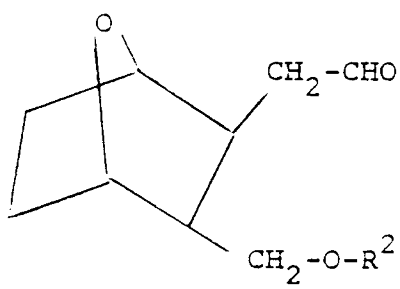

Employing the Wadsworth-Emmons procedure, aldehyde V is added to a solution of phosphonate D

D $$(CF_3CH_2O)_3\overset{O}{\overset{\|}{P}}(CH_2)_mCOOCH_3$$

18-Crown-6, and potassium hexamethyl silyl amide in tetrahydrofuran to form the ester VI (where m = 0)

VI 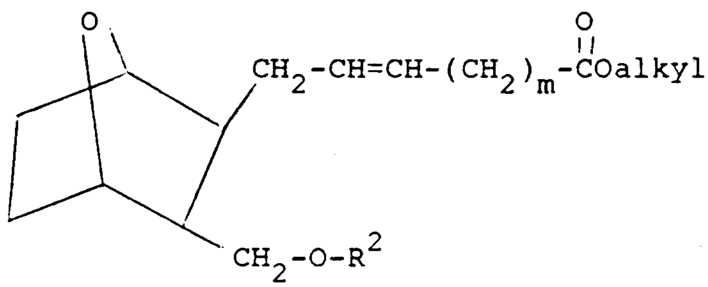

which is dissolved in inert solvent such as toluene and reduced by treatment with diisobutyl aluminum hydride at reduced temperatures of -78° to 0°C under argon to form alcohol VII (where m is 1 to 6).

Alternatively, the phosphonium salt E

E 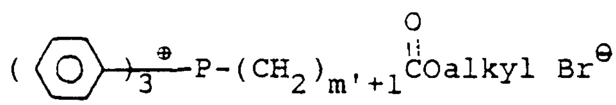

(wherein m' is 2 to 6)

is reacted with the aldehyde V in the presence of bases like potassium t-amylate, potassium t-butoxide, n-butyl lithium, etc. to form the ester VI (where m is 1 to 6) which is then reduced with diisobutyl aluminum hydride as above to the alcohol VII (where m = 1 to 6)

VII 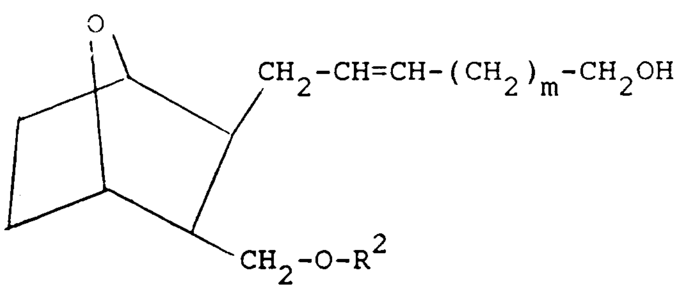

The alcohol VII is then reacted in dichloromethane with bromo compound F

F $Br(CH_2)_nCOOalkyl$

in the presence of a phase transfer catalyst like tetrabutyl ammonium sulfate and base such as aqueous sodium hydroxide under argon to form ester VIII

VIII 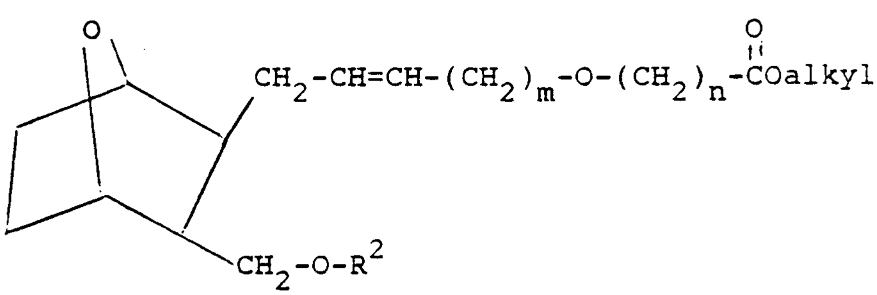

which is hydrolyzed by treatment with lithium hydroxide to form acid IX

IX

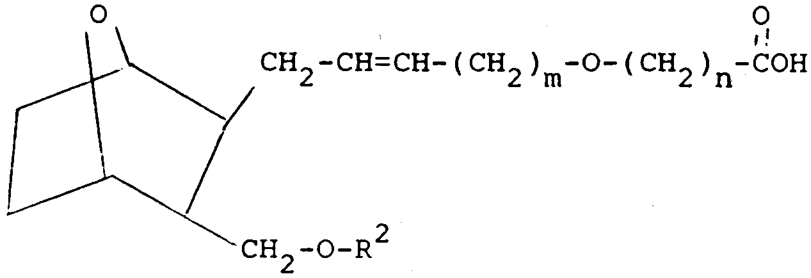

Acid IX may then be converted to the corresponding hydroxamate by first treating a solution of acid IX in an inert aromatic solvent such as benzene with oxalyl chloride and stirring the mixture at room temperature under nitrogen to form the acid chloride IX'

IX'

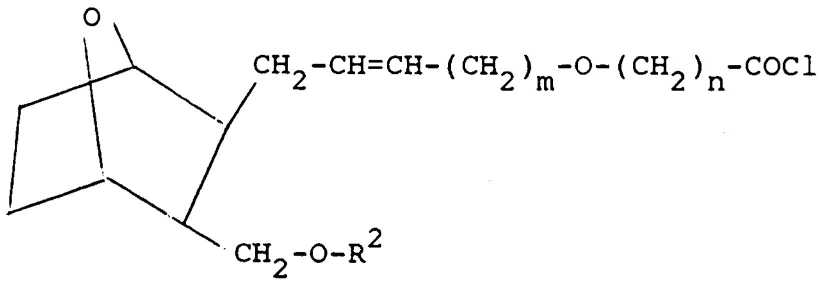

The acid chloride IX' is dissolved in an inert solvent such as tetrahydrofuran and added to a cold solution of IXA

IXA

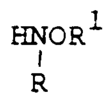

in tetrahydrofuran and water in the presence of organic base such as triethylamine. The mixture is stirred under nitrogen atmosphere while being cooled in an ice bath, to form hydroxamate IE

IE

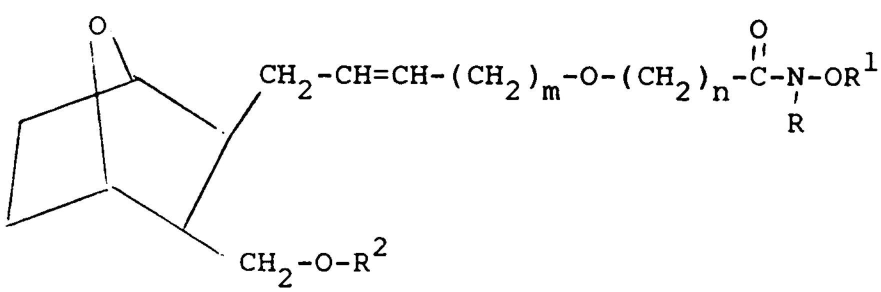

Compounds of Formula I wherein Q is $-CH_2-CH_2-CH_2-(CH_2)_m-$, m is 0 to 6, X is O, n is 1 to 4, p is 1 and Y is O, that is

IF

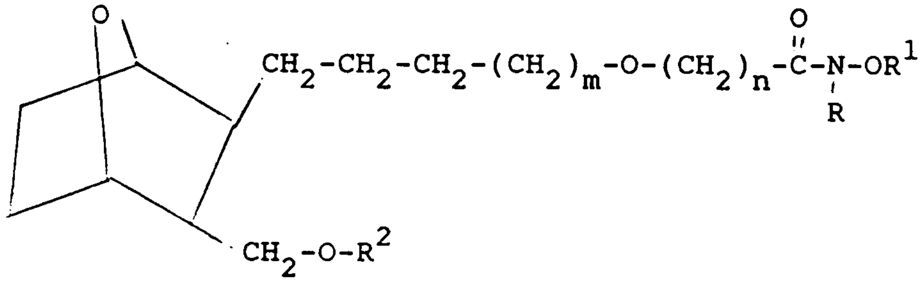

may be prepared by hydrogenating ester VI

VI

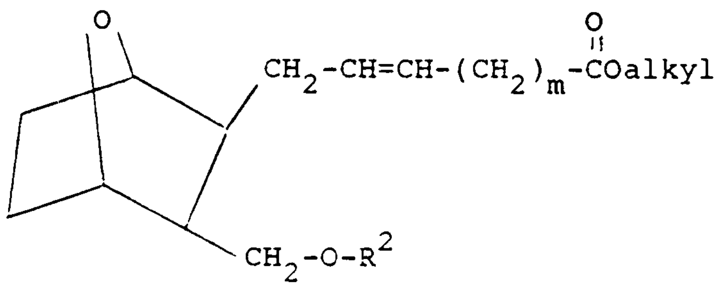

by treating VI dissolved in dry methanol, with hydrogen in the presence of palladium on carbon to form the ester X

X

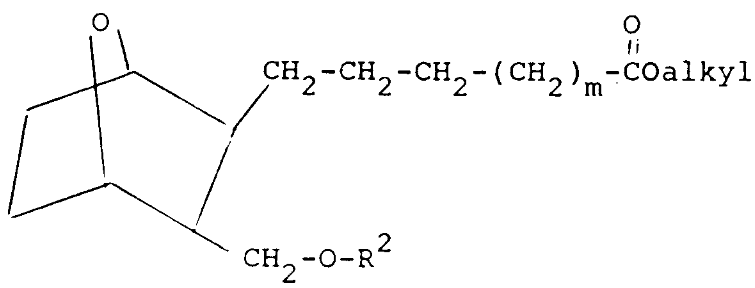

which is then reduced by treating with lithium aluminum hydride in the presence of tetrahydrofuran under argon to form the alcohol XI

XI

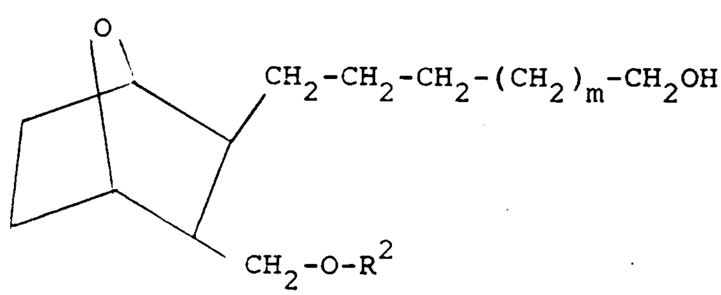

The alcohol XI is then treated with bromo compound F in the presence of tetrabutyl ammonium sulfate and base such as aqueous sodium hydroxide under argon to form ester XII

XII 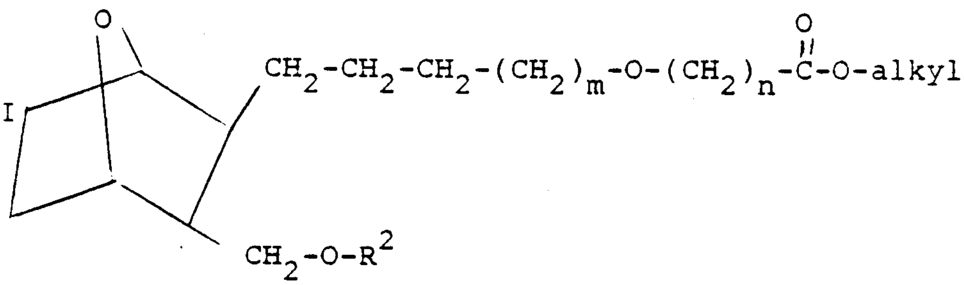

which is then hydrolyzed by treatment with sodium hydroxide (or lithium hydroxide) to form acid XIII

XIII 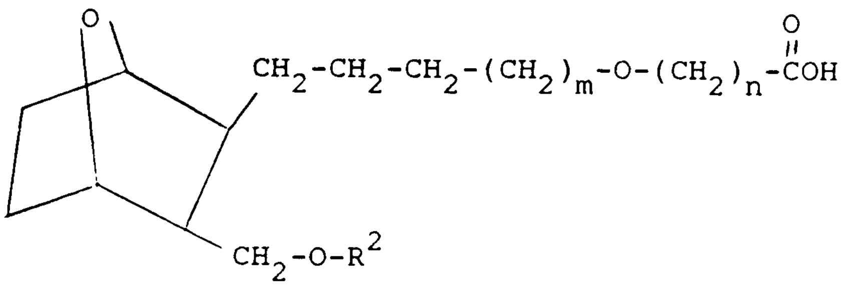

Acid XIII may then be converted to the corresponding hydroxamate IF

IF 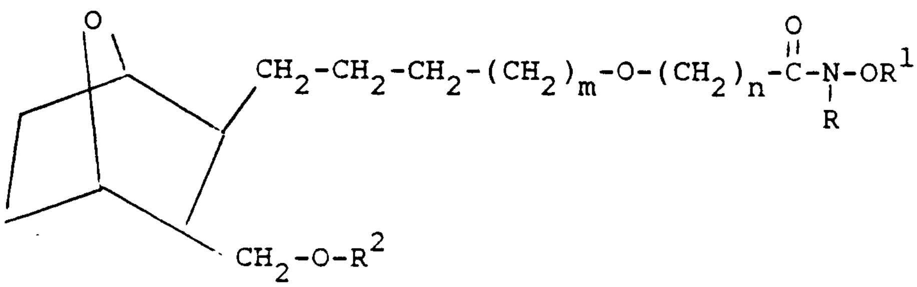

employing procedures as set out herein.

Compounds of formula I wherein Q is $-CH_2-(CH_2)_2-(CH_2)_m-$, X is S, n is 1 to 4, m is 1, p is 1 and Y is O may be prepared from alcohol XI

XI 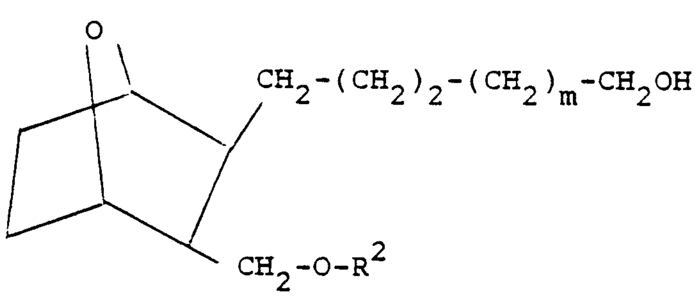

as follows.

Aldehyde V is treated with a reaction medium formed of sodium hydride or other base such as sodium methoxide or potassium t-butoxide, suspended in dry tetrahydrofuran, and a phosphonate of the structure

G $(CH_3O)_2P(O)CH_2COOCH_3$

to form the ester XIV

XIV

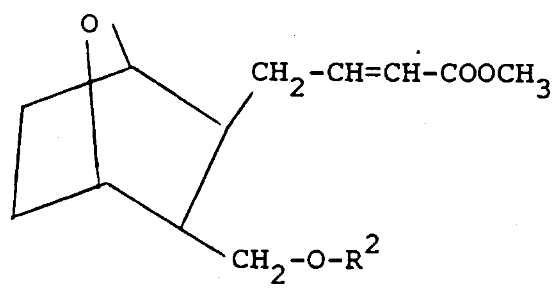

Ester XIV is then dissolved in methanol and reduced by treatment with hydrogen in the presence of a palladium on carbon catalyst to form the saturated ester XV

XV

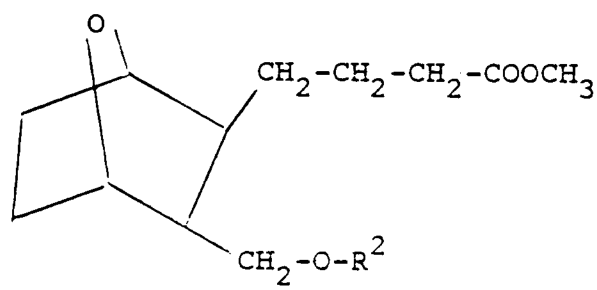

which is then further reduced by treatment with lithium aluminum hydride or other reducing agent in the presence of an inert solvent such as tetrahydrofuran to form the alcohol XVI

XVI

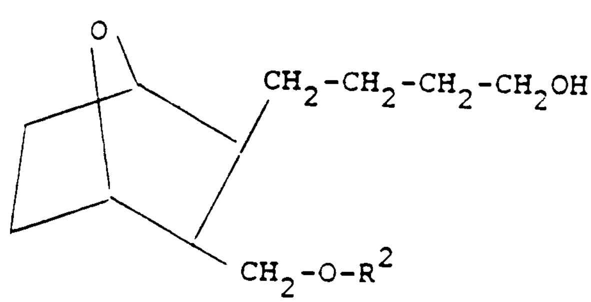

The alcohol XVI is then subjected to a modified Mitsonubu reaction wherein a mixture of the alcohol XVI and thiolacetic acid in an inert solvent such as tetrahydrofuran, ether or toluene is reacted with a mixture of triphenylphosphine and diisopropylazodicarboxylate in an inert solvent such as tetrahydrofuran at reduced temperatures of from about 0° to about 25°C to form thioacetate XVII

XVII

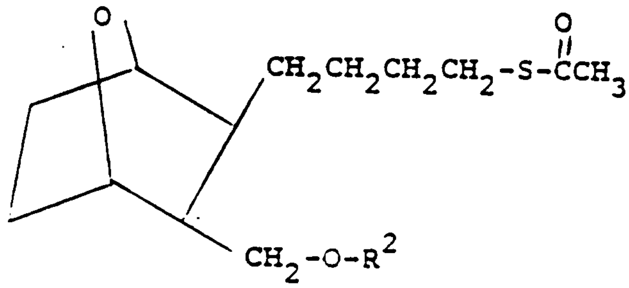

Thioacetate XVII is then reduced by treating with lithium aluminum hydride or diborane in the presence of an inert organic solvent such as tetrahydrofuran or ether to form the thiol XVIII

XVIII

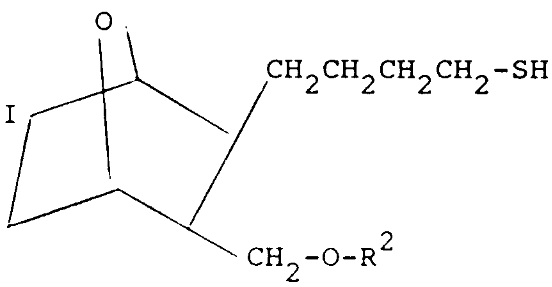

Thiol is then alkylated by reacting with alkylating agent H

H $\quad$ Hal-$(CH_2)_n$-$CO_2$alkyl

in the presence of a base such as sodium or potassium carbonate and an inert solvent such as acetone, tetrahydrofuran or dimethylformamide to form ester XIX

XIX

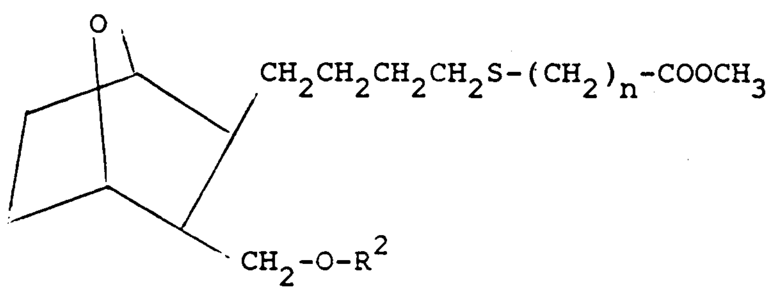

which is then hydrolyzed by treatment with alkali metal hydroxide such as sodium or lithium hydroxide, to form the acid XX

XX

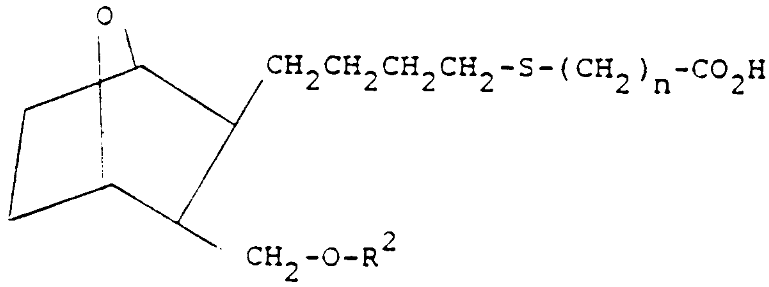

The acid XX may then be converted to the hydroxamate IG

IG

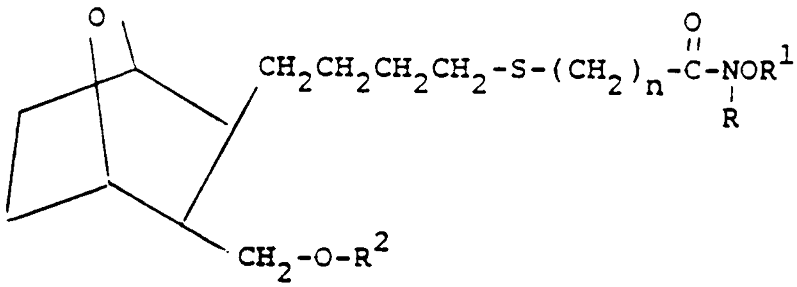

employing procedures as set forth herein.

The 7-oxabicycloheptane ether compounds of formula I of the invention wherein X is O, Q is $CH_2CH_2CH_2(CH_2)_m$, m is 1 to 6, p is 1, Y is S and n is 1 to 4, that is

IH

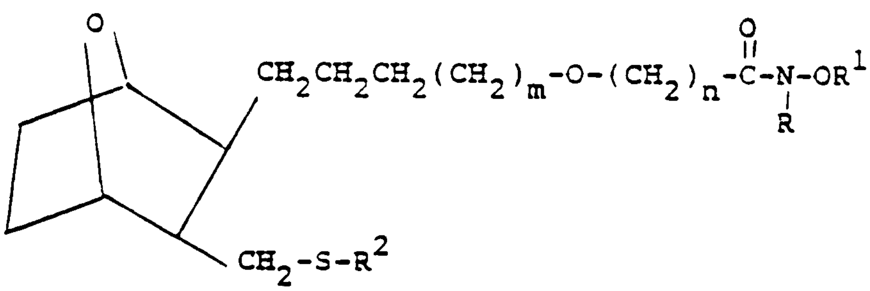

may be prepared starting with the cyanoalcohol XXI

XXI

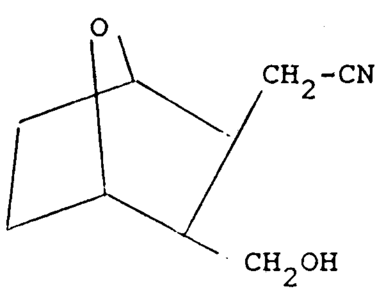

which is subjected to a benzylation wherein compound XXI is reacted with a base such as NaH, $NaOCH_3$, KH, $KOt\text{-}C_4H_9$ and the like in the presence of an inert solvent, such as dimethylformamide, dimethylsulfoxide, dimethoxyethane or tetrahydrofuran to form the mono benzylether compound XXII

XXII

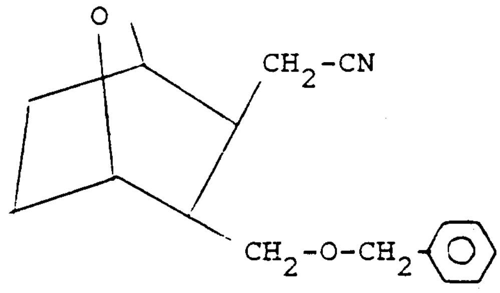

Compound XXII is reduced with diisobutyl aluminum hydride in the presence of an inert solvent, such as tetrahydrofuran, toluene or methylene chloride, to form the aldehyde XXIII

XXIII

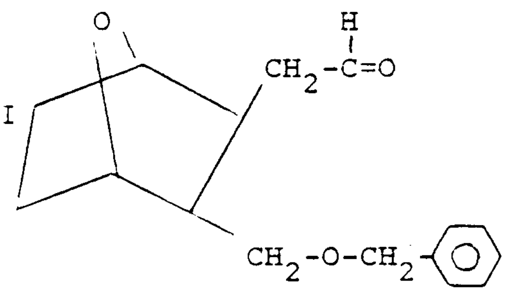

Aldehyde XXIII is then made to undergo a Wadsworth-Emmons reaction (employing the procedure set out above with respect to the conversion of aldehyde V to VI) to form the ester XXIV

XXIV

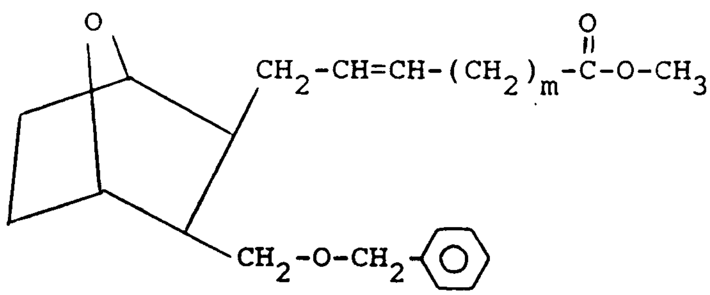

which is reduced with diisobutyl aluminum hydride in the presence of toluene at reduced temperatures of from -78° to 0°C to form the alcohol XXV

XXV

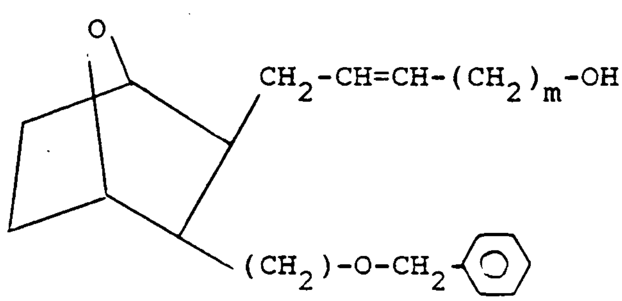

Alcohol XXV is then treated with tetrabutyl ammonium sulfate and an alkylating agent I

I    $Hal\text{-}(CH_2)_n\text{-}CO_2\text{-}t\text{-}C_4H_9$

in the presence of aqueous base such as sodium or potassium hydroxide and dichloromethane to form the ester XXVI

XXVI

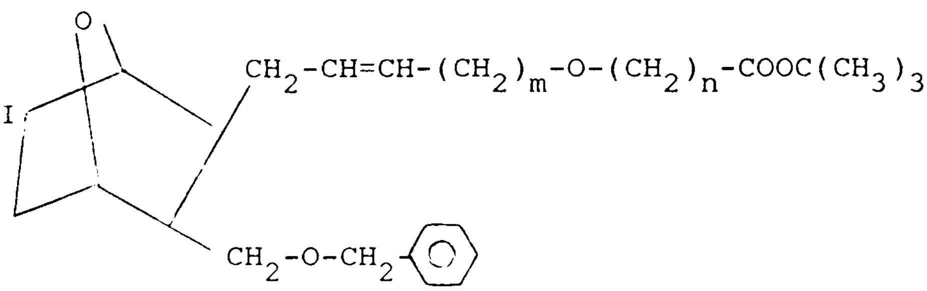

which is then reduced by treatment with hydrogen in the presence of methanol and palladium on carbon catalyst to form the alcohol XXVII

XXVII

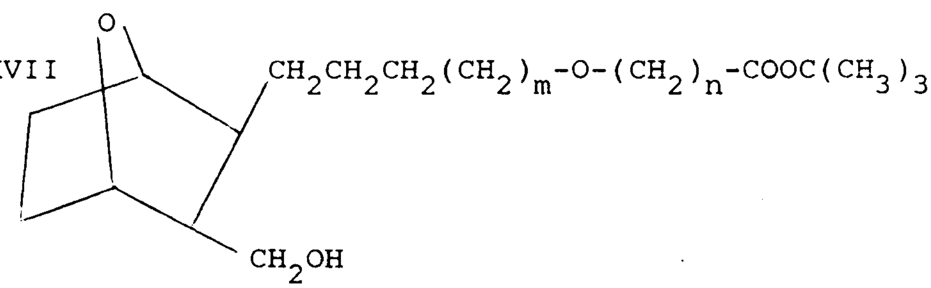

Alcohol XXVII is then subjected to a modified Mitsonubu reaction as described hereinbefore by treating a mixture of alcohol and thiolacetic acid in an inert solvent such as tetrahydrofuran with a mixture of triphenylphosphine and diisopropylazadicarboxylate to form the thiolacetate XXVIII

XXVIII

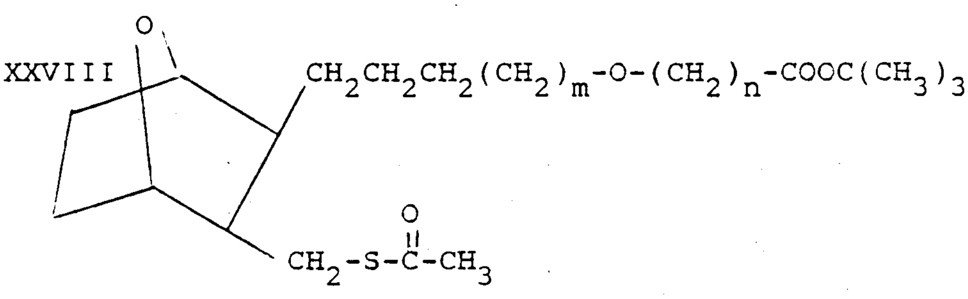

Thiolacetate XXVIII is then reacted with a halide C''

C''    $R^2Hal$

in the presence of a base such as potassium carbonate and alcohol solvent such as methanol to form the ester XXIX

XXIX 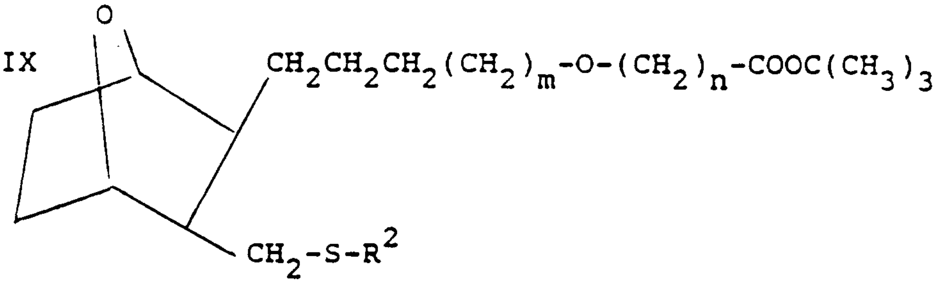

which is then treated with an acid such as trifluoroacetic acid in the presence of an inert solvent such as methylene chloride, preferably in the presence of anisole, to form the acid XXX

XXX 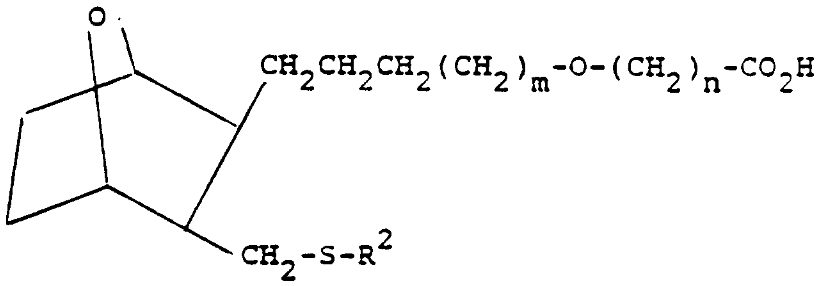

Acid XXX may then be converted to the hydroxamate IH employing procedures as set out herein.

Compounds of formula I wherein Q is $CH_2$-$(CH_2)_2$-$(CH_2)_m$ and m is O, X is O or S and Y is O or S, that is,

IJ 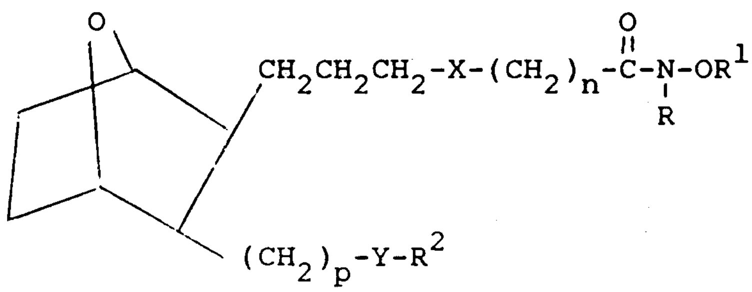

may be prepared by subjecting aldehyde XXIII

XXIII 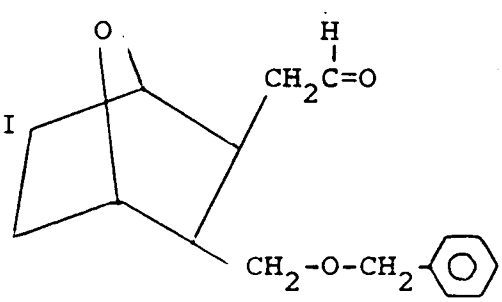

to a Wittig reaction by reacting aldehyde XXIII with an alkoxymethyltriphenylphosphonium halide, such as (methoxymethyl)triphenylphosphonium chloride and a base like potassium t-amylate to form the aldehyde

XXXI

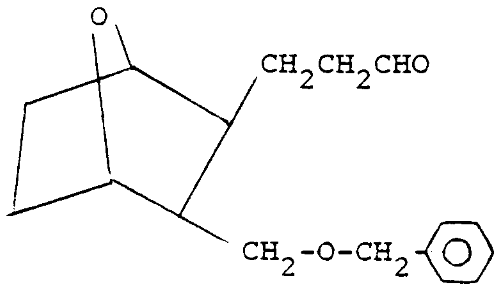

Aldehyde XXXI is then reduced by treatment with sodium borohydride to form the alcohol XXXII

XXXII

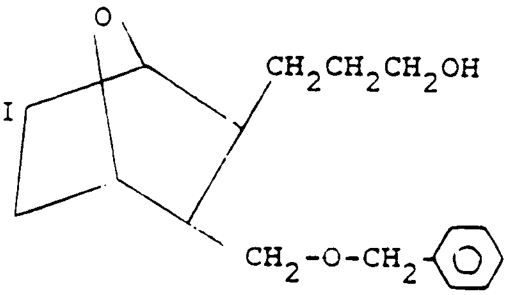

Alcohol XXXII may then be treated with appropriate alkylating agent J

J  $Hal(CH_2)_n$-$CO_2$t-butyl

in the presence of sodium or potassium hydroxide to form the ester XXXIII

XXXIII

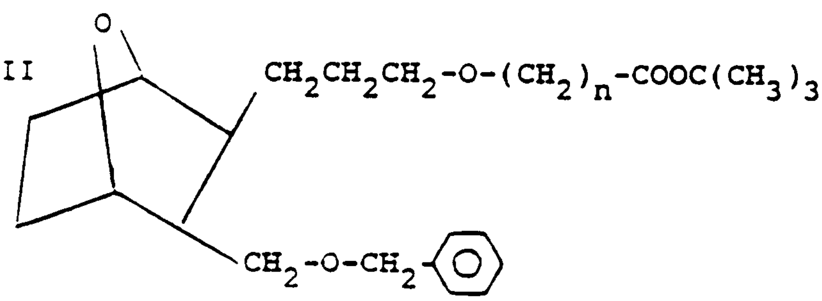

The ester XXXIII may then be employed to prepare compounds of the invention wherein m is O, and X is O or S and Y is O or S employing procedures as set out hereinbefore for preparing compounds wherein m is other than 1.

Compounds of formula I wherein Q is $CH_2$-$(CH_2)_2$-$(CH_2)_m$, m is 2 to 6, X is S and Y is O, that is

IK

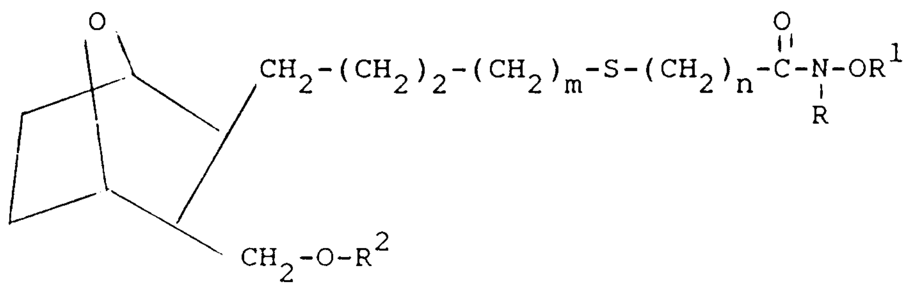

may be prepared by reducing ester XXXIV

XXXIV

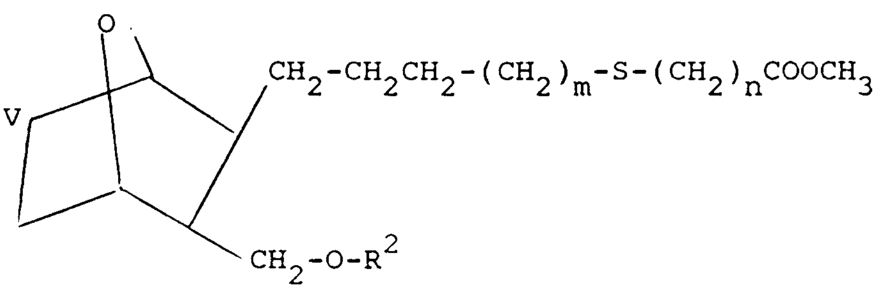

by treatment with lithium aluminum hydride to form the alcohol XXXV

XXXV

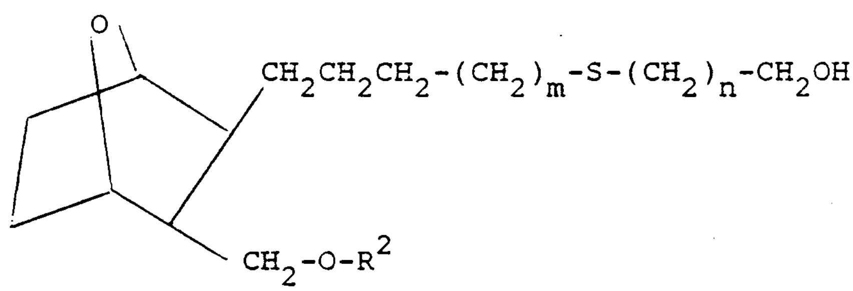

Alcohol XXXV is then subjected to a modified Mitsonubu reaction to form the corresponding thioacetate XXXVI

XXXVI

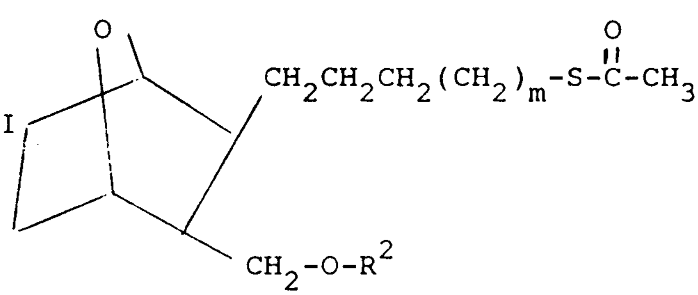

Thioacetate XXXVI is reduced by treatment with lithium aluminum hydride to form the thiol XXXVII

XXXVII 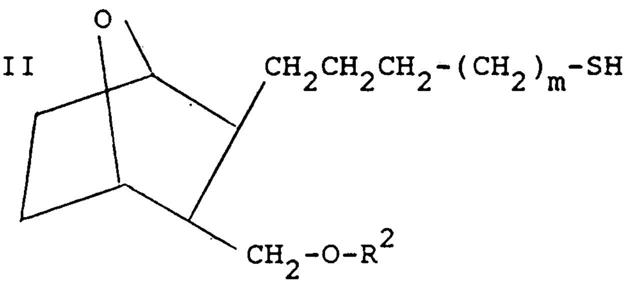

which is then alkylated by reaction with J

J  $Hal(CH_2)_nCO_2$t-butyl

as described above to form ester XXXVIII

XXXVIII 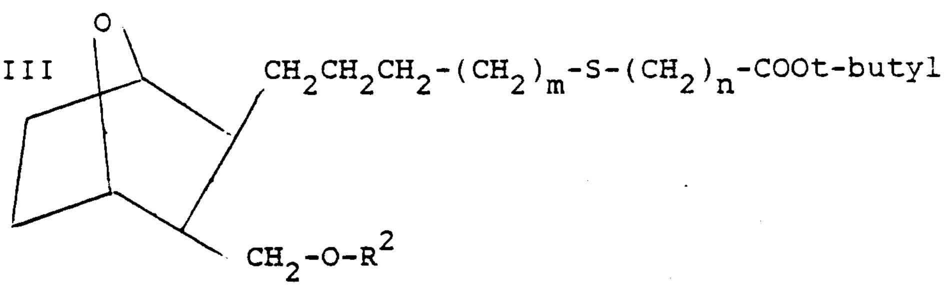

Ester XXXVIII is then converted into the corresponding acid which is then converted to the hydroxamate IK as described above.

Compounds of the invention wherein X is S, Q is $CH_2CH_2CH_2(CH_2)_m$ wherein m is 1 to 6, p is 1, Y is S and n is 1 to 4, that is

IL 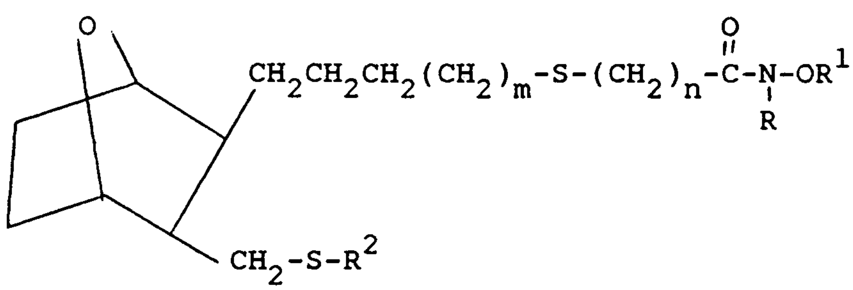

may be prepared starting with the alcohol XXXIX

XXXIX

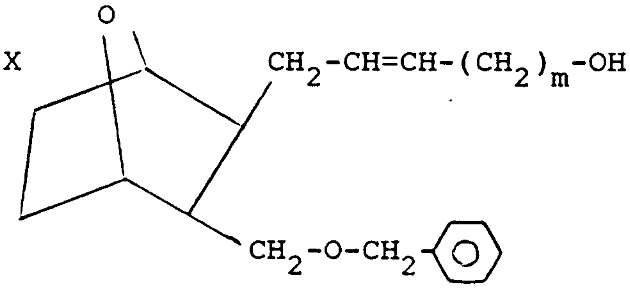

which is reduced by treatment with hydrogen in the presence of methanol and palladium on carbon catalyst to form the diol XL

XL

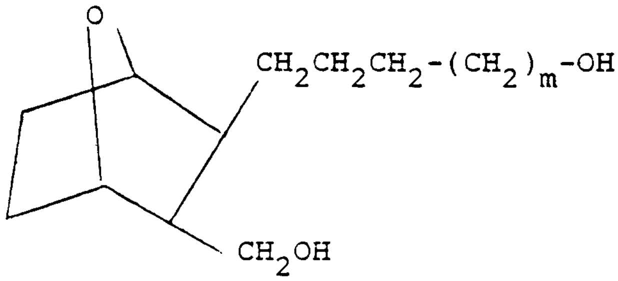

Diol XL is then subjected to a modified Mitsonubu reaction by treating with diisopropylazadicarboxylate, triphenylphosphine and thiolacetic acid as described hereinbefore to form the alcohol XLI

XLI

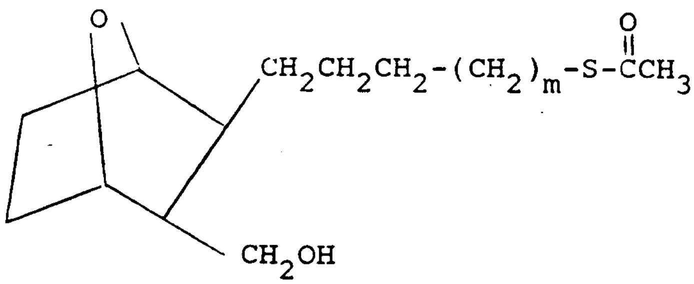

which is then reduced by treatment with lithium aluminum hydride in the presence of an inert solvent such as tetrahydrofuran to form thiol XLII

XLII

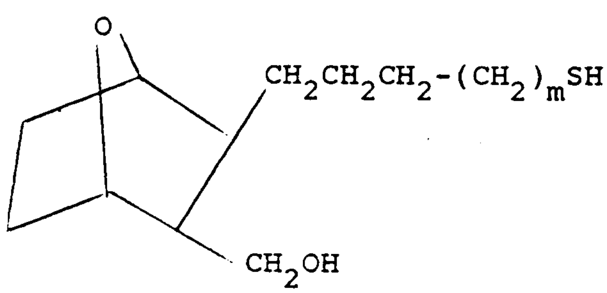

Alcohol XLII is then treated with alkylating agent J

J    Hal-$(CH_2)_n$-$CO_2$t-butyl

in the presence of a base such as potassium carbonate and methanol to form alcohol XLIII

XLIII

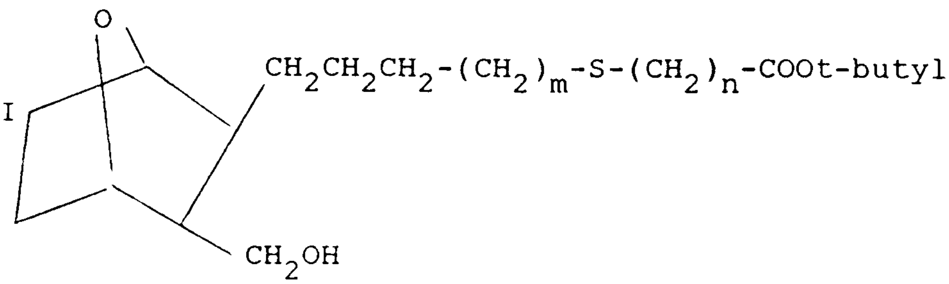

The alcohol XLIII is subjected to a modified Mitsonubu reaction as described above to form the thiolacetate XLIV

XLIV

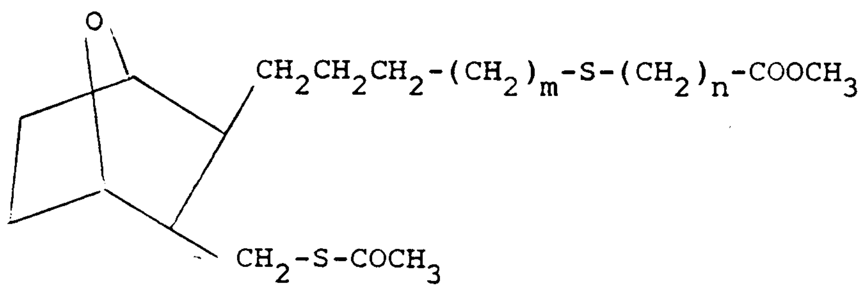

The thiolacetate XLIV is then treated with halide C''

C''    $R^2$Hal

in the presence of a base such as potassium carbonate and methanol to form the thio ester XLV

XLV

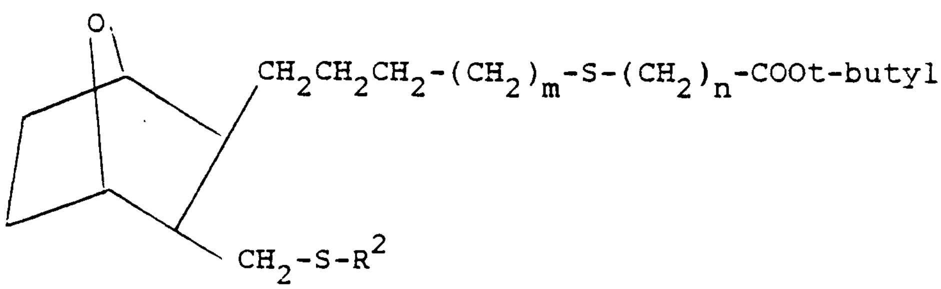

which is converted by reaction with trifluoroacetic acid as described hereinbefore to the corresponding acid XLVI

XLVI

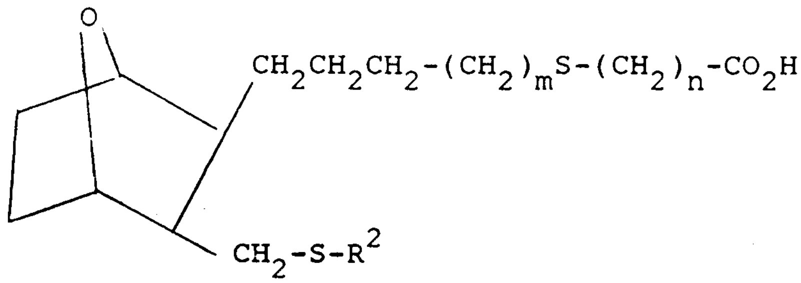

Acid XLVI is then converted to the hydroxamate IL employing procedures as described above.

Compounds of formula I wherein Q is $-CH_2CH=CH(CH_2)m-$, m is 2 to 6, X is S and Y is O, that is compounds of the structure IM

IM

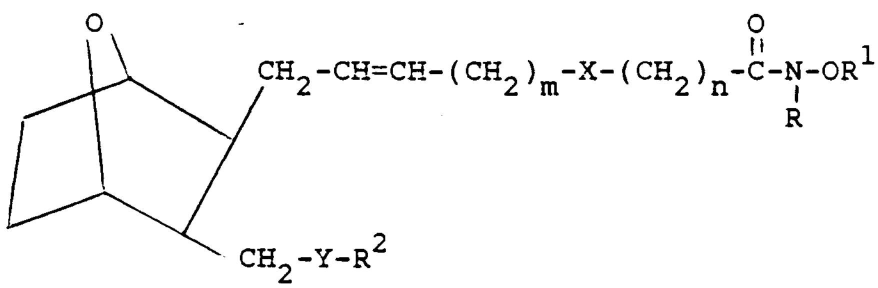

may be prepared by subjecting alcohol VII

VII

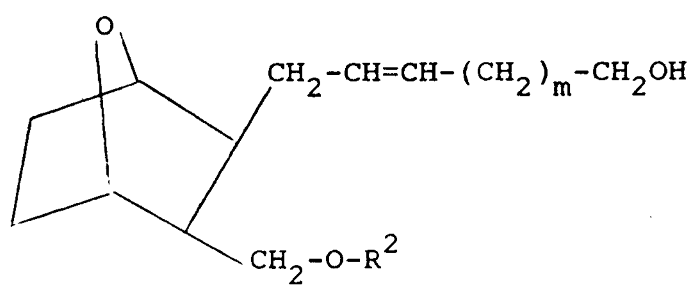

(wherein m is 1 to 6)

to a modified Mitsonubu reaction as described hereinbefore to form thioacetate XLVII

XLVII

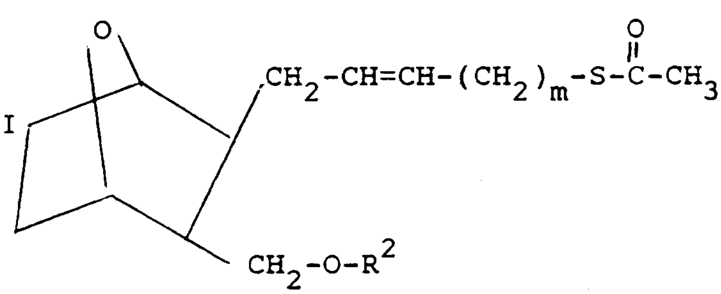

which is then reduced by treatment with lithium aluminum hydride to form the thiol XLVIII

XLVIII

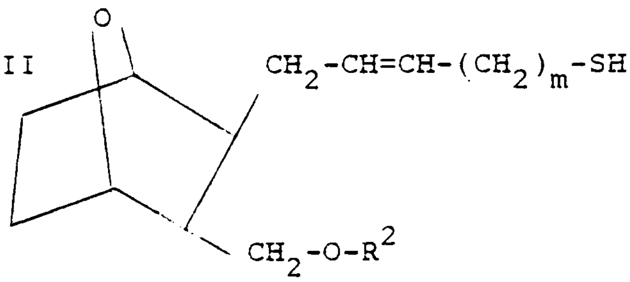

Thiol XLVIII is alkylated by reaction with J

J  $Hal(CH_2)_n-CO_2t$-butyl

as described above to form ester XLIX

XLIX

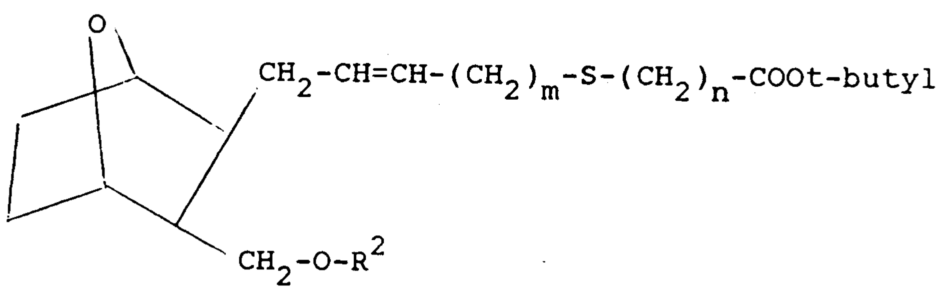

Ester compound XLIX is then converted into the corresponding acid L

L

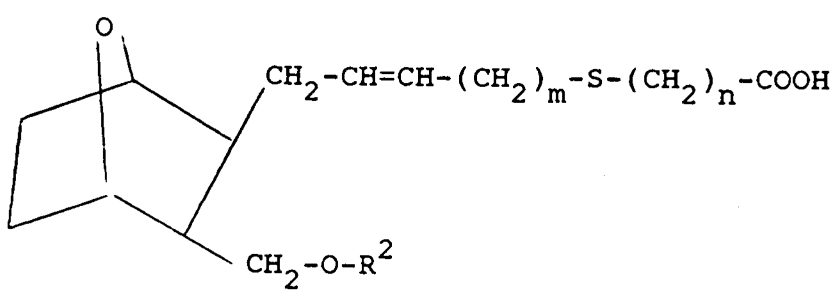

which is then converted to the hydroxamate IM as described herein.

Compounds of formula I wherein Q is $-CH_2-CH=CH-(CH_2)_m-$, m is 1 to 6, X is S, n is 1 to 4, p is 1 and Y is S, that is

IN

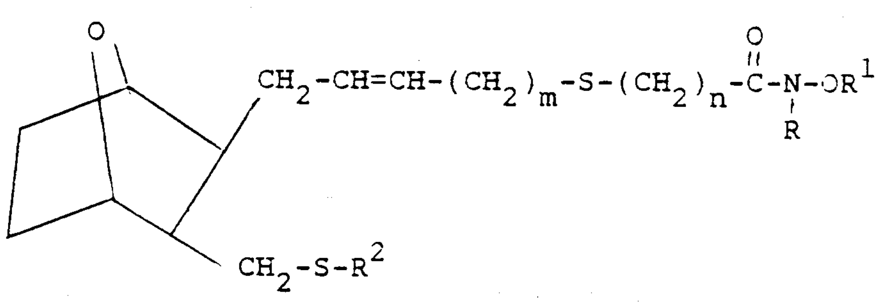

may be prepared by treating K

K

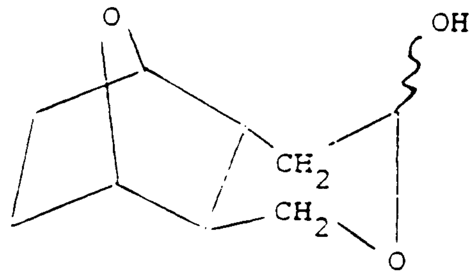

(described in U. S. Patent No. 4,143,054) with a Wittig reagent of the structure

M

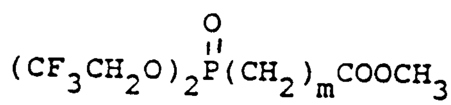

in the presence of a base such as potassium hexamethyl silylamide and 18-Crown-6 in the presence of an inert solvent such as tetrahydrofuran to form the alcohol LI

LI

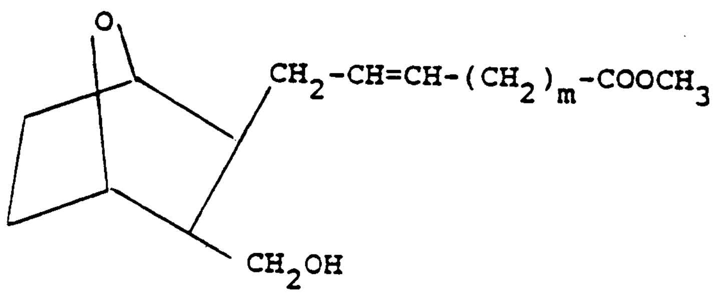

The alcohol LI is then subjected to a modified Mitsonubu reaction wherein a mixture of the alcohol LI and thiolacetic acid in an inert solvent such as tetrahydrofuran, ether or toluene is reacted with a mixture of triphenylphosphine and diisopropylazodicarboxylate in an inert solvent at reduced temperatures of from about 0° to about 25°C to form thioacetate LII

LII

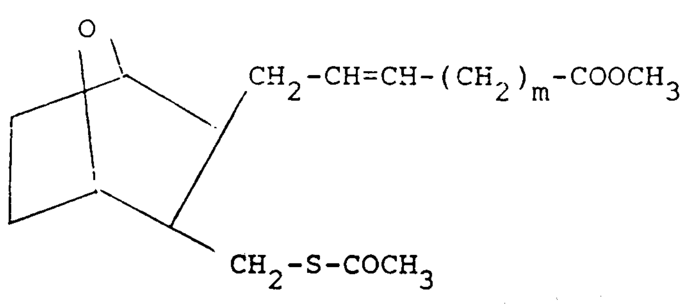

Thioacetate LII is then reacted with halide C'', mesyl-$OR^2$ (C) or tosyl-$OR^2$ (C') in the presence of methanol and base such as potassium carbonate to form the thioether LIII

LIII

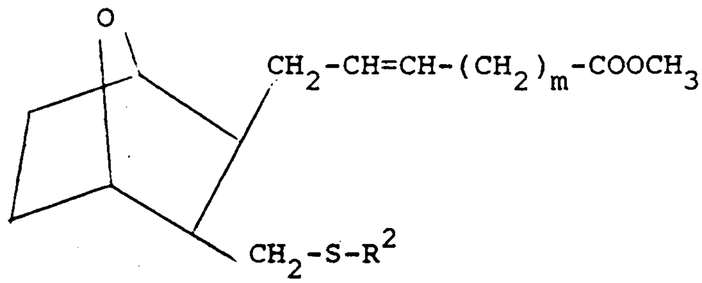

Thioether LIII is then reduced by treatment with diisobutyl aluminum hydride (DIBAL-H) at reduced temperatures (-78° to 0°C) in the presence of an inert solvent such as toluene, or tetrahydrofuran to form the alcohol LIV

LIV

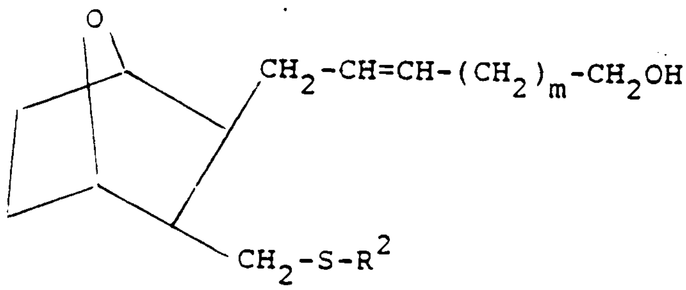

The alcohol LIV is then subjected to a modified Mitsonubu reaction, as described above with respect to the conversion of alcohol VII to thioacetate XLVII, to form the thioacetate LV

LV

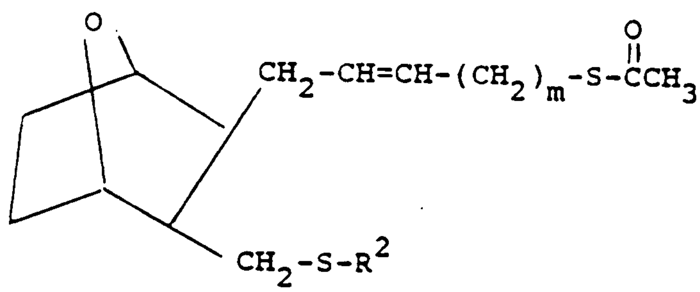

The thioacetate LV is then alkylated by reacting with alkylating agent J

J Hal-$(CH_2)_n$-$CO_2$t-butyl

in the presence of base such as potassium or sodium carbonate and methanol to form thioether LVI

LVI

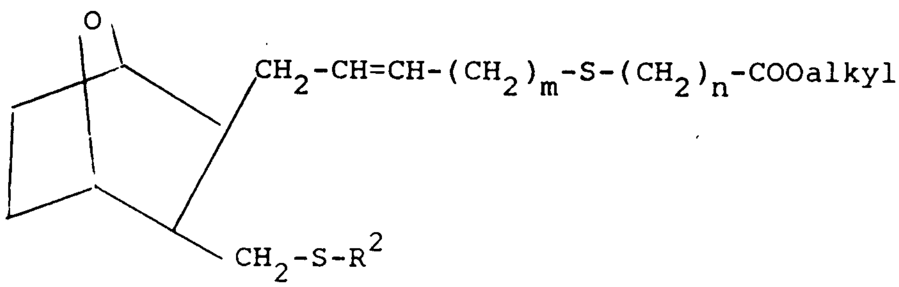

which is then hydrolyzed by treatment in methylene chloride with trifluoroacetic acid to form the acid LVII

LVII

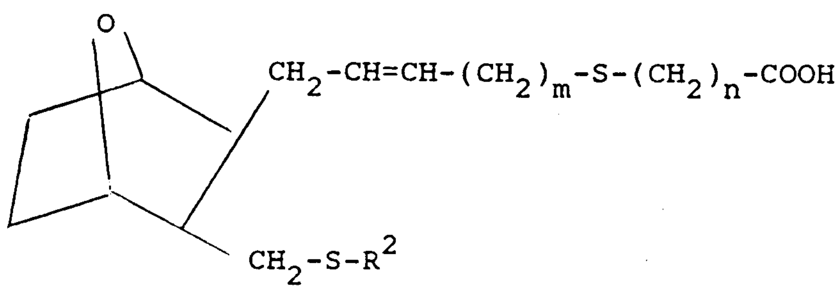

The acid LVII may then be converted to the hydroxamate IN employing procedures as set forth herein.

Compounds of formula I wherein Q is $-CH_2-CH=CH-(CH_2)_m-$, m is 1 to 6, X is O, n is 1 to 4, p is 1 and Y is S, that is compounds of the structure 10

IO

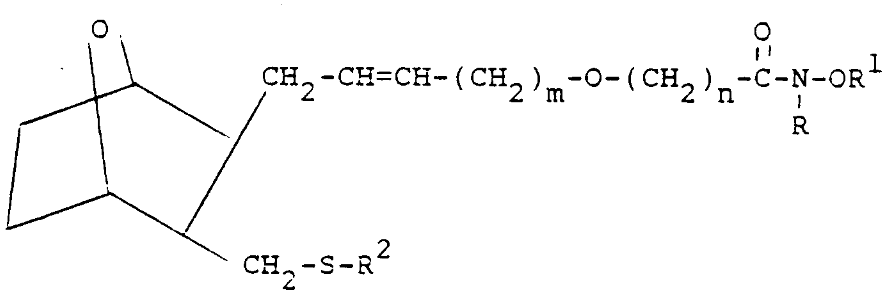

may be prepared starting with alcohol LIV and subjecting LIV to an alkylation by reaction with alkylating agent J,

J $Br(CH_2)_n-CO_2-t-C_4H_9$

in the presence of $(C_4H_9)_4N^+HSO_4^-$ and base and appropriate solvent such as tetrahydrofuran to form the ester LVIII

LVIII

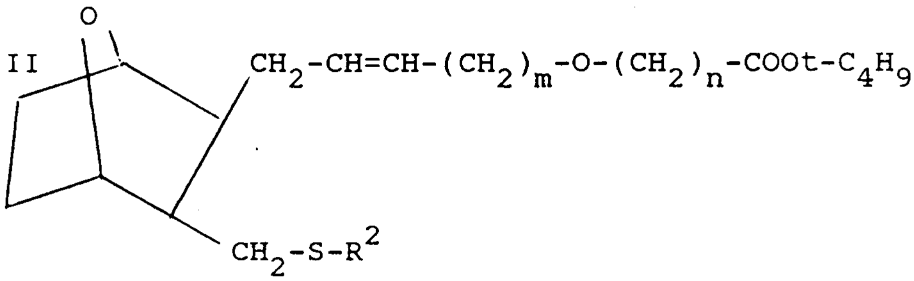

which is then reacted in methylene chloride with trifluoroacetic acid to form the acid LIX

LIX

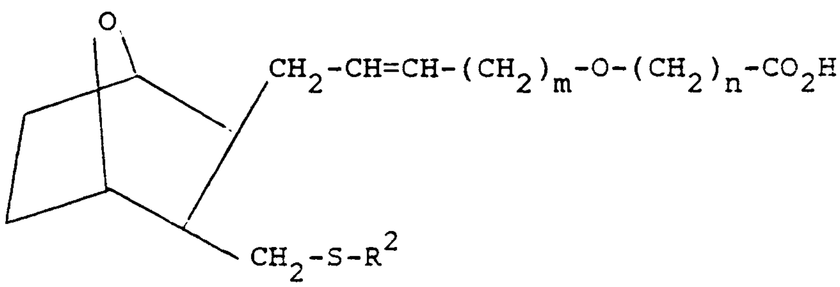

The acid LIX may then be converted to the hydroxamate 10 employing procedures as set forth herein.

Compounds of the invention wherein p is 2 to 5, that is

IP

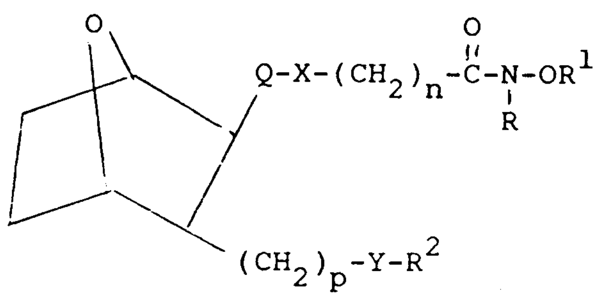

(where p is 2 to 5)

may be prepared as follows.

Alcohol LX or LXA

LX

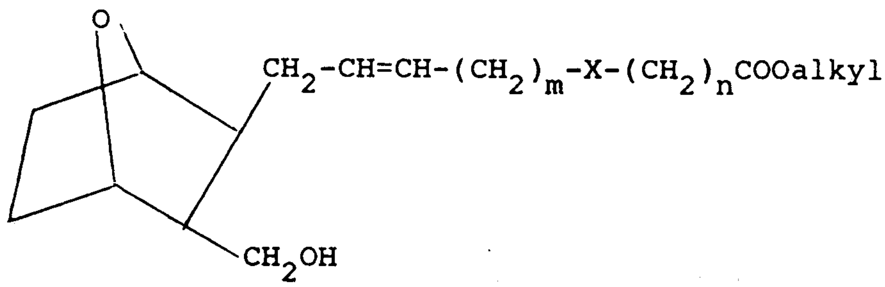

or

LXA

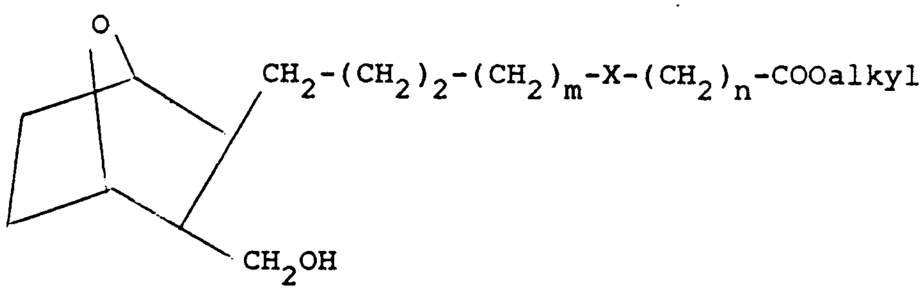

is used to form the aldehyde LXI (where Q is $-CH_2-CH=CH-(CH_2)_m-$)

LXI

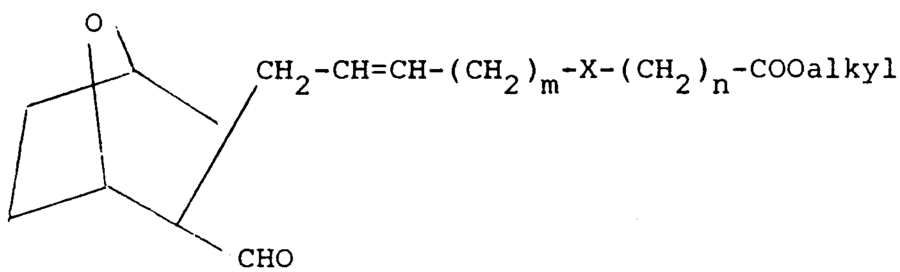

or LXIA (where Q is $-CH_2-(CH_2)_2-(CH_2)_m-$)

LXIA

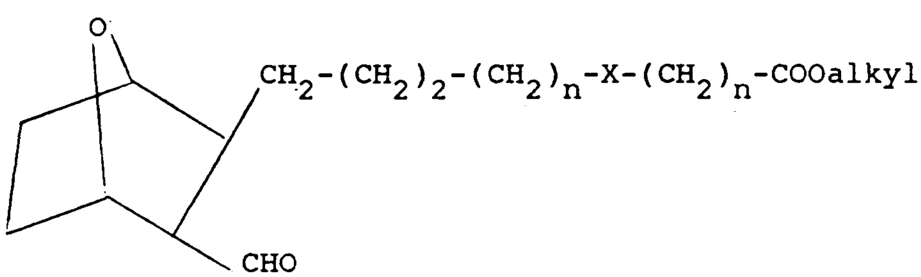

Thus, to form aldehyde LXI where Q is $-CH_2-CH=CH-(CH_2)_m-$, compound LX is subjected to a Collins oxidation, for example, by reacting LX with chromium trioxide pyridine complex. To form the aldehyde LXIA (where Q is $-CH_2-(CH_2)_2-(CH_2)_m-$) compound LX is reduced, for example, with hydrogen over a palladium on carbon catalyst, to form hydroxymethyl compound LXA (where Q is $CH_2-CH_2-CH_2-(CH_2)_m$) and

compound LXA is subjected to a Collins oxidation to form aldehyde LXIA (where Q is $CH_2$-$(CH_2)_2$-$(CH_2)_m$). The aldehyde LXI or LXIA is used to prepare aldehyde LXII

LXII 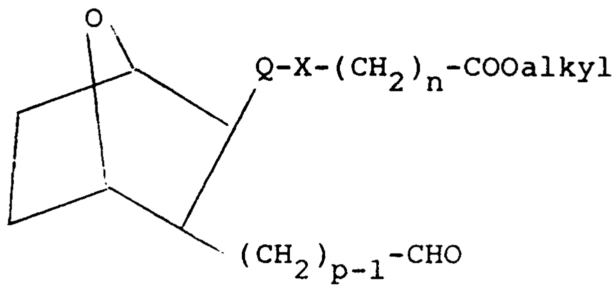

(where p is 2-5) by carrying out a homologation sequence, such as a Wittig reaction with $(C_6H_5)_3P=CHOMe$ followed by hydrolysis, (p-1) times. The aldehyde LXII (where p is 2 to 5) is then carried on to compounds of this invention where p is 2 to 5, that is, IP

IP 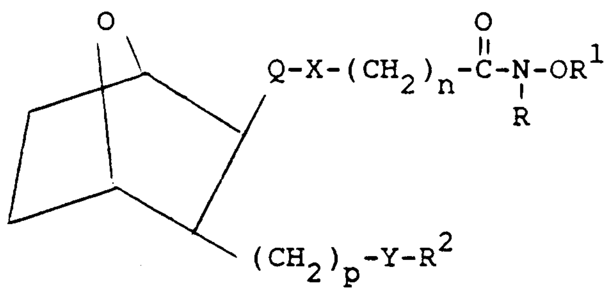

(where p is 2 to 5)

by reducing aldehyde LXII by reacting with a reducing agent such as sodium borohydride to form alcohol LXIII

LXIII 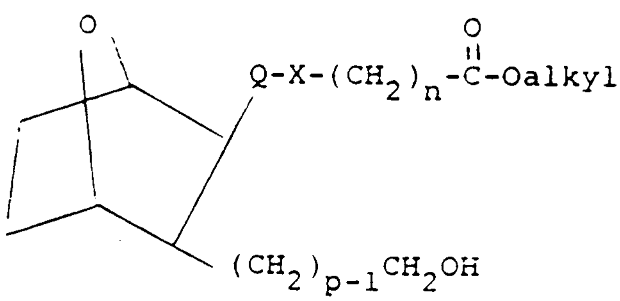

The alcohol LXIII may then be employed to form the various compounds of the invention employing procedures as set out hereinbefore.

The 7-oxabicycloheptane ether compounds of formula I of the invention wherein Q is $(CH_2)_t$,t is 2, X is O, n is 1 to 8, p is 1 and Y is O, that is, $IQ_x$

IQ

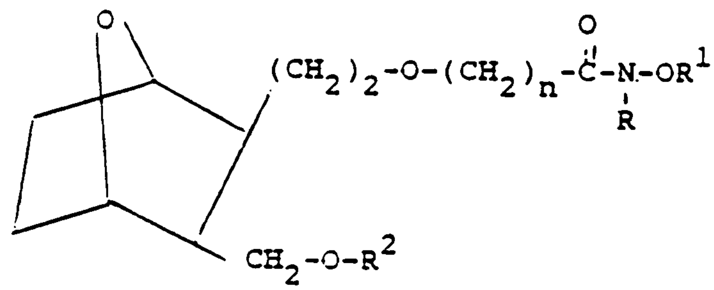

may be prepared starting with the aldehyde XXIII which may then be reduced by reaction with lithium aluminum hydride to form the alcohol V'

V'

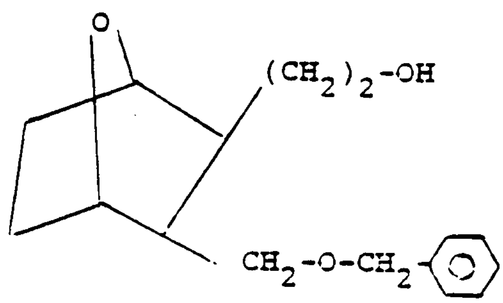

Compound V' is then used to prepare the final products wherein Q is $(CH_2)_t$ and t is 2 as will be described hereinafter.

The 7-oxabicycloheptane ether compounds of formula I of the invention wherein Q is $CH_2$, n is 1 to 8, X is O, Y is O, and p is 1, that is,

IR

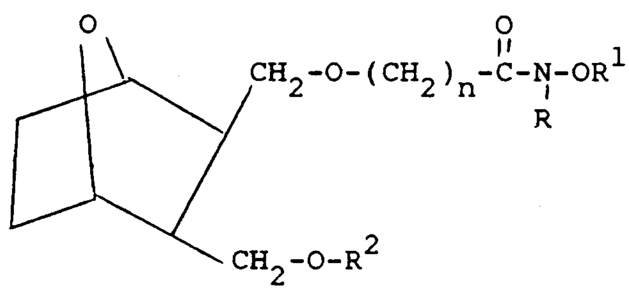

may be prepared as follows.

Diol B is subjected to a benzylation wherein compound VI is reacted with a base such as NaH, $NaOCH_3$, KH, KOt-$C_4H_9$ and the like and a benzyl halide such as benzylbromide in the presence of an inert solvent such as dimethylformamide, dimethoxyethane, tetrahydrofuran or benzene to form the monobenzylether V"

V"

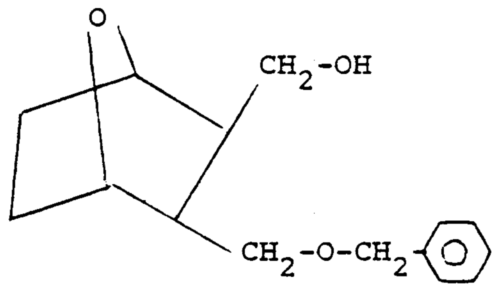

which is used to prepare compounds of formula I wherein Q is $CH_2$ as described hereinafter.

Compound V' or V" herein referred to as compounds V' - V", that is

V'
or
V"

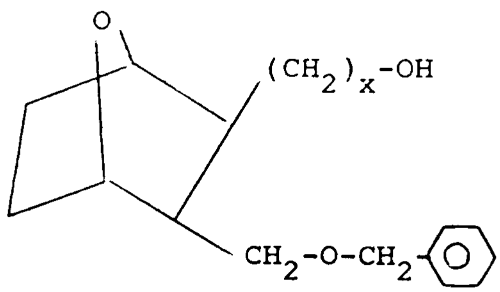

(wherein Q is $(CH_2)_2$ where compound V' is used and Q is $CH_2$ where compound V" is used)

is subjected to O-alkylation wherein it is reacted with a base such as KOH or NaOH and a silyl compound of the structure

A'

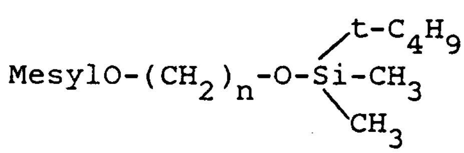

in the presence of an aromatic solvent such as xylene, toluene or mesitylene to form the silyl compound LXIV

LXIV

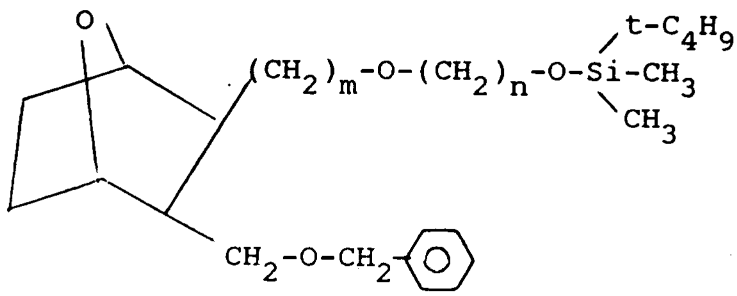

(Q is $(CH_2)_2$ if V' is used as the starting material or Q is $CH_2$ if V" is used as the starting material)

which is desilylated by reacting same with tetra-n-butyl ammonium fluoride in the presence of an inert solvent such as tetrahydrofuran, to form the alcohol LXV

LXV

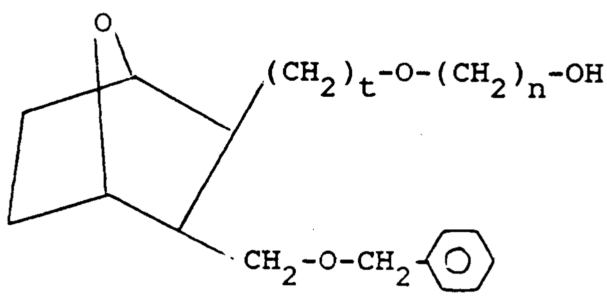

(wherein t is 2 or 1)

The alcohol LXV is then made to undergo a Jones oxidation by reacting LXV with chromium trioxide or other oxidizing agent such as pyridinium dichromate or pyridinium chlorochromate, in the presence of acetone or dimethylformamide to form the acid LXVI

LXVI

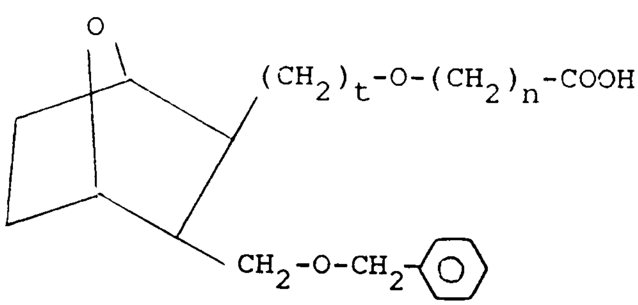

(where t is 2 or 1)

Acid LXVI is then subjected to esterification by reacting acid LXVI with diazomethane or other esterifying agent of the structure $RCHN_2$ (where R is an alkyl group) or an alkyl iodide and sodium bicarbonate in dimethylformamide to form the ester LXVII,

LXVII

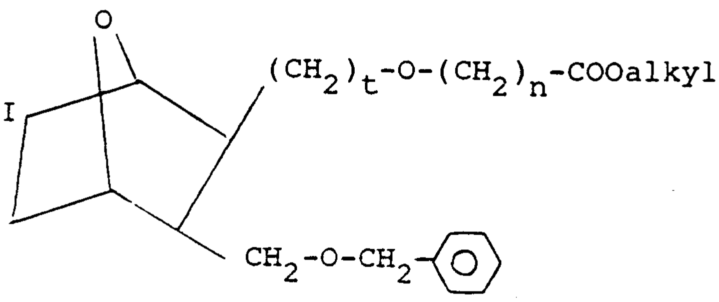

(wherein t is 2 or 1)

Ester LXVII is then subjected to hydrogenolysis by reacting ester LXVII with hydrogen in the presence of a catalyst such as palladium on carbon, platinum oxide and the like to form the alcohol LXVIII

LXVIII

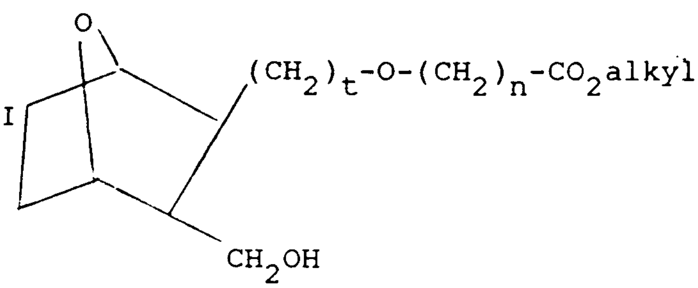

(wherein t is 2 or 1)

Other compounds of the invention within the scope of formula I may be prepared from alcohol LXVIII as follows.

The alcohol LXVIII is subjected to an ether formation reaction wherein LXVIII is reacted with a strong base such as KOH, NaOH or LiOH in the presence of an inert organic solvent such as xylene, toluene, benzene or mesitylene, and then after partial removal of solvent, reacting with a sulfonate compound of the structure

C Mesyl-$OR^2$ or

C' Tosyl-$OR^2$

or a halide of the structure

C'' $R^2Hal$

to form the ether LXIX

LXIX

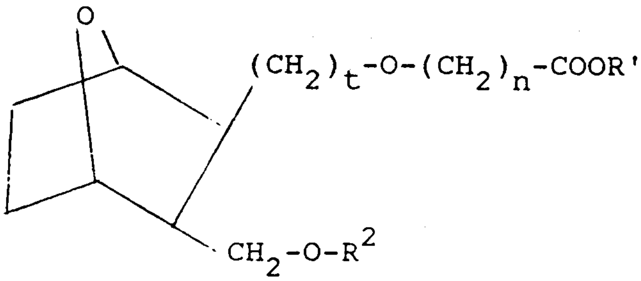

Ether LXIX is then hydrolyzed by treating with strong aqueous base such as LiOH, KOH or NaOH to form the corresponding alkali metal salt and then acidifying with a strong acid such as HCl or oxalic acid to form LXX

LXX

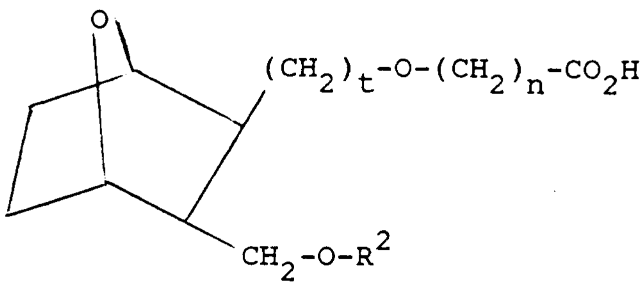

Acid LXX is then subjected to hydroxamate formation by treating a solution of LXX in an inert aromatic solvent such as benzene with oxalyl chloride and stirring the mixture at room temperature under nitrogen to form the acid chloride LXXI

LXXI

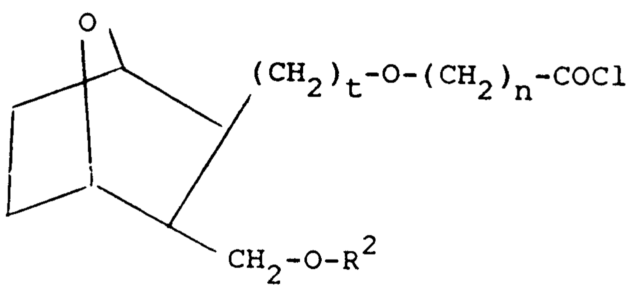

The acid chloride LXXI is dissolved in an inert solvent such as tetrahydrofuran and added to a cold solution of IXA

IXA

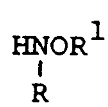

in tetrahydrofuran and water in the presence of organic base such as triethylamine. The mixture is stirred under nitrogen atmosphere while being cooled in an ice bath, to form hydroxamate IQ (where t is 2) or IR (where t is 1).

Compounds of formula I wherein X is S, Q is $(CH_2)_t$ and t is 2, n is 1 to 8, Y is O and p is 1, that is

IS

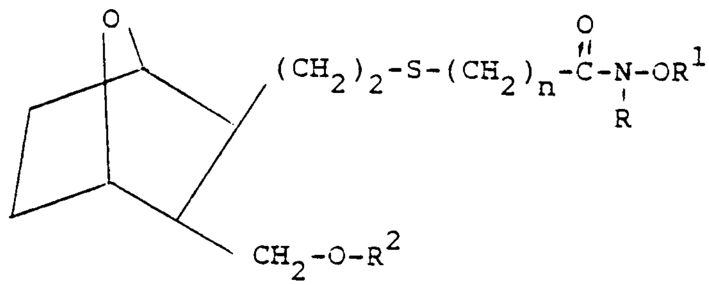

may be prepared starting with aldehyde V which is treated with lithium aluminum hydride or other reducing agent such as sodium borohydride to form the hydroxyethyl compound LXXII

LXXII

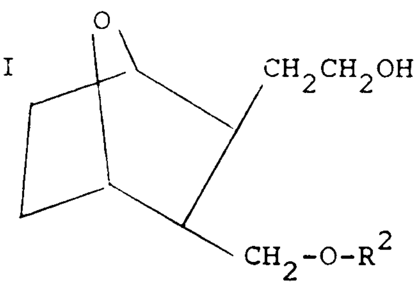

Hydroxyethyl compound XXI is used to make hydroxamate IS as follows.

Triphenylphosphine $((C_6H_5)_3P)$ is suspended in an inert solvent such as tetrahydrofuran, under a nitrogen atmosphere at a reduced temperature of from about 0° to about 20°C, and the suspension is treated with diisopropylazadicarboxylate. After 20 to 60 minutes, the suspension is treated with alcohol LXXII and thiolacetic acid in a dry solvent such as tetrahydrofuran to form thioacetate LXXIII

LXXIII

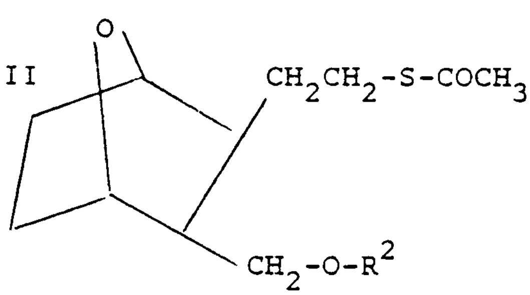

Thioacetate LXXIII is treated with lithium aluminum hydride under a nitrogen atmosphere at temperatures of from about 0 to about 20°C to form the thiol LXXIV

LXXIV

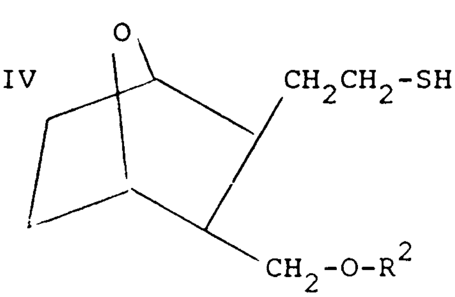

Thiol LXXIV is then treated with halo compound F'

F' $Hal(CH_2)_n-COOCH_3$

and base such as potassium carbonate in a dry inert solvent such as acetone under a nitrogen atmosphere to form ester LXXV

LXXV

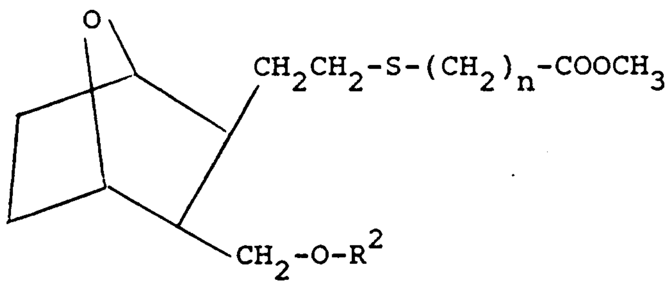

The ester LXXV is then hydrolyzed to the corresponding acid by treatment with lithium hydroxide and the acid is converted to hydroxamate IS employing procedures as set out hereinbefore.

Compounds of formula I wherein A is S, Q is $CH_2$, n is 1 to 8, X is O and p is 1, that is

IT

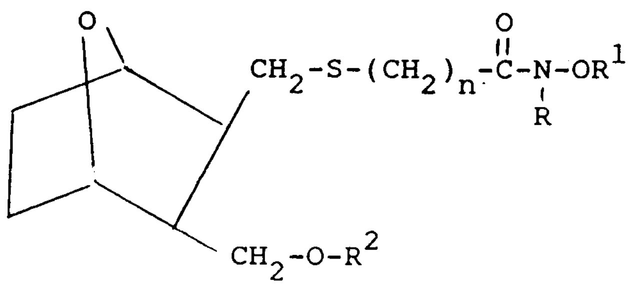

may be prepared by subjecting alcohol LXXVI

LXXVI

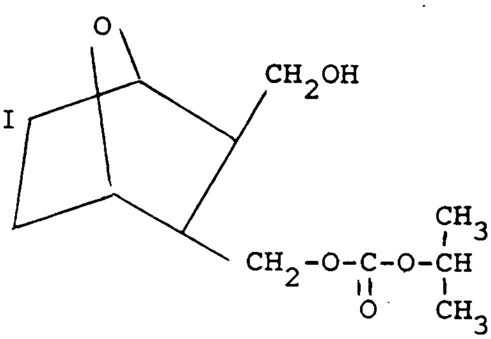

(prepared as described in U. S. Patent No. 4,515,972).

to a modified Mitsunobu reaction wherein a mixture of the alcohol LXXVI and thiolacetic acid in an inert solvent such as tetrahydrofuran, ether or toluene is reacted with a mixture of triphenylphosphine and diisopropylazadicarboxylate in an inert organic solvent such as tetrahydrofuran, ether or toluene at temperatures of from about 0 ° C to about 100 ° C to form thioacetate LXXVII.

LXXVII

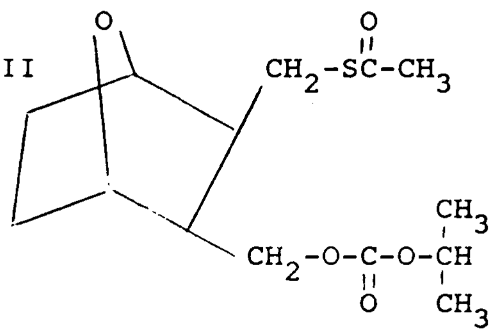

which is then reduced by treating with lithium aluminum hydride or diborane in the presence of an inert organic solvent such as tetrahydrofuran or other solvent such as ether to form the thiol LXXVIII

LXXVIII

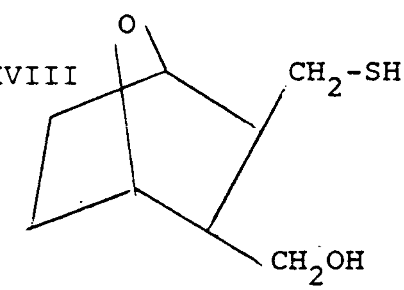

Thiol LXXVIII is then alkylated by reacting with alkylating agent J'

J' Hal-$(CH_2)_n$-$CO_2$alkyl

as described above with respect to thiol LXXV to form ester alcohol LXXIX

LXXIX

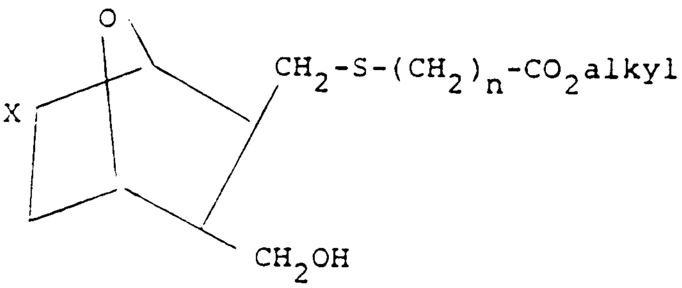

Ester alcohol LXXIX is then treated with reactant C, C' or C", in the presence of a strong base such as potassium hydroxide and an inert solvent such as xylene to form ether LXXX

LXXX 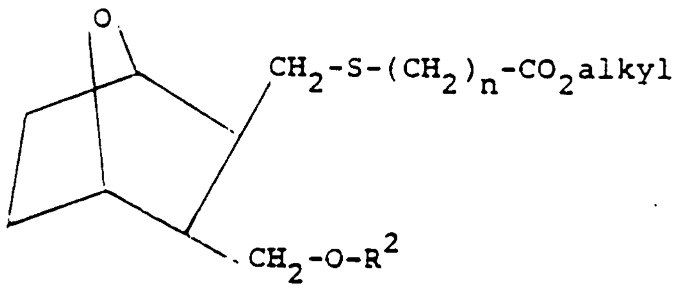

which is then hydrolyzed by reaction with lithium hydroxide as described above to form acid LXXXI

LXXXI 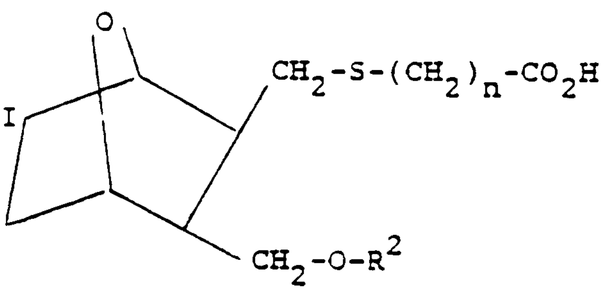

Acid LXXXI is then converted to the corresponding hydroxamate IT in a manner as described hereinbefore

Compounds of formula I wherein Y is O, X is S or O, Q is $(CH_2)_t$ and t is 1 or 2, and p is 1 may be prepared by starting with the hydroxymethyl compound LXVIII

LXVIII 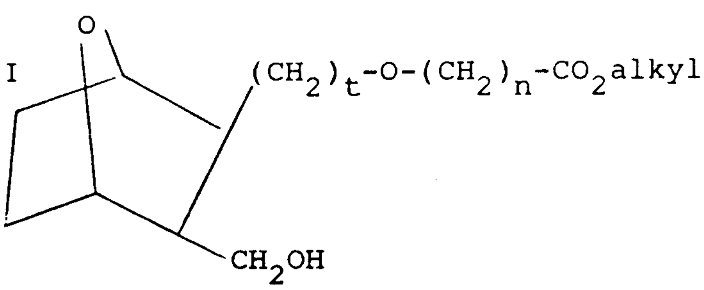

(wherein m is 2 or 1)

compound LXXXII

LXXXII

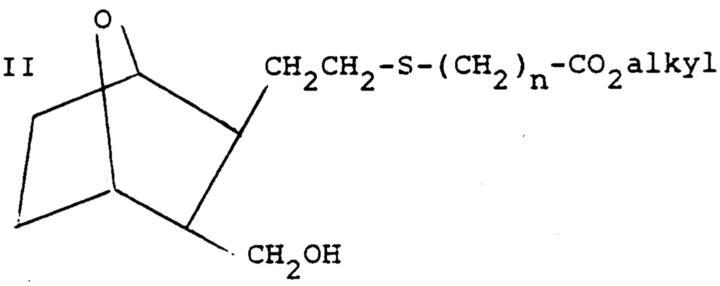

or

LXXIX

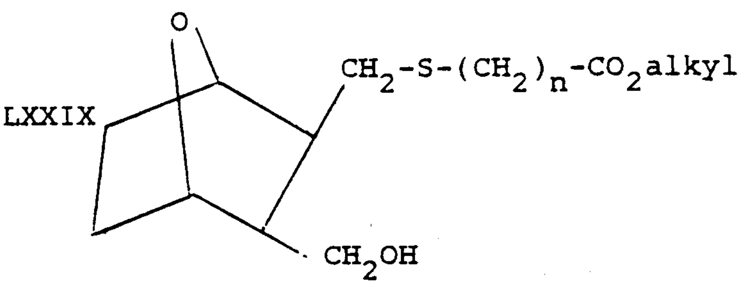

and subjecting one of the above hydroxymethyl compounds to a tosylation reaction, for example, by reacting the hydroxymethyl compound with tosyl chloride in pyridine and methylene chloride to form the corresponding tosylate LXXXIII

LXXXIII

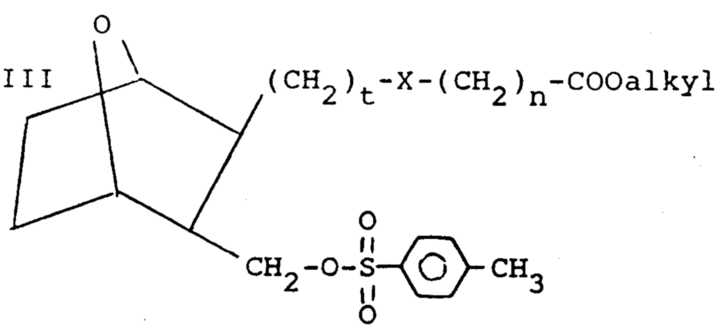

Thereafter, tosylate XXXIII is reacted with a thiol or mercaptan of the structure L

L HS$R^2$

in the presence of potassium t-butoxide and a solvent such as tetrahydrofuran, dimethyl sulfoxide or dimethylformamide to form thioether ester compounds of the structure LXXXIV. Alternatively, compounds of the type LXVIII, LXXXII or LXXIX may be reacted with triphenylphosphine, a dialkylazadicarboxylate like

diethylazadicarboxylate and a mercaptan of structure L to form thioether ester compounds of the structure LXXXIV

LXXXIV

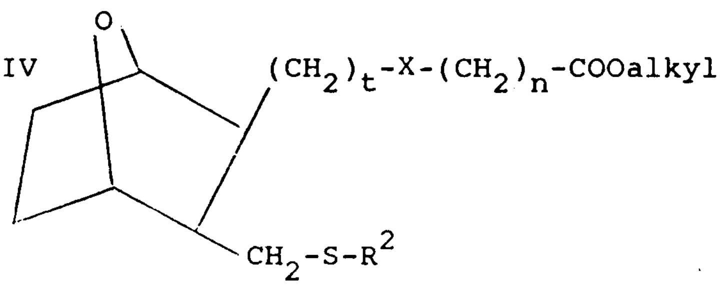

Ester compounds LXXXIV may be hydrolyzed to the corresponding acids LXXXV employing procedures as described herebefore.

LXXXV

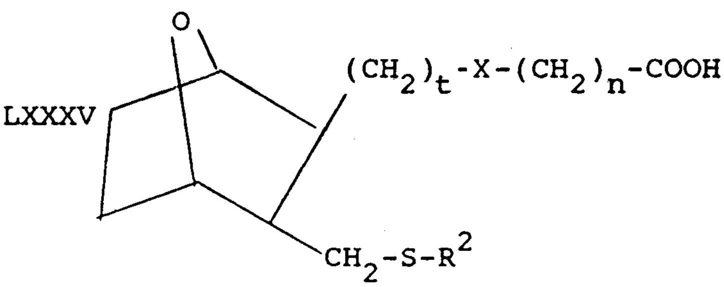

Intermediate compound LXXXII, that is,

LXXXII

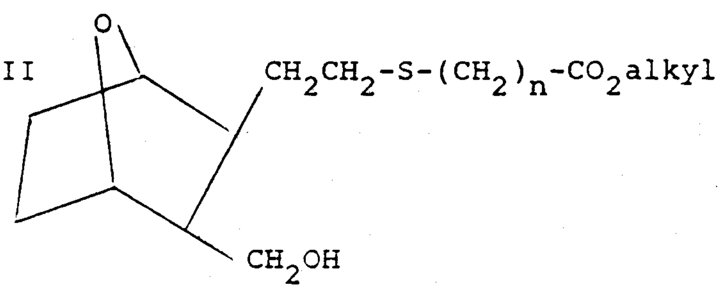

may be prepared as follows. Cyanoalcohol XXI is subjected to a silylation wherein compound XXI is reacted with a t-butyldimethylsilyl chloride having the structure M

M

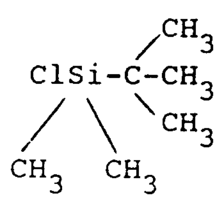

in the presence of dry dichloromethane and triethylamine and 4-dimethylaminopyridine to form the silyl ether LXXXVI

LXXXVI

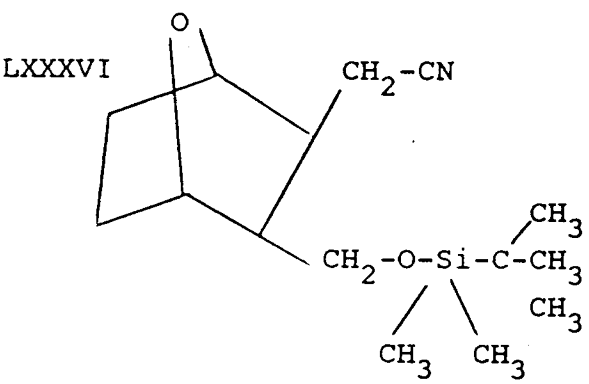

which is reduced by treating with a reducing agent such as diisobutylaluminum hydride, in the presence of an inert organic solvent such as toluene, tetrahydrofuran or methylene chloride in an inert atmosphere, at reduced temperatures of from about -78 ° C to about 0 ° C to form the aldehyde LXXXVII

LXXXVII

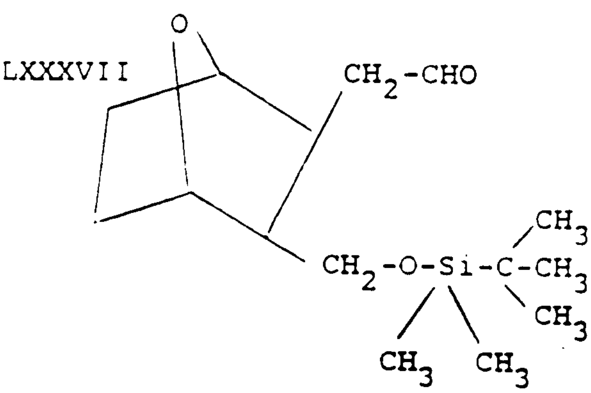

The aldehyde LXXXVII is further reduced by treatment with a reducing agent such as lithium aluminum hydride, sodium borohydride or lithium borohydride in the presence of an organic solvent such as tetrahydrofuran, ethanol or ether to form the alcohol LXXXVIII

LXXXVIII

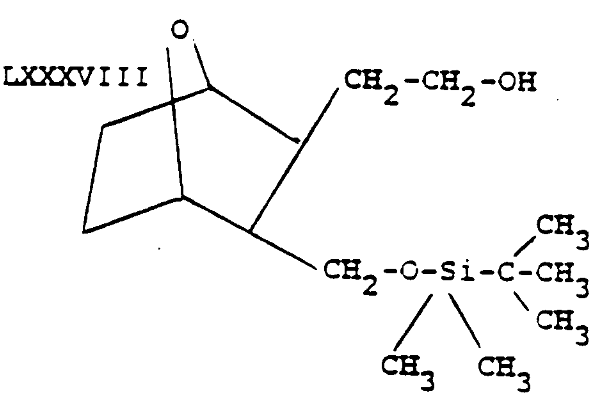

The alcohol LXXXVIII is then subjected to a modified Mitsunobu reaction wherein a mixture of the alcohol and thiolacetic acid in an inert solvent such as tetrahydrofuran, ether or toluene is reacted with a mixture of triphenylphosphine and diisopropylazo dicarboxylate in an inert organic solvent such as tetrahydrofuran, ether or toluene at temperatures of from about 0°C to about 100°C to form thioacetate LXXXIX

LXXXIX

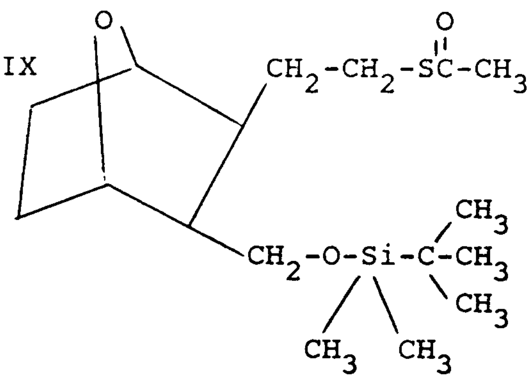

The silyl group is removed from thioacetate LXXXIX by reacting B with tetra-n-butylammonium fluoride trihydrate in the presence of an inert organic solvent such as tetrahydrofuran or ether to form alcohol thioacetate XC

XC

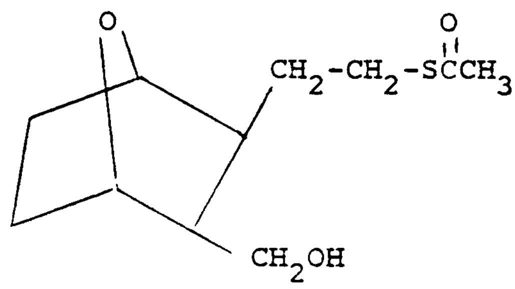

which is then deacetylated by treating with lithium aluminum hydride or bases like potassium carbonate or sodium methoxide in the presence of an inert organic solvent such as tetrahydrofuran or methanol to form thiol XCI

XCI

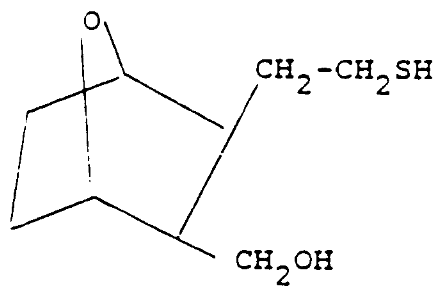

The thiol XCI is then alkylated by reacting same with an alkylating agent of the structure J'

J' Hal-$(CH_2)_n$-$CO_2$alkyl

in the presence of a base such as sodium or potassium hydride or potassium carbonate and an inert organic solvent such as acetone, THF or DMF, at reduced temperatures of from about 0°C to about 50°C, to form alcohol LXXXII.

Compounds of formula I wherein Q is $CH_2$ and p is 2 to 5 may be prepared by subjecting aldehyde XCII

XCII

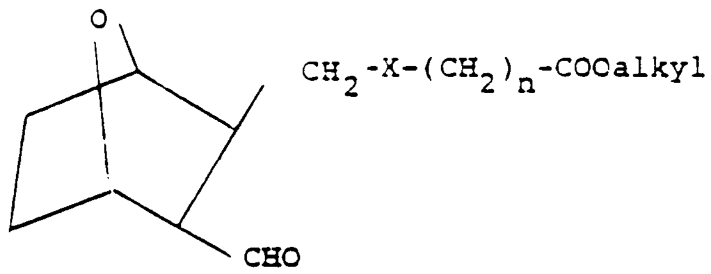

wherein X is O or S

to a homologation sequence, such as a Wittig reaction with $(C_6H_5)_3P^+Cl^-CH_2OCH_3$ followed by hydrolysis, (p-1) times, to form aldehyde XCIII

XCIII

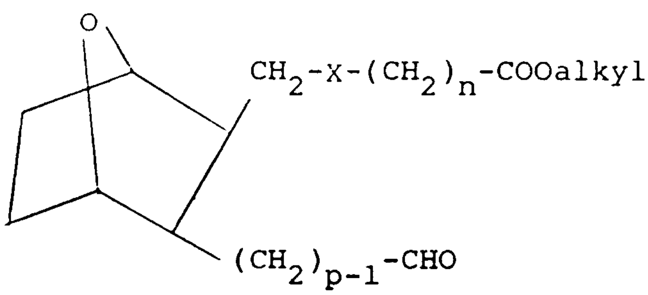

which is carried on to compounds of the invention where p is 2 to 5 by reducing aldehyde XCIII employing a reducing agent such as sodium borohydride in a solvent such as methanol to form alcohol ester XCIV

XCIV

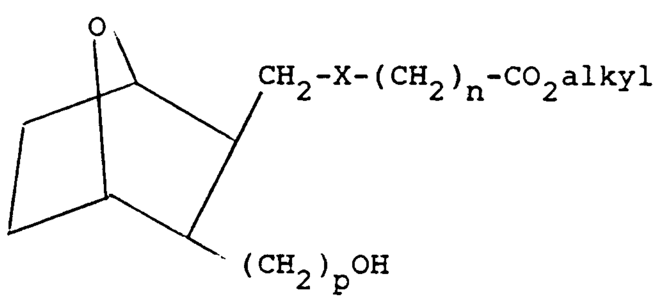

which is subjected to an etherification reaction with C, C' or C'' as described above or to a thioetherification reaction with thiol L to form XCV

XCV

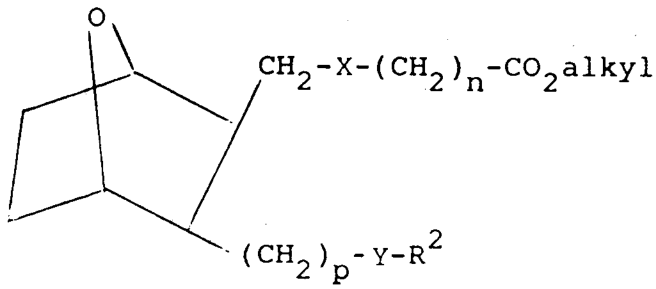

which may be hydrolyzed to the corresponding acid XCVA employing procedures as set out hereinbefore.

Aldehyde XCII wherein X is O may be prepared from alcohol LXVIII (wherein X is O) by carrying out a Collins oxidation wherein alcohol LXVIII is reacted with a complex of $CrO_3$ with organic base such as pyridine, in dichloromethane or with pyridinium chlorochromate in a solvent such as methylene chloride.

Aldehyde XCII wherein X is S may be prepared from alcohol LXXIX (wherein X is S) by subjecting such alcohol to a Corey-Kim oxidation wherein the appropriate alcohol in toluene is added to a mixture of dimethylsulfide and N-chlorosuccinimide in dry toluene or other inert organic solvent such as methylene chloride, and the mixture is stirred at 0°C and then cooled to -25°C. After stirring at -25°C, triethylamine is added and the mixture is then warmed and purified to give aldehyde XCII.

Ether intermediate compounds wherein Q is $(CH_2)_2$ and p is 2, 3, 4, or 5 may be prepared by oxidizing alcohol LXVIII (wherein X is O) via a Collins oxidation technique as described hereinbefore or by oxidizing alcohol LXXIX (wherein X is S) via a Corey-Kim oxidation or Swern oxidation as described hereinbefore to form aldehyde XCVI

XCVI

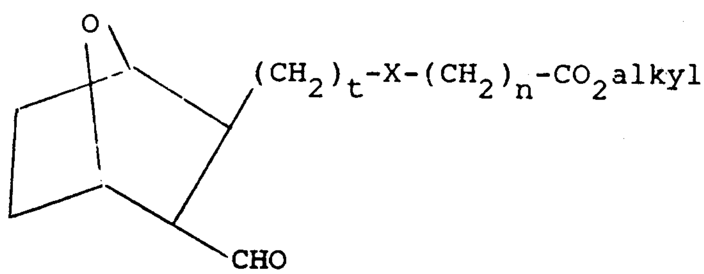

Aldehyde XCVI is then subjected to a homologation sequence such as a Wittig reaction with $(C_6H_5)_3P^+Cl^-CH_2OMe$ followed by hydrolysis, (p-1) times, to form aldehyde XCVII

XCVII

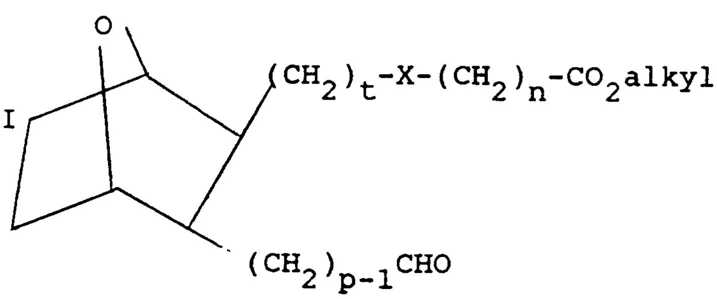

Aldehyde XCVII is then reduced to the corresponding alcohol XCVIII

XCVIII 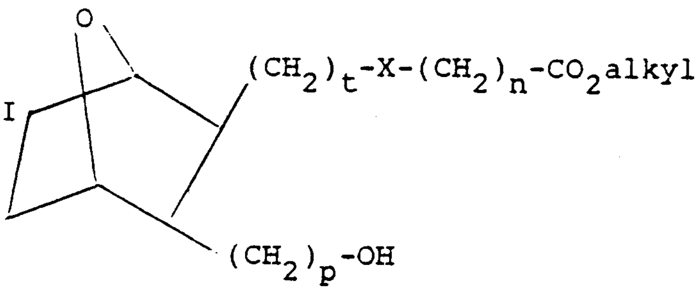

by reacting XCVIII with sodium borohydride in a solvent like methanol, ethanol or tetrahydrofuran. The alcohol XLVIII may then be subjected to an etherification reaction with C, C' or C'' or to a thioetherification reaction with L to form the compounds of the invention.

Ether intermediate compounds of formula I wherein $R^2$ is aryl such as phenyl or substituted phenyl may also be prepared by reacting the alcohol XXVII, XLIII, LI, XL, LXA, LXIII, LXVIII, LXXXII, LXXIX, XCII or XCIV with triphenylphosphine and diethylazodicarboxylate in solution with an inert solvent such as THF, and thereafter without isolating any products, reacting the above reaction mixture with an aryl alcohol wherein the hydroxy group is directly attached to the aromatic ring, such as phenol or a substituted phenol, under an inert atmosphere, such as argon or nitrogen, to form the ester of the structure XCIX

XCIX 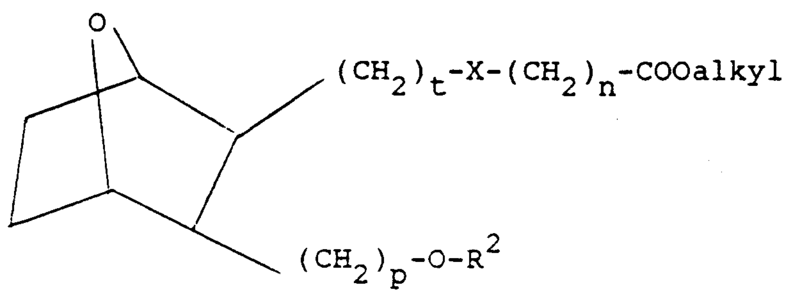

(wherein $R^2$ is phenyl or substituted phenyl).

The above esters can be converted to the free acid, that is, to P

P 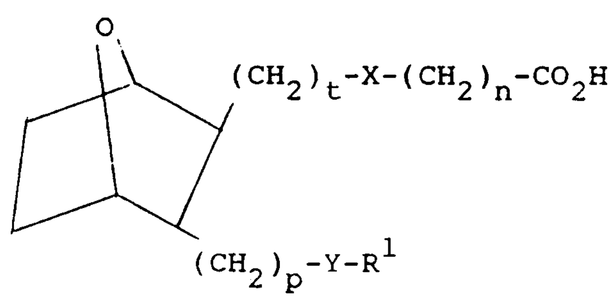

by treating the esters with an alkali metal hydroxide, such as lithium or sodium hydroxide to form the corresponding alkali metal salt (wherein R is an alkali metal such as Na, Li or K) followed by acidification with an acid, such as dilute hydrochloric acid or oxalic acid to form the acid L.

The acids XXXV, XLVA and P may then be converted to the corresponding hydroxamates employing procedures disclosed hereinbefore.

To form compounds of formula I wherein Y is

$$\underset{O}{\underset{\|}{S}} \text{ and } \overset{O}{\overset{\|}{\underset{O}{\underset{\|}{S}}}},$$

the sulfide derivative of formula I wherein Y is S and X is O is subjected to an oxidation reaction, for example, by reacting same with aqueous sodium periodate, in the presence of water, methanol or tetrahydrofuran, to form the corresponding sulfinyl derivative

$$(\underset{O}{\underset{\|}{S}})$$

and sulfonyl derivative

$$(\overset{O}{\overset{\|}{\underset{O}{\underset{\|}{S}}}}).$$

Where X is S and Y is S, oxidation of S in the lower side chain will cause similar oxidation of S in the upper side chain. The sulfinyl and sulfonyl derivatives may be separated by chromatography or other conventional separation procedures.

The compounds of this invention have four centers of asymmetry as indicated by the asterisks in formula I. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis-exo, cis-endo and all trans forms and stereoisomeric pairs may be prepared as shown in the working Examples which follow and by employing starting materials and following the procedures as outlined in U. S. Patent No. 4,143,054.

Examples of such stereoisomers are set out below.

Ia

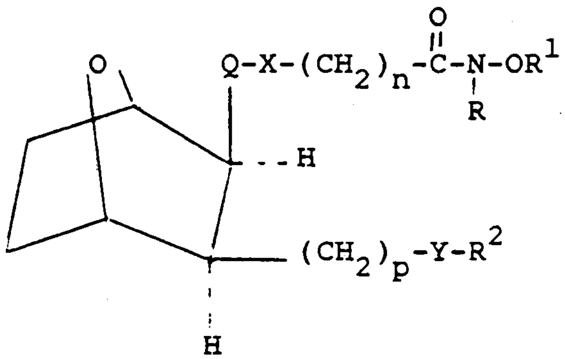

(cis-endo)

Ib

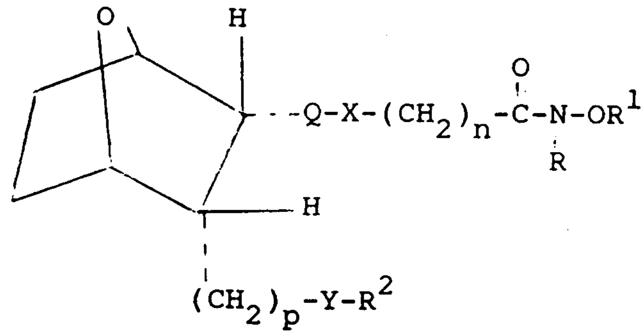

(cis-exo)

Ic

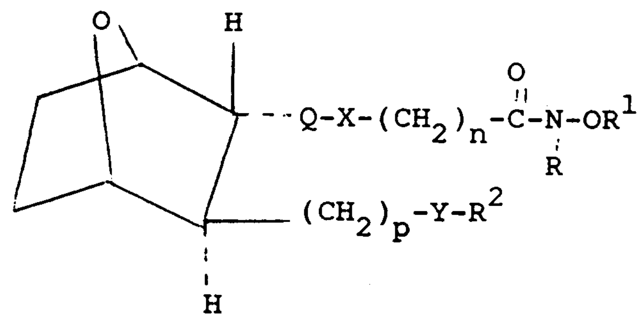

(trans)

Id

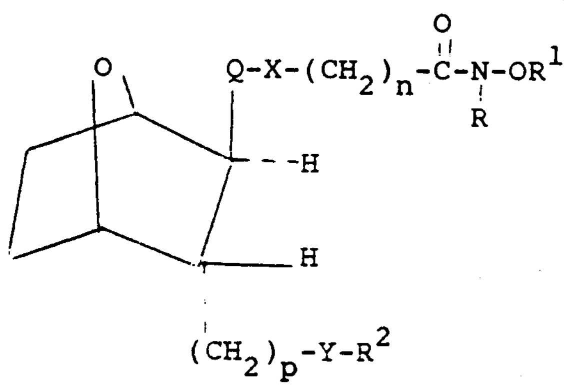

(trans)

The nucleus in each of the compounds of the invention is depicted as

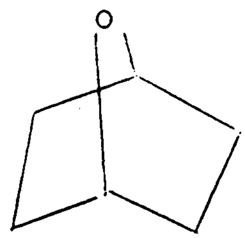

for matter of convenience; it will also be appreciated that the nucleus in the compounds of the invention may be depicted as

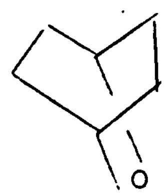

It will also be understood that the position of the bonds in the above nuclei are not intended to identify particular isomers but are intended to generally depict all isomers.

The compounds of this invention are cardiovascular agents useful as platelet aggregation inhibitors, such as inhibiting arachidonic acid-induced platelet aggregation (e.g., for treatment of thrombotic disease, such as coronary or cerebral thromboses) and in inhibiting bronchoconstriction as induced by asthma. They are also selective thromboxane $A_2$ receptor antagonists and synthetase inhibitors, e.g., having a vasodilatory effect for treatment of myocardial ischemic disease, such as angina pectoris. In addition, the compounds of the invention are thromboxane synthetase inhibitors and they may also be used for preventing gastrointestinal ulcer formation. They also increase the amount of endogenous prostacyclin and therefore may be used for controlling tumor cell metastasis or as antihypertensive agents.

The compounds of the invention are also arachidonic acid cyclooxygenase inhibitors. In addition, the compounds of the invention are useful as analgesic agents in the manner of aspirin and indomethacin as indicated by reaction thresholds to pressure in edematous hindpaws [Ref: Winter et al, J. Pharmacol, Exp. Ther. 150:165, 1965] and as antiinflammatory agents in mammals, as indicated by carrageenin-induced edema in the rat [Ref: Winter et al., J. Pharmacol., Exp. Ther. 141:369, 1963]. They may be used to decrease joint swelling, tenderness, pain and stiffness in conditions such as rheumatoid arthritis.

The compounds of this invention may also be used in combination with a cyclic AMP phosphodiesterase (PDE) inhibitor such as theophylline or papaverine in the preparation and storage of platelet concentrates.

In addition, the compounds of the invention are $\Delta^5$-lipoxygenase inhibitors and prevent prostaglandin and leukotriene $C_4$ formation in macrophages (Samuelsson, B., Science, Vol. 220, p. 568-575, 1983). The administration of compounds of this invention to humans or animals provides a method for treating allergy of a reagin or non-reagin nature. Asthma is preferably treated but any allergy wherein leukotrienes are thought to be involved as pharmacological mediators of anaphylaxis can be treated. For example, the compounds of this invention can be used for treatment of such conditions as allergic rhinitis, food allergy and urticaria as well as asthma. In addition, the compounds of the invention are useful as antipsoriatic agents.

The compounds of the invention can be administered orally or parenterally to various mammalian species known to be subject to such maladies, e.g., humans, cats, dogs, and the like in an effective amount within the dosage range of about 0.1 to 100 mg/kg, preferably about 1 to 50 mg/kg and especially about 2 to 25 mg/kg on a regimen in single or 2 to 4 divided daily doses.

The compounds of the invention may also be administered topically to any of the above mammalian species in amounts of from about 0.1 to 10 mg/kg in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional manner with a physiologically acceptable vehicle or carrier, excipient, binder, preservative, stabilizer, flavor, etc. as called for by accepted pharmaceutical practice. Also as indicated in the discussion above, certain members additionally serve as intermediates for other members of the group.

An effective but essentially non-toxic quantity of the compound is employed in treatment.

The following Examples represent preferred embodiments of the present invention. Unless otherwise indicated, all temperatures are expressed in degrees Centigrade.

Example 1

[1α,2β(Z),3β,4α]-2-[[4-[3-[(Hexyloxy)methyl]-7-oxabicyclo[2.2.1]hept-2-yl]-2-butenyl]oxy]-N-hydroxy-N-methylacetamide

A. (1α,2β,3β,4α)-3-[(Hexyloxy)methyl]-7-oxabicyclo[2.2.1]hept-2-ylacetaldehyde

(1) (1α,2β,3β,4α)-exo-7-oxabicyclo[2.2.1]heptane-2,3-dimethanol

To a suspension of 11.4 g lithium aluminum hydride (300 mmole) in 400 ml of dry THF at 0°C was added dropwise a solution of 32 g cis-exo 7-oxabicyclo[2.2.1]heptane-2,3-dicarboxylic anhydride (prepared as described in U. S. Patent No. 4,143,054) (190 mmole) in 400 ml of dry THF over a period of 1 hour. The reaction mixture was stirred at 25°C for 18 hours, cooled to 0°C and quenched by slow addition of a saturated $Na_2SO_4$ solution, and filtered. The solid was washed with three 100 ml portions of $CH_2Cl_2$. The combined organic layer was dried over $MgSO_4$ and concentrated to give 32 g of title diol as a colorless solid.

(2) (1α,2β,3β,4α)-3-[(Hexyloxy)methyl]-7-oxabicyclo[2.2.1]heptane-2-methanol

A suspension of 50% sodium hydride (16.7 g of 0.35 mmole; prewashed with ether) in dry dimethylformamide (350 ml) was cooled down to 0° under $N_2$ and treated dropwise with a solution of the Part (1) diol (50 g.; 0.316 mole) in dry dimethylformamide (150 ml). The reaction mixture was stirred at 0° for 30 minutes and at room temperature for 30 minutes after which n-hexylbromide (59.8 ml or 70.3 g; 0.42 mmole) was added. The mixture was then stirred at room temperature for 15 minutes, at 120° (oil bath) for 15 hours, cooled and quenched with a 25% ammonium chloride solution (300 ml). The resulting suspension was extracted three times with ether (1.0 liter), the organic extracts were dried (anhydrous $MgSO_4$), filtered and evaporated to a syrup (90.0 g). The crude product mixture was chromatographed (gravity) on a silica gel column (Woelm; 1.2 kg), eluting the column with EtOAc-hexane (1:4, 24.3 liters). The desired fractions were combined and evaporated to give 26.94 g of homogeneous (tlc) title compound. An additional 28.7 g of the desired compound containing a trace of another component was obtained from other fractions giving a total yield of 72.6%. An analytical sample was obtained by distilling 1.0 g of material on a Buchi GKR-50 apparatus, at 225° and 0.4 mm.

$^1$H-NMR (270 MHz, $CDCl_3$):
δ 0.89 (t, 3H, J = ~8, $H_{21}$)
1.29-1.7 (m, 12H)
2.2 (m, 2H, J = ~4, $H_8$ + $H_{13}$)
3.3-3.80 (m, 7H, --, $H_7$, $H_{14}$ + $H_{16}$)
4.23 (d, 1H, J = ~2, $H_9$)
4.29 (d, 1H, J = ~2, $H_{12}$) ppm

| | | |
|---|---|---|
| Anal Calcd for $C_{14}H_{26}O_3$: | C, 69.38; | H, 10.81 |
| Found: | C, 69.36; | H, 10.60 |

(3) (1α,2β,3β,4α)-2-Chloromethyl-3-[(hexyloxy)methyl]-7-oxabicyclo[2.2.1]heptane

5.0 g (20.6 mole) of (1α,2β,3β,4α)-3-(hexyloxy)methyl-7-oxabicyclo[2.2.1]heptane-2-methanol, 4.73 g (24.8 mmole) of p-toluenesulfonyl chloride, 873 mg (20.6 mmole) of lithium chloride and 3.3 ml of dry pyridine were stirred together in dichloromethane (15 ml) at room temperature under nitrogen for 24 hours. The reaction mixture was partitioned between ether (250 ml) and saturated sodium chloride solution (20 ml). The aqueous phase was re-extracted with ether (250 ml), the combined organic extracts were dried (anhydrous $MgSO_4$), filtered and the clear filtrate was evaporated down to a syrup (5.3 g). The crude product was flash chromatographed on a silica gel column (LPS-1), eluting the column with $Et_2O$:hexane (1:9, 6.0 liters) and $Et_2O$:hexane (1:1, 6.0 liters). The fractions containing the desired product were combined and evaporated to give 3.35 g (62.4%) of the title chloro compound as a homogeneous (tlc) oil with consistent $H^1$ and $C^{13}$ spectral data.

(4) (1α,2β,3β,4α)-2-Cyanomethyl-3-[(hexyloxy)methyl]-7-oxabicyclo[2.2.1]heptane

A solution of Part (3) compound (3.35 g, 12.8 mole) and sodium cyanide (1.29 g) in dry dimethylsulfoxide (4.6 ml) was heated at 90-95° (oil bath) under argon for 19 hours with stirring. The mixture was cooled to room temperature, diluted with water (12 ml) and extracted twice with ether (75 ml). The organic extracts were dried (anhydrous $MgSO_4$), filtered and the clear filtrate concentrated in vacuo to a light yellow oil (3.18 g).

This oil was flash chromatographed on a silica gel column (LPS-1), eluting the column with $Et_2O$:Hexane (1:2, 7.5 liters). The desired fractions were combined and concentrated to give 3.06g(95%) of the title cyano compound as a homogeneous (tlc) light yellow oil with consistent $H^1$ and $C^{13}$-NMR spectral data.

(5) (1α,2β,3β,4α)-3-([Hexyloxy)methyl]-7-oxabicyclo[2.2.1]hept-2-ylacetaldehyde

1.5 g (5.97 mmole) of the Part (4) cyano compound was dissolved in dry toluene (7.0 ml), cooled, stirred in a bath at -78° (dry ice-acetone) under argon, and treated dropwise with 5.4 ml of diisobutylaluminum hydride (25% by wt. in toluene; 9.49 mmole or 1.5 eq.). After 4 hours, the mixture was quenched at -78° with 25% $NH_4Cl$ (6.0 ml), stirred for 30 minutes, warmed to about 0°, acidified with 1N HCl (16 ml), and stirred for about 30 minutes. The mixture was then extracted twice with dichloromethane (50 ml), the organic extracts were washed with saturated sodium chloride solution, (20 ml), dried (anhydrous $MgSO_4$), filtered and concentrated in vacuo to give 1.45 g (95.4%) of the title A aldehyde as a homogeneous (tlc) yellow oil with consistent $H^1$ and $C^{13}$-NMR spectral data.

B. [1α,2β(Z),3β,4α]-4-[3-[(Hexyloxy)methyl]-7-oxabicyclo[2.2.1]hept-2-yl]-2-butenoic acid, methyl ester [Wadsworth-Emmons reaction - W. C. Still, C. Gennari, Tetrahedron Letters, 26, No. 41, 4405 (1983)]

A solution of bis(2,2,2-trifluoroethyl) (methoxycarbonyl methyl)phosphonate(1.3 ml, 6.02 mmole) and 18-crown-6 (5.0 g, 19 mmole) in dry THF (80 ml) was stirred in a bath at -78° under argon and 0.6M potassium hexamethyl silyl amide in THF (5.1 mmole, 8.5 ml), was added in the course of 3 minutes. After 15 minutes, a solution of the Part A aldehyde (1.0 g, 3.94 mmole) in dry THF (15 ml) was added. After 4 hours, the reaction was quenched by the addition of 25% $NH_4Cl$ (35 ml). The mixture was then concentrated in vacuo to remove most of the THF, and was extracted with $Et_2O$ (2 x 100 ml). The extracts were combined, washed with brine, dried ($MgSO_4$ anhydrous) and evaporated to afford the crude product as an oil.[1] This was subjected to flash chromtography (silica gel, LPS-1) eluting the column with hexane-EtOAc (9:1) to isolate pure cis double bond compound (860 mg, 70.4%), a mixture of cis- and trans double bond compounds (110 mg, 9.0%) and pure trans double bond compound (130 mg, 10.6%).

C. [1α,2β(Z),3β,4α]-4-[3-[(Hexyloxy)methyl]-7-oxabicyclo[2.2.1]hept-2-yl]-2-butenol

A solution of Part B ester (1.0 g, 3.22 mmole) in dry toluene (15 ml) was cooled and stirred in a bath at -78° under argon and a 1.5M solution of diisobutyl aluminum hydride in toluene (6.5 ml) was added. After 4.0 hours, the mixture was added under stirring into 10% HCl (25 ml) and extracted with ether (3x50 ml).

[1] The $H^1$-NMR spectrum of this showed the presence of 16.4% trans and 86.3% cis double bond products.

The extracts were combined, washed with dilute brine, dried ($MgSO_4$ anhydrous) and evaporated to afford the homogeneous (tlc; silica gel, $Et_2O$) title compound as an oil (900 mg, 99.7%) with consistent $H^1$- and $C^{13}$-NMR spectral data. This specimen was used in the next step without further purification.

D. [1a,2β(Z),3β,4α]-2-[[4-[3-[(Hexyloxy)methyl]-7-oxabicyclo[2.2.1]hept-2-yl]-2-butenyl]oxy]acetic acid, t-butyl ester

A mixture of Part C alcohol (900 mg, 3.2 mmole), t-butylbromoacetate (5.43 ml), tetrabutyl ammonium sulfate (1.7 g), THF (20 ml) and 50% NaOH (20 ml) was stirred vigorously under argon for 2.5 hours. Most of the THF was then removed in vacuo and the concentrate was extracted with $CH_2Cl_2$ (3x30 ml). The extracts wre combined, washed with dilute brine (2x20 ml), dried ($MgSO_4$ anhydrous) and evaporated to afford the crude product as an oil. On the basis of tlc, this was a mixture of one major and two minor components in addition to impurities derived from the excess t-butylbromo acetate; the starting alcohol was absent. This mixture was flash chromatographed on a column of silica gel (LPS-1) eluting with EtOAc-hexane (1:9) to isolate homogeneous (tlc) title compound as an oil (1.16 g, 91%) with consistent $H^1$- and $C^{13}$-NMR spectral data.

E. [1α,2β(Z),3β,4α]-2-[[4-[3-[(Hexyloxy)methyl]-7-oxabicyclo[2.2.1]hept-2-yl]-2-butenyl]oxy]acetic acid

A solution of Part D t-butyl ester (350 mg, 0.88 mmole) in distilled dioxane (7.0 ml) was stirred under reflux in an atmosphere of nitrogen with 2N LiOH (2.5 ml) for 1.0 hour. The mixture was then cooled to room temperature, acidified with 10% HCl (5.0 ml) and most of the dioxane was removed by concentration in vacuo. The concentrate was diluted with brine (20 ml) and extracted with ether (3x30 ml). The extracts were combined, washed with brine (2x25 ml), dried ($MgSO_4$ anhydrous) and evaporated to afford the product as an oil. This was chromatographed on a column of silica gel (Baker 60-200 mesh, 15 g) eluting the column with hexane, $Et_2O$-hexane (3:7, 1:1), $Et_2O$, $Et_2O$-$CH_3OH$ (1:4, 1:1), and $CH_3OH$. The relevant fractions were combined and evaporated. The residual oil was dissolved in $Et_2O$ (75 ml) and was washed successively with 1.0N HCl (2x10 ml) and brine (2x10 ml) to remove silica gel. The $Et_2O$ solution was then dried ($MgSO_4$ anhydrous), evaporated and dried in vacuo to afford the analytical specimen of title acid as a homogeneous (tlc) oil (235 mg, 78.4%), with consistent MS, IR (1761, 1736 $cm^{-1}$, strong, C=O), $H^1$- and $C^{13}$-NMR data.

| | | |
|---|---|---|
| Anal Calcd for $C_{19}H_{32}O_5$: | C, 67.03; | H, 9.48 |
| Found: | C, 67.04; | H, 9.41 |

$H^1$-NMR spectrum (FX-270, $CDCl_3$):
δ 0.90 (t, 3H, J = ~7.0, $H_{21}$)
2.10 (t, 3H, -, $H_{13}$ + $H_7$)
3.43 (m, 4H, -, $H_{14}$ + $H_{16}$)
4.10 (s, 2H, -, $H_2$)
4.17 (m, 2H, -, $H_4$)
4.26 (d, 1H, J = ~4.0, $H_9$)
4.40 (d, 1H, J = ~4.0, $H_{12}$)
5.62 (m, 2H, -, $H_5$ + $H_6$) ppm

F. [1α,2β(Z),3β,4α]-2-[[4-[3-[(Hexyloxy)methyl]-7-oxabicyclo[2.2.1]hept-2-yl]-2-butenyl]oxy]-N-hydroxy-N-methylacetamide

A solution of Part E acid (620 mg, 1.82 mmole) and oxalyl chloride (2 ml, 22.5 mmole or 12.4 eq.) in dry benzene (10 ml) was cooled down to 0° (ice-water bath), treated with dry dimethylformamide (3 drops) and stirred at 0° for 30 minutes under nitrogen and at room temperature for 1 hour. The excess oxalyl chloride and solvent were blown off under a stream of nitrogen while heating the flask in a warm water bath and the residual oil was dried in vacuo for 1 hour. This acid chloride was dissolved in dry tetrahydrofuran (3.5 ml) and added dropwise under stirring into a cold solution (~0°, ice-water) of 98% methylhydroxylamine hydrochloride (318.7 mg, 3.74 mmole) and triethylamine (0.92 ml, 7.48 mmole) in tetrahydrofuran (4.6 ml) and water (4.6 ml). The mixture was stirred at 0° for 30 minutes, diluted with water (25 ml) and

extracted twice with dichloromethane (125 ml). The combined organic extracts were washed with 1N HCl (25 ml), 5% $NaHCO_3$ (12 ml) and brine (20 ml), dried (anhydrous $MgSO_4$), filtered and evaporated to dryness giving an oil (720 mg) containing the desired product and traces of a less polar component.

This mixture was dissolved in ether (50 ml) with warming, concentrated down to a volume of ~15 ml and diluted with hexane (30 ml). The white precipitates obtained on scratching provided a homogeneous (TLC) specimen of title product[1] (524.2 mg, 77.9%) with consistent elemental analysis, MS, IR (1654 and 1636 $cm^{-1}$, strong, C=O), $H^1$- and $C^{13}$-NMR data.

$H^1$-NMR Spectrum (FX-270, $CDCl_3$)
δ 0.89 (t, 3H, J = ~7, $H_{21}$)
1.20-2.21 (m, 16H, -, -)
3.20-3.44 (m, 7H, -, $H_{14}$ + $H_{16}$ + $H_{22}$)
3.95-4.5 (m, broad, 6H, -, $H_2$ + $H_4$ + $H_9$ + $H_{12}$)
5.55-5.72 (m, broad, 2H, -, $H_5$ + $H_6$)
8.68 (S, broad, 1H, -, N-OH) ppm

Example 2

(1α,2β,3β,4α)-2-[[4-[3-[(Hexyloxy)methyl]-7-oxabicyclo[2.2.1]hept-2-yl]butyl]oxy]-N-hydroxy-N-methylacetamide

A. (1α,2β,3β,4α)-4-[3-[(Hexyloxy)methyl]-7-oxabicyclo[2.2.1]hept-2-yl]butanol

(1) (1α,2β,3β,4α)-4-[3-[(Hexyloxy)methyl]-7-oxabicyclo[2.2.1]hept-2-yl]-2-butenoic acid, methyl ester

420 mg (8.75 mmole) of 50% NaH on paraffin was suspended in dry distilled tetrahydrofuran (60 ml), cooled down to 0° under argon and was treated dropwise with trimethylphosphonoacetate (2 ml; 12.4 mmole). The thick slurry was stirred at 0° for 30 minutes, room temperature for 1 hour and cooled back down to 0°. It was then treated dropwise with a solution of Example 1 Part A aldehyde (2.0 g; 7.86 mmole) in tetrahydrofuran (20 ml). The mixture was stirred at 0° for 30 minutes at room temperature for 2 hours and was then acidified with glacial acetic acid (2.0 ml). It was then stirred for 30 minutes, evaporated to dryness in vacuo and the resulting solid was partitioned twice between saturated $NaHCO_3$ solution (100 ml) and ether (400 ml). The organic phase was washed with water (200 ml), dried (anhydrous $MgSO_4$), filtered through a bed of silica gel (Baker; 30 ml), and was evaporated to dryness to give 2.48 g (100%) of title methyl ester as an oil with a consistent $^{13}C$ NMR spectrum. It was a mixture of cis and trans double bond isomers.

(2) (1α,2β,3β,4α)-4-[3-[(Hexyloxy)methyl]-7-oxabicyclo[2.2.1]hept-2-yl]butanoic acid, methyl ester

2.48 g (7.86 mmole) of Part (1) methyl ester was dissolved in dry methanol (140 ml) and hydrogenated at atmospheric pressure, at room temperature, in the presence of 5% Pd/C (420 mg) for 3 to 4 hours. The suspension was filtered and concentrated to give the title compound as a homogeneous (TLC) oil (2.49; 96.6%) with a consistent $^{13}C$ spectrum which showed the absence of the double bond.

(3) (1α,2β,3β,4α)-4-[3-[(Hexyloxy)methyl]-7-oxabicyclo[2.2.1]hept-2-yl]butanol

A solution of ester compound (2) (2.1 g; 6.72 mmole) in dry tetrahydrofuran (10 ml) was added dropwise to a suspension of lithium aluminum hydride (420 mg; 11.1 mmole) in dry tetrahydrofuran (50 ml) at 0° under argon. The mixture was stirred at 0° for 30 minutes at room temperature for 3 hours, and quenched by the successive addition of water (0.42 ml), 10% NaOH (0.7 ml) and water (1.26 ml). The granular precipitates were filtered off and washed with small amounts of ether. The filtrate was diluted with ether (300 ml), dried (anhydrous $MgSO_4$), filtered and was concentrated to give a homogeneous (TLC) oil (1.9; 100%). This (1.4 g) was chromatographed (flash) on a silica gel column (LPS-1), eluting with $Et_2O$:hexane (1:1; 1.5 liters) and $Et_2O$:hexane (3:1; 2.0 liters) to give, after drying in vacuo, the analytical specimen of title alcohol as a clear oil (1.25 g) with consistent MS, $H^1$-NMR, $C^{13}$-NMR and IR spectral data.

[1] An additional 141.1 mg of crude product was recovered from the filtrate giving a total crude yield of 98.9%.

| | | |
|---|---|---|
| Anal Calcd for $C_{17}H_{32}O_3$: | C 71.79; | H, 11.34 |
| Found: | C, 71.56; | H, 11.29 |

$^1$H-NMR (270 MHz, $CDCl_3$):
δ 0.91 (t, 3H, J = ~9; $H_{21}$)
1.2-2.09 (m, 21H, -, -)
3.25-3.42 (m, 4H, -, $H_{14}$ + $H_{16}$)
3.63 (t, 2H, J = ~6, $H_4$)
4.28 (d, 1H, J = ~4, $H_9$)
4.40 (d, 1H, J = ~4, $H_{12}$) ppm

B. [1α,2β(Z),3β,4α]-2-[[4-[3-[(Hexyloxy)methyl]-7-oxabicyclo[2.2.1]hept-2-yl]butyl]oxy]acetic acid, t-butyl ester

A solution of 500 mg (1.76 mmole) of Part A alcohol in 50% NaOH (10.3 ml) and tetrahydrofuran (10.3 ml) was treated with t-butylbromoacetate (2.84 ml, 17.6 mmole) and tetrabutylammonium hydrogen sulfate (935 mg, 2.75 mmole) and stirred under argon at room temperature for 20 hours. The organic solvent was evaporated off and the resulting slurry diluted with water (27.5 ml). The aqueous solution was acidified with 10% HCl (~25 ml) and extracted twice with dichloromethane (125 ml). The organic phase was washed with water (50 ml), dried (anhydrous $MgSO_4$) and evaporated to dryness. The residual syrup was chromatographed (flash) on a silica gel column (LPS-1), eluting the column with $Et_2O$:hexane (1:4, 4.0 liters), $Et_2O$:hexane (1:1, 4.0 liters) and $Et_2O$:hexane (4:1, 3.0 liters). The desired fractions were combined to give the title ester compound as a homogeneous (TLC) oil (381.4 mg, 84.1%) with a consistent $H^1$-NMR-spectrum.

C. [1α,2β(Z),3β,4α]-2-[[4-[3-[(Hexyloxy)methyl]-7-oxabicyclo[2.2.1]hept-2-yl]butyl]oxy]acetic acid

A solution of Part B ester (314.9 mg, 0.79 mmole) in tetrahydrofuran (3.16 ml) and 50% NaOH (3.16 ml) was stirred at room temperature under argon for 19 hours. A solution of the impure product (~39 mg) from a previous run in tetrahydrofuran (0.5 ml) was combined with the reaction mixture and the entire mixture was diluted with water (40 ml) and extracted twice with ether (25 ml). The aqueous phase was acidified with 12N HCl (4 ml) and extracted twice with dichloromethane (200 ml). The organic extract was washed with a saturated sodium chloride solution (25 ml), dried (anhydrous $MgSO_4$) and evaporated to give a thick oil (207.4 mg, 66.2%) which was homogeneous by TLC. This was chromatographed (gravity) on a silica gel column (Baker, 15 g), eluting the column with dichloromethane and increasing amounts of methanol in dichloromethane up to 10%. The desired fractions were combined and evaporated to dryness. In order to remove occluded silica gel, the oil obtained was dissolved in ether (75 ml) and washed with 10% HCl (10 ml), saturated NaCl (10 ml), dried (anhydrous $MgSO_4$) and concentrated to give a thick syrup (124 mg) with consistent MS, IR (the IR spectrum showed a strong C=O absorption at 1650 $cm^{-1}$ and no carboxylic acid absorption at ~3400 $cm^{-1}$ indicating strong H-bonding of the acid function), $H^1$-NMR and $C^{13}$-NMR spectra.

| | | |
|---|---|---|
| Anal Calcd for $C_{19}H_{34}O_5$: | C, 66.63; | H, 10.01 |
| Found: | C, 66.81; | H, 9.90 |

$H^1$-NMR spectrum (FX270; $CDCl_3$):
δ 0.90 (t, 3H, J = ~8, $H_{21}$)
1.21-1.70 (m, 20H, -, -)
2.03 (q, 1H, J = ~7, $H_{13}$)
3.21-3.45 (m, 4H, -, $H_{14}$ + $H_{16}$)
3.55 (broad s, 2H, $H_4$)
3.92 (s, 2H, -, $H_2$)
4.22 (d, 1H, J = ~4, $H_9$)
4.42 (d, 1H, J = ~4, $H_{12}$) ppm

D. (1α,2β,3β,4α)-2-[[4-[3-[(Hexyloxy)methyl]-7-oxabicyclo[2.2.1]hept-2-yl]butyl]oxy]-N-hydroxy-N-methylacetamide

A solution of Part C acid (640 mg, 1.87 mmole) in dry benzene (10 ml) was treated with oxalyl chloride (2 ml, 22.5 mmole) and stirred at room temperature under nitrogen for 2 hours. The excess oxalyl chloride and solvent were blown off by a stream of nitrogen while heating the flask in a warn water bath and the residual oil dried in vacuo (oil pump) for one hour. This acid chloride was dissolved in dry tetrahydrofuran (3.5 ml) and added dropwise into a cold (ice-water) solution of 98% methylhydroxylamine hydrochloride (318.7 mg, 3.74 mmole) and triethylamine (0.92 ml, 7.48 mmole) in tetrahydrofuran (4.6 ml) and water (4.6 ml). The mixture was stirred at 0° under nitrogen for 30 minutes and at room temperature for 7.0 hours, diluted with water (23 ml) and extracted twice with dichloromethane (125 ml). The organic extract was washed with 1N HCl (25 ml), 5% $NaHCO_3$ (12 ml) and brine (20 ml), dried (anhydrous $MgSO_4$), filtered and evaporated to dryness giving an oil (724 mg) containing the desired product and traces of four other components and some Part C acid.

This mixture was chromatographed (gravity) on a silica gel column (Baker, 50 ml), eluting the column with dichloromethane and $CH_2Cl_2$:MeOH (98:2, 95:5, 9:1, 4:1). The pure fractions (TLC) were combined to give an oil (300.8 mg)[1]. In order to remove the entrained silica gel, it was dissolved in ether (50 ml) and washed with 1N HCl (5 ml) and brine (5 ml). The organic phase was dried (anhydrous $MgSO_4$), filtered and evaporated to dryness to give the title compound as a homogeneous oil (which became a waxy solid upon standing) (300 mg) with consistent analytical, IR (1641 $cm^{-1}$ strong, C=O), mass, $H^1$- and $C^{13}$ NMR-spectral data.

| | | | |
|---|---|---|---|
| Anal calcd for $C_{20}H_{37}NO_5$: | C, 64.66; | H, 10.04; | N, 3.77 |
| Found: | C, 64.33; | H, 9.83; | N, 3.43 |

Example 3

(1α,2β,3β,4α)-2-[[4-[3-[(Hexyloxy)methyl]-7-oxabicyclo[2.2.1]hept-2-yl]butyl]oxy]-N-hydroxy-N,2,2-trimethylacetamide

A. (1α,2β,3β,4α)-2-[[4-[3-[(Hexyloxy)methyl]-7-oxabicyclo[2.2.1]hept-2-yl]butyl]oxy]-2-methyl acetic acid, t-butyl ester

A solution of dry diisopropyl amine (0.225 ml, 1.6 mmole) in THF (20 ml)$_x$ was cooled and stirred in a bath at -78° under argon and 1.65 M n-BuLi in hexane (0.73 ml, 1.2 mmole) was added. After 5.0 minutes a solution of Example 2 Part B compound (400 mg, 1.0 mmole) in dry THF (5.0 ml) was added in the course of 2 minutes. After 20 minutes, a solution of dry hexamethylphosphoric triamide (HMPA) (1.0 ml) in dry THF 1.0 ml) and methyl iodide (filtered through basic alumina, 4.0 mmole, 0.25 ml) were added. After 3 hours at -78°, the mixture was poured into 10% hydrochloric acid (10 ml) and concentrated in vacuo at room temperature to remove most of the THF. The concentrate was diluted with brine (25 ml) and extracted with ether (3x30 ml). The extracts were combined, washed with brine (2x10 ml), dried ($MgSO_4$ anhydrous) and evaporated to afford the crude product as an oil. This was chromatographed on a column of silica gel (Baker 60-200 mesh, 30 g) eluting the column with hexane and $Et_2O$-hexane mixtures (1:9, 1:4, 1:1) to isolate the homogeneous (tlc) title compound as an oil (363 mg, 88%) with consistent $H^1$- and $C^{13}$-NMR spectral data.

B. (1α,2β,3β,4α)-2-[[4-[3-[(Hexyloxy)methyl]-7-oxabicyclo[2.2.1]hept-2-yl]butyl]oxy]-2-methylacetic acid, t-butyl ester

A solution of dry diisopropyl amine (0.154 ml, 1.1 mmole) in dry THF (12 ml) was cooled and stirred in a bath at -78° under argon and a 1.65 M solution of n-BuLi in hexane (0.61 ml, 1.0 mmole) was added. After 5 minutes, a solution of Part A compound (360 mg, 0.87 mmole) in dry THF (4.0 ml) was added in the course of 2 minutes. After 20 minutes, a solution of dry HMPA (0.5 ml) in dry THF (0.5 ml) and methyl

[1] More title compound (117.8 mg) containing a trace of an impurity was obtained from the later fractions.

iodide (filtered through basic alumina, 0.22 ml, 3.5 mmole) were added. After 2 hours, the mixture was poured into 10% hydrochloric acid and concentrated in vacuo at room temperature to remove most of the THF. The concentrate was diluted with brine (20 ml) and extracted with ether (3x30 ml). The extracts were combined, washed with brine (2x10 ml), dried ($MgSO_4$ anhydrous) and evaporated to afford the product as an oil. This was chromatographed on a column of silica gel (20 g, Baker 60-200 mesh) eluting the column with hexane and $Et_2O$-hexane mixtures (1:9, 1:4, 1:1) to isolate the homogeneous (tlc) title compound as an oil (330 mg, 89%) with consistent $H^1$- and $C^{13}$-NMR spectral data.

C. (1α,2β,3β,4α)-2-[4-[[3-[(Hexyloxy)methyl]-7-oxabicyclo[2.2.1]hept-2-yl]butyl]oxy]-2-methylacetic acid

A solution of Part B compound (315 mg, 0.74 mmole) in distilled dioxane (5.0 ml) was refluxed under stirring with 3N LiOH (3.0 ml) in an atmosphere of argon for 36 hours. The mixture was then cooled to room temperature, acidified with concentrated HCl (0.9 ml) and concentrated in vacuo to remove most of the dioxane. The concentrate was diluted with brine (20 ml) and extracted with ether (3x20 ml). The extracts were combined, washed with brine (2x20 ml), dried ($MgSO_4$ anhydrous) and evaporated to afford crude Part C acid as an oil. A tlc examination of this (silica gel, EtOAc) revealed the presence of Part C acid and a minor more polar impurity. This was chromatographed on a column of silica gel (15 g, Baker 60-200 mesh) eluting the column with hexane, $Et_2O$-hexane (3:7, 1:1, 7:3), $Et_2O$, $Et_2O$-$CH_3OH$ (95:5, 1:4, 1:1) and $CH_3OH$. The relevant fractions were combined to isolate Part C acid and a mixture of Part C acid and the unidentified more polar component as oils.

The Part C acid thus isolated contained some silica gel. It was, therefore, dissolved in $Et_2O$ (50 ml), washed with 1N HCl (2x10 ml) and brine (2 x 10 ml), dried ($MgSO_4$), evaporated and re-dried in vacuo to afford the analytical specimen as an oil (212 mg, 74%), with consistent MS, IR (1736 $cm^{-1}$, strong, C=O), $H^1$-NMR and $C^{13}$-NMR data.

| | | |
|---|---|---|
| Anal calcd for $C_{21}H_{38}O_5$: | C, 68.07; | H, 10.34 |
| Found: | C, 68.07; | H, 10.25 |

$H^1$-NMR spectrum (FX-270, $CDCl_3$):
δ 0.90 (t, 3H, J=~7.0, $H_{21}$)
1.46 (S, 6H, -, $H_{22}$ + $H_{23}$)
2.05 (m, 1H, -, $H_{13}$)
3.35 (m, 6H, -, $H_4$ + $H_{14}$ + $H_{16}$)
4.28 (d, 1H, J=~4.0, $H_9$)
4,42 (d, 1H, J=~4.0, $H_{12}$) ppm

D. (1α,2β,3β,4α)-2-[[4-[3-[(Hexyloxy)methyl]-7-oxabicyclo[2.2.1]hept-2-yl]butyl]oxy]-N-hydroxy-N,2,2-trimethylpropanamide

A solution of Part C acid (411 mg, 1.11 mmole) in dry benzene (10 ml) was cooled and stirred in a bath at 5° and oxalyl chloride (0.5 ml) was added, followed dropwise by dry DMF (0.15 ml). Vigorous gas evolution was noted. After 30 minutes, the solvents were removed under a jet of nitrogen at ambient temperature and the residue was dried in vacuo to afford the acid chloride as an oil containing a small amount of solid (dimethyliminium chloride). This oil was dissolved in THF (2.0 ml) and added into a stirred ice-cold solution of methylhydroxylamine hydrochloride (200 mg, 2.4 mmole) and triethylamine (0.5 ml, 3.6 mmole) in 50% aqueous THF (8.0 ml). After 30 minutes, the mixture was warmed to room temperature and stirred for 4.0 hours. It was then concentrated in vacuo, the residue diluted with brine (15 ml) and 10% HCl (15 ml) and extracted with ether (3x20 ml). The extracts were combined, washed with brine, a dilute $NaHCO_3$ solution and brine, dried ($MgSO_4$•anhydrous) and evaporated to afford the crude product as an oil. A tlc (silica gel; 5:95 $CH_3OH$-$CH_2Cl_2$) examination of this showed the presence of one major spot. The starting acid was absent and traces of less polar products were present. This was chromatographed on a column of silica gel (30 g, Baker 60-200 mesh) eluting the column with hexane, $Et_2O$-hexane (1:4, 1:1) and $Et_2O$-$CH_3OH$ (98:2) to isolate, after drying in vacuo, the analytical specimen of title compound as an oil (385 mg, 88%) with consistent IR (1631 $cm^{-1}$, strong, C=O; 3242 $cm^{-1}$, weak, OH, 1778, 1738 $cm^{-1}$, weak,

C=O)[1], mass, $H^1$- and $C^{13}$-NMR spectral data.

| | | | |
|---|---|---|---|
| Anal Calcd for $C_{22}H_{41}NO_5$: | C, 66.13; | H, 10.34; | N, 3.50 |
| Found: | C, 66.26; | H, 10.23; | N, 3.36 |

$H^1$-NMR Spectrum (FX-270, $CDCl_3$)[2]:
δ 0.90 (t, 3H, J=~7.0, $H_{21}$)
1.48 (s, 6H, -, $H_{22}$ + $H_{23}$)
2.02 (q, 1H, J =~4.0, $H_{13}$)
3.31, 3,50 (m and broad hump respectively, 9H, $H_{14}+H_{16}+H_6+H_{24}$)
4.24 (d, 1H, J =~4.0, $H_9$)
4.40 (d, 1H, J = ~4.0, $H_{12}$)
8.62 (broad s, -, -, OH) ppm

Example 4

[1α,2β(Z),3β,4α]-2-[[4-[3-[(Hexyloxy)methyl]-7-oxabicyclo[2.2.1]hept-2-yl]2-butenyl]oxy]-N-hydroxy-N,2,2-trimethylpropanamide

A. [1α,2β(Z),3β,4α]-2-[[4-[3-[(Hexyloxy)methyl]-7-oxabicyclo[2.2.1]hept-2-yl]-2-butenyl]oxy]-2-methylacetic acid, t-butyl ester

A solution of dry diisopropylamine (0.640 ml; 4.57 mmole) in dry THF (30 ml) was cooled down to -78° under argon and a 1.65M solution of n-BuLi in hexane (2.0 ml; 1.5 eq.) was added. After 5 minutes, a solution of Example 1 Part D compound (906 mg; 2.28 mmole) in dry THF (10 ml) was added over a period of 2 minutes. After 20 minutes, a solution of dry hexamethyl phosphoric triamide (1.3 ml) in dry THF (1.3 ml) was added, followed by $CH_3I$ (0.6 ml). The mixture was stirred at -78° for 3 hours, poured into 10% hydrochloric acid (25 m) and concentrated in vacuo to remove most of the THF. The concentrate was diluted with brine (50 ml) and extracted twice with ether (200 ml) and once with dichloromethane (100 ml). The extracts were washed with a 2.5% sodium thiosulfate solution (40 ml), brine (40 ml), dried (anhydrous $MgSO_4$), filtered and evaporated to afford the crude product as an oil. This was chromatographed on a column of silica gel (Baker, 60-200 mesh, 100 ml), eluting the column with $Et_2O$:hexane (1:9, 1:4) to isolate the homogeneous (TLC) title compound as an oil (840 mg, 89.7%) with a consistent $H^1$-spectrum.

B. [1α,2β(Z),3β,4α]-2-[[4-[3-[(Hexyloxy)methyl]-7-oxabicyclo[2.2.1]hept-2-yl]-2-butenyl]oxy]-2,2-dimethylacetic acid, t-butyl ester

A solution of dry diisopropylamine (0.640 ml; 4.57 mmole) in dry THF (30 ml) was cooled down to -78° under argon and a 1.65M solution of n-BuLi in hexane (2.0 ml; 3.42 mmole) was added. After 5 minutes, a solution of Part A compound (840 mg, 2.05 mmole) in dry THF (10 ml) was added over a period of 2 minutes. After 20 minutes, a solution of dry HMPA (1.3 ml) in dry THF (1.3 ml) was added, followed by $CH_3I$ (0.6 ml, 3.5 eq.). The mixture was stirred at -78° for 3.5 hours, poured into 10% hydrochloric acid (25 ml) and concentrated in vacuo to remove most of the THF. The concentrate was diluted with brine (50 ml) and extracted twice with ether (200 ml) and once with dichloromethane (100 ml). The extracts were washed with a 2.5% sodium thiosulfate solution (40 ml) and brine (40 ml), dried (anhydrous $MgSO_4$), filtered and evaporated to afford the crude product as an oil. This was chromatographed on a column of silica gel (Baker, 60-200 mesh, 100 ml), eluting the column with $Et_2O$:hexane (1:9) to isolate the homogeneous (TLC) title compound as an oil (713.6 mg, 82%) with consistent $H^1$ and $C^{13}$-spectral data.

[1] The weak C=O peaks could not be eliminated by rechromatography. The specimen was homogeneous by tlc.

[2] Weak singlets due to unidentified impurities were present at 2.82δ and 3.70δ.

C. [1a,2β(Z),3β,4α]-2-[[4-[3-[(Hexyloxy)methyl]-7-oxabicyclo[2.2.1]hept-2-yl]-2-butenyl]oxy]-2,2-dimethylpropanoic acid

A solution of Part B ester (368 mg, 0.87 mmole) and 480 µl (5.17 mmole) of trifluoroacetic acid in dry dichloromethane (25 ml) was refluxed with stirring under nitrogen for 28 hours. The reaction mixture was then cooled to room temperature, diluted with dichloromethane (100 ml) and washed with brine (25 ml). The organic phase was dried (anhydrous $MgSO_4$), filtered and evaporated to afford the crude product as an oil. This was chromatographed on a silica gel column (Baker, 60-200 mesh, 50 ml) eluting the column with $Et_2O$:hexane mixtures (1:9; 1:4; 1:1), $Et_2O$ and $Et_2O$:MeOH (9:1) to isolate the title acid as an oil (268 mg, 83.6%) with consistent analytical, MS, IR, (1740 $cm^{-1}$, strong, C=O), $H^1$-NMR and $C^{13}$-NMR data.

| | | |
|---|---|---|
| Anal calcd for $C_{21}H_{36}O_5$: | C, 68.44; | H, 9.85 |
| Found: | C, 68.34; | H, 9.73 |

$H^1$-NMR spectrum (FX-270, $CDCl_3$):
δ 0.88 (t, 3H, J =~7, $H_{21}$)
1.18-2.17 (m, 23H, -, -)
3.30-3.47 (m, 4H, -, $H_{14}$ + $H_{16}$)
3.98 (d, 2H, J =~6, $H_4$)
4.38 (d, 1H, J =~4, $H_9$)
4.41 (d, 1H, J =~4, $H_{12}$)
5.54-5.69 (m, 2H, -, $H_5$ + $H_6$) ppm

D. [1α,2β(Z),3β,4α]-2-[[4-[3-[(Hexyloxy)methyl]-7-oxabicyclo[2.2.1]hept-2-yl]-2-butenyl]oxy]-N-hydroxy-N,2,2-trimethylpropanamide

A solution of Part C acid (622 mg, 1.68 mmole) and oxalyl chloride (0.4 ml, 4.5 mmole) in dry benzene (10 ml) was cooled down to 0° (ice-water bath), treated with dry dimethylformamide (2 drops) and stirred at 0° for 30 minutes and at room temperature for one hour. The excess oxalyl chloride and solvent were blown off under a stream of nitrogen while heating the flask in a warm water bath and the residual oil was dried in vacuo for one hour. This acid chloride was dissolved in dry tetrahydrofuran (3.3 ml) and added dropwise with stirring into a cold solution (~0°, ice-water) of 98% methylhydroxylamine hydrochloride (294.2 mg, 3.44 mmole) and triethylamine (0.85 ml, 6.91 mmole) in tetrahydrofuran (4.3 ml) and water (4.8 ml). The mixture was stirred at 0° for 30 minutes and at room temperature for 6.5 hours, diluted with water (23 ml) and extracted twice with dichloromethane (110 ml). The combined organic extracts were washed with 1N HCl (23 ml), 5% $NaHCO_3$ (11 ml) and brine (20 ml), dried (anhydrous $MgSO_4$), filtered and evaporated to dryness giving an oil (717 mg) containing the desired product and traces of 2 other components.

The mixture was chromatographed on a silica gel column (Baker, 60-200 mesh, 50 ml), eluting the column with mixtures of $Et_2O$:hexane (1:4, 1:1), $Et_2O$ and $Et_2O$:MeOH (9:1) to isolate after drying in vacuo the homogeneous (TLC) title compound as an oil (443.4 mg) with consistent elemental analysis, MS, IR (1628 $cm^{-1}$, strong, C=O), $H^1$- and $C^{13}$-NMR data. An additional 78.4 mg of impure product was obtained from the column giving a total crude yield of 77.49%. The complexity of both NMR spectra is believed to be due to the presence of several $C_1$-N rotamers.
$H^1$-NMR spectrum (FX-270, $CDCl_3$):
δ0.89 (t, 3HH, J =~7, $H_{21}$)
1.20-2.16 (m, 22N, -, -)
3.20-3.80 (m, 7H, -, $H_{14}$ + $H_{16}$ + $H_{24}$)
3.95 (S, broad, 2H, -, $H_4$)
4.19 (d, 1H, J =~4, $H_9$)
4.89 (d, 1H, J =~4, $H_{12}$)
5.55 (m, 2H, -, $H_5$ + $H_6$)
8.57 (s, broad, 1H, -, N-OH) ppm

Example 5

(1α,2β,3β,4α)-2-[[4-[3-[(Hexyloxy)methyl]-7-oxabicyclo[2.2.1]hept-2-yl]butyl]thio]-N-hydroxy-N-methylacetamide

A. (1α,2β,3β,4α)-4-[[3-[(Hexyloxy)methyl]-7-oxabicyclo[2.2.1]hept-2-yl]butyl]thioacetate

A suspension of 217.3 mg (0.82 mmole) of triphenylphosphine in dry distilled tetrahydrofuran (2.52 ml) was cooled down to 0° under $N_2$ and treated dropwise with diisopropylazadicarboxylate (0.17 ml; 0.84 mmole). The mixture was then stirred for 30 minutes and treated, dropwise, with a solution of [1α,2β,3β,4α]-4-[3-[(hexyloxy)methyl]-7-oxabicyclo[2.2.1]hept-2-yl]butanol (prepared as described in Example 2 Part A) (117 mg, 0.41 mmole) and thiolacetic acid (0.06 ml) in dry tetrahydrofuran (0.6 ml). The stirring was then continued at 0° for 1 hour and at room temperature for 3 hours. The mixture was then evaporated to dryness on a rotary evaporator and the residue was triturated twice with $Et_2O$:hexane (1:4, 15 ml), filtering off the white precipitates that formed. The clear filtrate was concentrated to dryness and the resulting syrup was flash chromatographed on a silica gel column (LPS-1) eluting the column with $Et_2O$:hexane (1:9, 1.0 liter). The desired fractions were combined to give 128.8 mg (91.7%) of the title thioacetate as a homogeneous (TLC) oil with consistent $H^1$ and $C^{13}$-NMR spectral data.

B. (1α,2β,3β,4α)-4-[3-[(Hexyloxy)methyl]-7-oxabicyclo[2.2.1]hept-2-yl]butanethiol

A suspension of lithium aluminum hydride (17.1 mg, 0.45 mmole) in dry distilled tetrahydrofuran (1 ml) was cooled down to 0° under $N_2$ and treated with a solution of 128.8 mg (0.367 mmole) of the Part A thioacetate in dry tetrahydrofuran. The mixture was stirred at 0° for 1 hour then quenched by the successive addition of water (0.02 ml), 10% $Na_2SO_4$ (0.05 ml) and water (0.06 ml). The mixture was stirred for 30 minutes, diluted with dichloromethane (10 ml) and filtered, washing the salts well with dichloromethane (20 ml). The filtrate was dried (anhydrous $MgSO_4$), filtered and concentrated to give 113 mg (100%) of title thiol compound as an oil (homogeneous by TLC) with consistent $H^1$ and $C^{13}$-NMR spectra.

C. (1α,2β,3β,4α)-2-[[4-[3-[(Hexyloxy)methyl]-7-oxabicyclo[2.2.1]hept-2-yl]butyl]thio]acetic acid, methyl ester

A solution of 340 mg (1.13 mmole) of the Part B thiol in dry acetone (12 ml) was treated with 609 mg (4.41 mmole) of potassium carbonate (anhydrous) and methyl bromoacetate (0.21 ml, 2.20 mmole) and stirred at room temperature for 3 hours. The mixture was then diluted with ether (30 ml), dried (anhydrous $MgSO_4$) and filtered, washing the precipitates well with ether (120 ml). The filtrate and washings were combined and evaporated to give 449 mg of title ester compound as an oil which had trace amounts of two other by-products (TLC). This crude mixture was flash-chromatographed on a silica gel column (LPS-1) using $Et_2O$:hexane (1:9; 6.0 liters). The fractions containing the desired product were combined and concentrated to give 367 mg (87.2%) of title ester compound as a homogeneous oil (TLC) with consistent $H^1$ and $C^{13}$-NMR data.

D. (1α,2β,3β,4α)-2-[[4-[3-[(Hexyloxy)methyl]-7-oxabicyclo[2.2.1]hept-2-yl]butyl]thio]acetic acid

A stirred solution of 367.3 mg (0.99 mmole) of Part C ester in tetrahydrofuran (70.7 ml) and water (17.3 ml) was cooled down to 0°C and argon was bubbled in for 30 minutes. Hydroquinone (10 mg) was then added to the solution followed by 1N LiOH (10 ml) and the bubbling of argon was continued. After 30 minutes at 0° and 4 hours at room temperature the mixture was acidified with 1N HCl (10 ml). After 30 minutes the organic solvent was evaporated in vacuo and the aqueous suspension was diluted with water (25 ml) and extracted twice with dichloromethane (130 ml). The organic phase was dried (anhydrous $MgSO_4$), filtered and concentrated to give 311 mg of a yellow oil containing the desired product and a small amount of another component at the solvent front. This oil was chromatographed 3 times on a silica gel column (Baker) in order to get rid of the contaminant and hydroquinone, eluting the columns with decreasing concentrations of hexane in ethyl acetate:hexane solutions (50% to 10%). The desired fractions were combined, evaporated and dried in vacuo to give 144 mg (40.6%) of the title acid as a homogeneous (tlc) light yellow oil with consistent IR, MS, $H^1$-NMR and $C^{13}$-NMR spectral data.

| | | | |
|---|---|---|---|
| Anal Calcd for $C_{19}H_{34}O_4S$: | C, 63.65; | H, 9.56; | S, 8.94 |
| Found: | C, 63.58; | H, 9.54; | S, 8.78 |

$^1$H-NMr (270 MHz, $CDCl_3$):
δ0.89 (t, 3H, J = ~7.0, $H_{21}$)
1.2-1.8 (m, 20H, -, -)
2.04 (q, 1H, J = ~4.0, $H_{13}$)
2.65 (t, 2H, J = ~7, $H_4$)
3.23 (s, 2H, -, $H_2$)
3.2-3.5 (m, 4H, -, $H_{14}$ & $H_{16}$)
4.26 (d, 1H, J = ~4, $H_9$)
4.43 (d, 1H, J = ~4, $H_{10}$) ppm

E. (1α,2β,3β,4α)-2-[[4-[3-[(Hexyloxy)methyl]-7-oxabicyclo[2.2.1]hept-2-yl]butyl]thio]-N-hydroxy-N-methylacetamide

Following the procedure of Example 1 Part F except substituting the above Part D acid for the Example 1 Part E acid, the title compound is obtained.

Example 6

(1α,2β,3β,4α)-2-[[4-[3-[(Hexylthio)methyl]-7-oxabicyclo[2.2.1]hept-2-yl]butyl]oxy]-N-hydroxy-N-methylacetamide

A. (1α,2β,3β,4α)-Cis-exo-7-oxabicyclo[2.2.1]heptane-2,3-dimethanol

To a suspension of 11.4 g lithium aluminum hydride (300 mmole, 1.6 eq.) in 400 ml of dry THF at 0°C was added dropwise a solution of 32 g cis-exo-7-oxabicyclo[2.2.1]heptane-2,3-dicarboxylic anhydride (190 mmole) in 400 ml of dry THF over a period of 1 hour. The reaction mixture was stirred at 25°C for 18 hours, cooled to 0°C and quenched by slow addition of a saturated $Na_2SO_4$ solution, and filtered. The solid was washed with three 100 ml portions of $CH_2Cl_2$. The combined organic layer was dried over $MgSO_4$ and concentrated to give 32 g of title diol as a colorless acid.

A'. (1α,2β,3β,4α)-Cis-exo-2-hydroxymethyl-3-[(phenylmethoxy)methyl]-7-oxabicyclo[2.2.1]heptane

To a suspension of 3.08 g of sodium hydride (70 mmole, 50% oil dispersion), washed with ether, in 100 ml of dry DMF was added with stirring at 0°C a solution of 10.0 g title A diol (64 mmole) in 30 ml of DMF over a period of 15 minutes. The mixture was stirred for 30 minutes at 0°C, 20 minutes at 25°C, recooled to 0°C and 12.0 g of benzyl bromide (70 mmole) was added dropwise. After stirring at 25°C for 2 hours, the reaction was quenched with an aqueous ammonium chloride solution, extracted with ether, dried over anhydrous $MgSO_4$ and concentrated.

Purification was done on a silica gel column, eluting with 10-20% ethyl acetate in hexane to give 11.8 g of the title monobenzylether.

B. (1α,2β,3β,4α)-2-Chloromethyl-3-[(Phenylmethoxy)methyl]-7-oxabicyclo[2.2.1]heptane

A solution of the Part A' compound (20 mmole), p-toluene sulfonyl chloride (21 mmole) and pyridine (4.0 ml) is stirred in dichloromethane (20 ml) for 20 hours at room temperature. The mixture is then diluted with ether (100 ml), washed with cold 10% hydrochloric acid (2x10 ml), a 10% $Na_2CO_3$ solution and water, dried ($MgSO_4$ anhydrous), evaporated and the residual oil is subjected to flash chromatography on LPS-1 silica gel to afford the title compound.

C. (1α,2β,3β,4α)-2-(Cyanomethyl)-3-[(Phenylmethoxy)methyl]-7-oxabicyclo[2.2.1]heptane

A solution of Part B compound (13 mmole) and sodium cyanide (1.5 g) in dry dimethylsulfoxide (15 ml) is heated in a bath at 90-95° for 18 hours. The mixture is then cooled to room temperature, diluted with water (75 ml) and is extracted with ether (3 x 40 ml). The ether extracts are combined, washed with water (2x10 ml), dried ($MgSO_4$ anhydrous) and the residual oil is flash chromatographed on a silica gel (LPS-1) column to isolate the title compound.

D. (1α,2β,3β,4α)-2-(Formylmethyl)-3-[(Phenylmethoxy)methyl]-7-oxabicyclo[2.2.1]heptane

A solution of Part C compound (10 mmole) in dry toluene (20 ml) is stirred under argon in a bath at -78° and a 1.5 molar solution of diisobutyl aluminum hydride (10 ml) is added. After 4 hours, the mixture is quenched by the addition of 10% hydrochloric acid (30 ml), warmed to room temperature and is extracted with ether (3x30 ml). The extracts are combined, washed with 10%-hydrochloric acid and dilute brine, dried ($MgSO_4$ anhydrous) and evaporated to afford the title compound as an oil.

E. (1α,2β,3β,4α)-4-[[3-(Phenylmethoxy)methyl]-7-oxabicyclo[2.2.1]hept-2-yl]-2-butenoic acid, methyl ester [Wadsworth-Emmons reaction - W. C. Still, C. Gennari,, Tetrahedron Letters, 26, No. 41, 4405 (1983)]

A suspension of 50% sodium hydride in paraffin (8.75 mmole, 420 mg) in dry THF (60 ml) is cooled and stirred in an ice-bath under an atmosphere of argon, and trimethylphosphonoacetate (12.4 mmole, 2.4 ml) is added. The resulting slurry is stirred for 30 minutes at room temperature for 1 hour. It is then recooled in the ice-bath and a solution of the Part D aldehyde (7.9 mmole) in dry THF (20 ml) is added. After stirring for 30 minutes in the ice-bath and at ambient temperature for 2 hours, glacial acetic acid (2.0 ml) is added and the mixture is evaporated to dryness in vacuo. After dilution of the residue with water (100 ml), the product is extracted into ether (3x50 ml). The extracts were combined, washed with water, dried ($MgSO_4$ anhydrous), filtered, evaporated and the residue is chromatographed on a column of silica gel to isolate the title compound.

F. (1α,2β,3β,4α)-4-[[3-(Phenylmethoxy)methyl]-7-oxabicyclo[2.2.1]hept-2-yl]-2-butenol

A solution of Part E compound (10 mmole) in dry toluene is reduced using 1.5 molar diisobutyl aluminum hydride in toluene (20 ml) as in Example 1 Part C to afford the title compound as an oil.

G. (1α,2β,3β,4α)-2-[[[4-[3-(Phenylmethoxy)methyl]-7-oxabicyclo[2.2.1]hept-2-yl]-2-butenyl]oxy]acetic acid, 1,1-(dimethyl)ethyl ester

A mixture of Part F compound (3.0 mmole), t-butylbromoacetate (5.0 ml), tetrabutyl ammonium sulfate (1.7 g), tetrahydrofuran (20 ml) and 50% sodium hydroxide (20 ml) is vigorously stirred under argon for 2½ hours. Most of the tetrahydrofuran is removed by concentration in vacuo and the residue is extracted with dichloromethane (3x30 ml). The extracts are combined, washed with water, dried ($MgSO_4$ anhydrous), evaporated and the residue is flash chromatographed on LPS-1 silica gel to afford the title compound.

H. (1α,2β,3β,4α)-2-[[4-[3-(Hydroxymethyl)-7-oxabicyclo[2.2.1]hept-2-yl]-2-butyl]oxy]acetic acid, 1,1-(dimethyl)ethyl ester

A solution of Part G compound (5.0 mmole) in methanol (25 ml) containing 5% palladium on carbon (50 mg) is stirred under an atmosphere of hydrogen for 2 hours. It is then filtered through a bed of Celite and is evaporated to afford the title compound.

I. (1α,2β,3β,4α)-2-[[4-[3-[(Acetylthio)methyl]-7-oxabicyclo[2.2.1]hept-2-yl]-2-butyl]oxy]acetic acid, 1,1-dimethyl (ethyl) ester

Triphenyl phosphine (8 mmole) is stirred in dry THF (30 ml) in an ice-bath under an atmosphere of argon and diisopropylazadicarboxylate (8.0 mmole) is added. After 15 minutes, a solution of Part H compound (8.0 mmole) in dry THF (10 ml) is added followed by thiolacetic acid (8.1 mmole) in dry THF (5.0 ml). After stirring for ½ hour in the ice-bath and 4 hours at room temperature, the mixture is concentrated in vacuo and the residual syrup is flash chromtographed to isolate the title compound.

J. (1α,2β,3β,4α)-2-[[4-[3-[(Hexylthio)methyl]-7-oxabicyclo[2.2.1]hept-2-yl]-2-butyl]oxy]acetic acid, 1,1-(dimethyl) ethyl ester

A solution of Part I compound (5.0 mmole) in dry methanol (20 ml) containing anhydrous $K_2CO_3$ (10 mmole) and n-hexyl bromide (12 mmole) is stirred in an ice-bath under an atmosphere of argon for 4 hours. The mixture is then concentrated in vacuo, diluted with water and is extracted with ether. The ether extract is dried ($MgSO_4$ anhydrous), evaporated and the residue is flash chromatographed on silica gel (LPS-1) to isolate the title compound.

K. (1α,2β,3β,4α)-2-[[4-[3-[(Hexylthio)methyl]-7-oxabicyclo[2.2.1]hept-2-yl]-2-butyl]oxy], acetic acid

A solution of Part J compound (5.0 mmole) in dry dichloromethane (20 ml) is stirred at room temperature with a solution of trifluoroacetic acid (10 mmole) in dichloromethane (5.0 ml) for 4 hours. The solution is then washed with dilute brine (3x10 ml), dried ($MgSO_4$ anhydrous) and is evaporated to afford the title compound.

L. (1α,2β,3β,4α)-2-[[4-(3-[(Hexylthio)methyl]-7-oxabicyclo[2.2.1]hept-2-yl]butyl]oxy]-N-hydroxy-N-methylacetamide

Following the procedure of Example 1 Part F except substituting the Example 8 Part K acid for the Example 1 Part E acid, the title compound is obtained.

Example 7

(1α,2β,3β,4α)-2-[[4-[3-[(Hexylthio)methyl]-7-oxabicyclo[2.2.1]hept-2-yl]butyl]thio]-N-hydroxy-N-methylacetamide

A. (1α,2β,3β,4α)-4-[3-(Hydroxymethyl)-7-oxabicyclo[2.2.1]hept-2-yl]butanol

A solution of Example 6 Part F compound (5.0 mmole) in methanol (25 ml) containing 5% Pd/c (50 mg) is stirred under an atmosphere of hydrogen for 4 hours. Filtration of the mixture through a bed of Celite followed by evaporation gives the title compound.

B. (1α,2β,3β,4α)-2-[4-(Acetylthio)butyl]-7-oxabicyclo[2.2.1]heptane-3-methanol

A solution of Part A compound in dry THF is reacted as described in Example 6 Part I and the product is isolated to afford the title compound.

C. (1α,2β,3β,4α)-4-[3-(Hydroxymethyl)-7-oxabicyclo[2.2.1]hept-2-yl]butanethiol

To a cooled (ice-bath) and stirred suspension of lithium aluminum hydride (10 mmole) in dry tetrahydrofuran (20 ml) is added a solution of the Part B compound (3.0 mmole) in dry THF (50 ml) in the course of 3 minutes. The mixture is stirred for 1 hour, and is carefully decomposed by the addition of a 20% sodium sulfate solution. The mixture is then filtered through a bed of Celite and the Celite is washed with small amounts of dry tetrahydrofuran. The filtrate and washings are combined, dried ($MgSO_4$ anhydrous) and the resulting mixture is evaporated to afford the title compound as an oil.

D. (1α,2β,3β,4α)-2-[[4-[3-(Hydroxymethyl)-7-oxabicyclo[2.2.1]hept-2-yl]butyl]thio]acetic acid, methyl ester

A solution of Part C compound (5 mmole), powdered $K_2CO_3$ (20 mmole) and methylbromoacetate (10 mmole) is stirred in dry methanol (30 ml) for 3 hours under an atmosphere of nitrogen. The mixture is then concentrated in vacuo, diluted with ether (50 ml), washed with water, dried ($MgSO_4$ anhydrous) and is evaporated to afford an oil. This is flash chromatographed on a column of silica gel (LPS-1) to isolate the title compound.

E. (1α,2β,3β,4α)-2-[[[4-[3-(Acetylthio)methyl]-7-oxabicyclo[2.2.1]hept-2-yl]butyl]thio]acetic acid, methyl ester

Part D compound is reacted with thioacetic acid by the procedure described in Example 6 Part I and is processed to afford the title compound.

F. (1α,2β,3β,4α)-2-[[4-[3-[(Hexylthio)methyl]-7-oxabicyclo[2.2.1]hept-2-yl]butyl]thio]acetic acid, methyl ester

A solution of Part E compound (5.0 mmole) in dry methanol (25 ml) containing anhydrous $K_2CO_3$ is stirred at room temperature in an ice bath under an atmosphere of argon for 2 hours. Then, n-hexyl bromide (7 mmole) is added and the stirring is continued for 5 hours. The mixture is then concentrated in vacuo, diluted with water and is extracted with ether. The ether extracts are combined, dried ($MgSO_4$ anhydrous), evaporated and the residue is chromatographed on a column of silica gel to isolate the title compound.

G. (1α,2β,3β,4α)-2-[[4-[3-[(Hexylthio)methyl]7-oxabicyclo[2.2.1]hept-2-yl]butyl]thio]acetic acid

A carefully degassed solution of Part F compound (3.0 mmole) in tetrahydrofuran (15 ml) containing 2N lithium hydroxide (10 ml) is refluxed under an atmosphere of argon for 5 hours. The mixture is then cooled to room temperature and is acidified with 10% hydrochloric acid. It is then concentrated in vacuo, diluted with water and is extracted with ether. The ether extracts are combined, washed with water, dried ($MgSO_4$ anhydrous) and the mixture is evaporated to afford the title compound.

H. (1α,2β,3β,4α)-2-[[4-[3-[(Hexylthio)methyl]-7-oxabicyclo[2.2.1]hept-2-yl]butyl]thio]-N-hydroxy-N-methylacetamide

Following the procedure of Example 1 Part F except substituting the Part G acid for the Example 1 Part C acid, the title compound is obtained.

Example 8

[1α,2β(Z),3β,4α]-2-[[4-[3-[(Hexyloxy)methyl]-7-oxabicyclo[2.2.1]hept-2-yl]-2-butenyl]thio]acetic acid

A. [1α,2β(Z),3β,4α]-4-[3-[(Hexyloxy)methyl]-7-oxabicyclo[2.2.1]hept-2-yl]-2-butene thiol, acetic acid ester

To a cooled (ice bath) and stirred suspension of triphenylphosphine (6.0 mmole) in dry THF (20 ml) is added diisopropylazadicarboxylate (6.0 mmole). After 30 minutes a solution of [1α,2β(Z),3β,4α]-4-[3-[-(hexyloxy)methyl]-7-oxabicyclo[2.2.1]hept-2-yl]-2-butenol (3.0 mmole) and thiol acetic acid (6.0 mmole) in dry THF (5.0 ml) is added. The mixture is then stirred in the ice-bath for 1 hour and at room temperature for 3 hours. It is then concentrated in vacuo and the residue is triturated twice with ether-hexane (1:3, 30 ml each), removing the solids by filtration. The filtrate is evaporated and the residue is flash chromatographed on a silica gel (LPS-1) column eluting with ether-hexane (1:9) to isolate the title compound.

B. [1α,2β(Z),3β,4α]-4-[3-[(Hexyloxy)methyl]-7-oxabicyclo[2.2.1]hept-2-yl]-2-butene thiol

To a cooled (ice bath) and stirred suspension of lithium aluminum hydride (2.0 mmole) in dry THF (10 ml) is added a solution of Part A ester compound (2.0 mmole) in dry THF. After 1 hour, a mixture of water (0.5 ml) and THF (2.0 ml) is added dropwise. After stirring for 30 minutes, the mixture is filtered through a bed of Celite, washing the Celite with small amounts of THF. The filtrate and washings are combined, dried ($MgSO_4$ anhydrous), filtered and is evaporated to afford the title compund as an oil.

C. [1α,2β(Z),3β,4α]-2-[[4-[3-[(Hexyloxy)methyl]-7-oxabicyclo[2.2.1]hept-2-yl]-2-butenyl]thio]acetic acid, methyl ester

A solution of Part B thiol compound (1.5 mmole) in dry acetone (15 ml) is stirred under an atmosphere of nitrogen with anhydrous $K_2CO_3$ (4.0 mmole) and methyl bromo acetate (3.0 mmole) at ambient temperature for 3 hours. The mixture is then diluted with ether (60 ml) and is filtered. The filtrate is evaporated in vacuo and is flash chromatographed on a column of silica gel (LPS-1) using $Et_2O$-hexane (1:9) for elution to isolate the title compound.

D. [1α,2β(Z),3β,4α]-2-[[4-[3-[(Hexyloxy)methyl]-7-oxabicyclo[2.2.1]hept-2-yl]-2-butenyl]thio]acetic acid

A mixture of THF (30 ml), water (8.0 ml) and 2N LiOH (5.0 ml) is stirred in an ice bath and argon gas is bubbled in for 30 minutes. A solution of Part C ester compound (1.0 mmole) and hydroquinone (15 mg) in THF (2.0 ml) is then added and the bubbling of argon is continued. After stirring in the ice bath for 30 minutes and at ambient temperature for 4 hours, the mixture is acidified with 1N HCl (11 ml) and is concentrated in vacuo. The concentrate is diluted with water (30 ml) and is extracted with $CH_2Cl_2$ (2x30 ml). The extracts are combined, washed with water, dried ($MgSO_4$ anhydrous), filtered and the filtrate is evaporated to afford the crude product. This is purified by chromatography on silica gel (Baker 60-200 mesh) eluting the column with ether hexane mixtures to isolate the title compound.

Example 9

[1α,2β(Z),3β,4α]-2-[[4-[3-[(Hexylthio)methyl]-7-oxabicyclo[2.2.1]hept-2-yl]-2-butenyl]thio]acetic acid

A. [1α,2β(Z),3β,4α]-4-[3-[(Hydroxy)methyl]-7-oxabicyclo[2.2.1]hept-2-yl]-2-butenoic acid, methyl ester

A solution of (exo)-octahydro 5,8-epoxy-1H-benzopyran-3-ol (3.9) mmole and carboxymethyl triphenyl phosphorane (3.9 mmole) in dry THF (20 ml) is stirred at room temperature. After 20 hours, a 25% $NH_4Cl$ solution (40 ml) is added and the mixture is concentrated in vacuo to remove most of the THF. The concentrate is extracted with ether (3x70 ml). The extracts are combined, washed with water (2x20 ml), dried ($MgSO_4$ anhydrous), filtered and the filtrate is evaporated to an oil. This is subjected to a flash chromatography on silica gel (LPS-1) eluting the column with ethyl acetatehexane (3:7) to isolate the title compound.

B. [1α,2β(Z),3β,4α]-2-[4-[3-[(Acetyl)thio]methyl]-7-oxabicyclo[2.2.1]hept-2-yl]-2-butenoic acid, methyl ester

To a cooled (ice bath) and stirred suspension of triphenylphosphine (8.0 mmole) in dry THF (30 ml) is added diisopropylazadicarboxylate (8.0 mmole). After 30 minutes, a solution of Part A alcohol compound (6.0 mmole) and thiol acetic acid (6.0 mmole) in dry THF (5.0 ml) is added. The mixture is then stirred in the ice bath for 30 minutes and at room temperature for 8 hours. It is then concentrated in vacuo and the residue is triturated twice with ether-hexane (1:3, 30 ml each) removing the insoluble solids by decantation. The solvent is then evaporated and the residue is flash chromatographed on a column of silica gel (LPS-1) eluting the column with ether-hexane (15:85) to isolate the title compound.

C. [1a,2β(Z),3β,4α]-2-[4-[3-[(Hexylthio)methyl]-7-oxabicyclo[2.2.1]hept-2-yl]-2-butenoic acid, methyl ester

A solution of Part B thiolacetate (4.0 mmole), anhydrous $K_2CO_3$ (12 mmole), and n-hexyl bromide (6.0 mmole) in argon-purged methanol (20 ml) is stirred under an atmosphere of argon for 18 hours. The mixture is then acidified with 1N HCl (12 ml) and is concentrated in vacuo. The concentrate is diluted with brine (25 ml) and is extracted with ether (3x20 ml). The extracts are combined, washed with water, dried ($MgSO_4$ anhydrous) and evaporated to afford an oil. This is purified by flash chromatography on a silica gel column (LPS-1) eluting with ether-hexane (15:85) to isolate the title compound.

D. [1α,2β(Z),3β,4α]-4-[3-[(Hexylthio)methyl]-7-oxabicyclo[2.2.1]hept-2-yl]-2-butenol

A solution of Part C thioether compound (4.0 mmole) in dry toluene (35 ml) is stirred in a bath at -78° under argon and a 1.5 M solution of diisobutyl aluminum hydride in toluene (8.0 ml) is added. After 4 hours, the mixture is added under stirring into 10% hydrochloric acid (30 ml) and is extracted with ether (3x40 ml). The extracts are combined, washed with water, dried ($MgSO_4$ anhydrous) and evaporated to afford the title compound.

E. [1α,2β(Z),3β,4α]-4-[3-[(Hexylthio)methyl]-7-oxabicyclo[2.2.1]hept-2-yl]-2-butenethiol, acetic acid ester

A solution of Part D thioether compound (3.0 mmole) is reacted under the conditions described above for the conversion of compound A into compound B to isolate the title compound.

F. [1α,2β(Z),3β,4α]-2-[[4-[3-[(Hexylthio)methyl]-7-oxabicyclo[2.2.1]hept-2-yl]-2-butenyl]thio]acetic acid, 2,2-(dimethyl) ethyl ester

A solution of Part E thioether compound (2.5 mmole) in argon-purged methanol (20 ml) containing anhydrous $K_2CO_3$ (7.5 mmole) and t-butylbromoacetate (5.0 mmole) is stirred under an atmosphere of argon for 6 hours. The mixture is then acidified with 1N HCl (8 ml) and is concentrated in vacuo. The concentrate is diluted with brine (20 ml) and is extracted with ether (3x20 ml). The extracts are combined, washed with water, dried ($MgSO_4$ anhydrous) and evaporated to afford an oil. This is purified by flash chromatography on a column of silica gel (LPS-1) eluting with ethyl acetate hexane (1:9) to isolate the title compound.

G. [1α,2β(Z),3β,4α]-2-[[4-[3-[(Hexylthio)methyl]-7-oxabicyclo[2.2.1]hept-2-yl]-2-butenyl]thio]acetic acid

A solution of Part F thioether compound (1.0 mmole) in dry $CH_2Cl_2$ (10 ml) is stirred in an ice bath, and trifluoroacetic acid (0.2 ml) and anisole (0.1 ml) is added. After 2 hours, the mixture is diluted with $CH_2Cl_2$ -(10 ml), washed with water (3x10 ml), dried ($MgSO_4$ anhydrous) and is evaporated to afford an oil. This is purified by chromatography on a column of silica gel (Baker 60-200 mesh) eluting with ether-hexane mixtures to isolate the title compound.

Example 10

(1α,2β,3β,4α)-2-[[4-[3-[2-(Hexylthio)ethyl]-7-oxabicyclo[2.2.1]hept-2-yl]butyl]oxy]-N-hydroxy-N-methylacetamide

A. [1α,2β(Z),3β,4α]-7-[[[3-(2-Oxo)ethyl-7-oxabicyclo[2.2.1]hept-2-yl]-butyl]oxy]acetic acid, methyl ester

Into a dry 100 ml round bottom 3-necked flask containing a stir bar was added dried 12.9 g (37.7 mmoles) methoxymethyltriphenylphosphonium chloride ($(C_6H_5)_3P^+$-$CH_2OCH_3Cl^-$) and 235 ml distilled toluene (stored over molecular sieves). The resulting suspension was stirred in an ice-bath, under argon, until cold and then a 1.55 M solution of 18.3 ml (28.3 mmol) of potassium t-amylate in toluene was added dropwise. A bright red solution formed which was stirred at 0°C for an additional 35 minutes. Thereafter, a solution of 4.97 g (18.8 mmol) (1α,2β,3β,4α)-2-[[4-[3-(formyl)-7-oxabicyclo[2.2.1]hept-2-yl]butyl]oxy]acetic acid, methyl ester in 60 ml toluene was added by means of a dropping funnel over a 35 minute period with the ice-bath still in place. After 5 hours, the reaction was quenched by addition of 2.3 g (39 mmol) acetic acid in 5 ml ether. The reaction mixture immediately turned pale yellow and was immediately poured into 200 ml saturated $NH_4Cl$, and extracted with ether (4 x 200 ml). The combined ether phases were washed with a saturated NaCl solution, dried ($MgSO_4$) and concentrated to yield a yellow oil in a white crystalline solid (phosphine oxide). The white solid removed after trituration with EtOAc and the mother liquor was treated with trifluoroacetic acid and was purified by chromatography to afford the title compound.

B. (1α,2β,3β,4α)-2-[[4-[3-(2-Hydroxyethyl)-7-oxabicyclo[2.2.1]hept-2-yl]butyl]oxy]acetic acid, methyl ester

The aldehyde (1.4 g, 5 mmol) from part A in methanol (50 ml) is treated with $NaBH_4$ (0.19 g, 5 mmol) in an argon atmosphere at 0°C. After stirring at 0° for 1 hour, the reaction is quenched by addition of 2N HCl (to pH 2). The methanol is removed in vacuo and the reaction mixture is taken up in ether. The ether solution is washed with saturated $KHCO_3$, saturated NaCl and dried ($MgSO_4$ anhydrous). The ether is evaporated to yield the title B compound.

C. (1α,2β,3β,4α)-2-[[4-[3-[2-(Hexylthio)ethyl]-7-oxabicyclo[2.2.1]hept-2-yl]butyl]oxy]-N-hydroxy-N-methylacetamide

Following the procedure of Example 6 except substituting the above part B alcohol for the alcohol used in Example 6 Part I, the title compound is obtained.

Example 11

(1α,2β,3β,4α)-2-[[4-[3-[4-(Hexylthio)butyl]-7-oxabicyclo[2.2.1]hept-2-yl]butyl]oxy]-N-hydroxy-N-methylacetamide

A. (1α,2β,3β,4α)-[[4-[3-(3-Oxo)propyl-7-oxabicyclo[2.2.1]hept-2-yl]butyl]oxy]acetic acid, methyl ester

Following the procedure of Example 10 Part A except substituting (1α,2β(Z),3β,4α)-7-[3-(2-oxo)ethyl-7-oxabicyclo[2.2.1]hept-2-yl]-5-heptenoic acid, methyl ester for [1α,2β(Z),3β,4α]-7-[3-formyl-7-oxabicyclo-[2.2.1]hept-2-yl]-5-heptenoic acid, methyl ester, the title A compound is obtained.

B. (1α,2β,3β,4α)-2-[[4-[3-(4-Oxo)butyl-7-oxabicyclo[2.2.1]hept-2-yl]butyl]oxy]acetic acid, methyl ester

Following the procedure of Example 10 Part A except substituting the aldehyde from Part A above for (1α,2β,3β,4α)-2-[[[4-[(formyl)-7-oxabicyclo[2.2.1]hept-2-yl]butyl)oxy]acetic acid, methyl ester, the title B compound is obtained.

C. (1α,2β,3β,4α)-2-[[4-[3-(4-Hydroxybutyl)-7-oxabicyclo[2.2.1]hept-2-yl]butyl]oxy]acetic acid, methyl ester

Following the procedure of Example 10 Part B except substituting the title B aldehyde for (1α,2β,3β,4α)-2-[[4-[3-(2-oxo)ethyl-7-oxabicyclo[2.2.1]hept-2-yl]butyl]oxy]acetic acid, methyl ester, the title C alcohol is obtained.

D. (1α,2β,3β,4α)-2-[[4-[3-[4-(Hexylthio)butyl]-7-oxabicyclo[2.2.1]hept-2-yl]butyl]oxy]-N-hydroxy-N-methylacetamide

Following the procedure of Example 6 except substituting the above Part C alcohol for the alcohol used in Example 1 Part I, the title compound is obtained.

Examples 12 to 55

Following the procedures outlined above and set out in the working Examples, the following additional compounds, in accordance with the following invention, may be prepared.

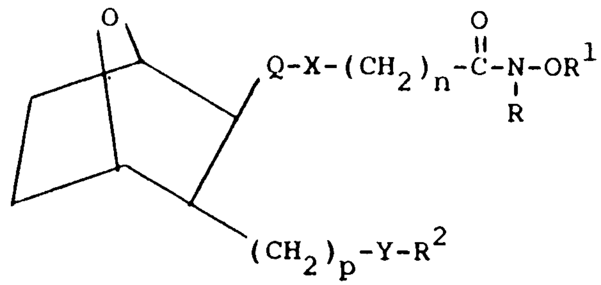

| Ex. No. | Q | X | $(CH_2)_n$ | R | $R^1$ | p | Y | $R^2$ |
|---|---|---|---|---|---|---|---|---|
| 12. | $-CH_2-CH=CH-(CH_2)_2-$ | O | $(CH_2)_2$ | $C_3H_7$ | $CH_3$ | 1 | O | $C_6H_5$ |
| 13. | $-CH_2-CH=CH-$ | O | $-CH_2CH(CH_3)-$ | $C_2H_5$ | H | 2 | O | $C_6H_5CH_2$ |
| 14. | $-CH_2-CH=CH-(CH_2)_3-$ | O | $-(CH_2)_3-$ | H | $C_2H_5$ | 2 | O | 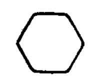 |
| 15. | $-CH_2-CH=CH-(CH_2)_4-$ | O | $-(CH_2)_4-$ | $CH_3$ | $C_6H_5$ | 1 | O | 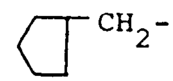 |
| 16. | $-CH_2CH=CH-CH_2-$ | O | $-(CH_2)_2-$ | $CH_3$ | $CH_3$ | 3 | O | $CH_3CH_2CH=CH-CH_2-$ |
| 17. | $-CH_2-CH=CH-$ | O | $-(CH_2)_3$ | H | H | 3 | O | $CH_3CH_2\equiv C-CH_2-$ |
| 18. | $-CH_2-(CH_2)_2-CH_2-$ | O | $(CH_2)_2-$ | $CH_3$ | $C_6H_5CH_2$ | 1 | O | $C_3H_7$ |
| 19. | $-CH_2-(CH_2)_2-$ | O | $-(CH_2)_3-$ | $C_6H_{13}$ | 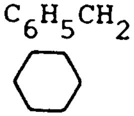 | 2 | O | $C_6H_5$ |

| Ex. No. | Q | X | $(CH_2)_n$ | R | $R^1$ | p | Y | $R^2$ |
|---|---|---|---|---|---|---|---|---|
| 20. | $-CH_2-(CH_2)_2-(CH_2)_2-$ | O | $-(CH_2)_4-$ | H | 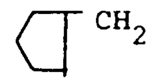 | 4 | O | $C_6H_5-(CH_2)_2-$ |
| 21. | $-CH_2-(CH_2)_2-(CH_2)_4-$ | O | $-(CH_2)_3$ | $C_2H_5$ | $CH_3C(=O)-$ | 2 | O | 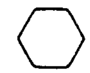 |
| 22. | $-CH_2-(CH_2)_3-(CH_2)_3-$ | O | $-(CH_2)_2-$ | $C_3H_7$ | $C_6H_5-C(=O)$ | 3 | O | 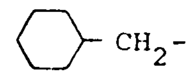 |
| 23. | $-CH_2-CH=CH-(CH_2)_2-$ | S | $(CH_2)_2$ | $(CH_3)_3C$ | $CH_3$ | 1 | O | $C_6H_5$ |
| 24. | $-CH_2-CH=CH-$ | S | $-CH_2CH(CH_3)-$ | $C_4H_9$ | H | 2 | O | $C_6H_5CH_2$ |
| 25. | $-CH_2-CH=CH-(CH_2)_3-$ | S | $-(CH_2)_3-$ | H | $C_2H_5$ | 2 | O | 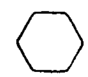 |
| 26. | $-CH_2-CH=CH-(CH_2)_4-$ | S | $-(CH_2)_4-$ | $CH_3$ | $C_6H_5$ | 1 | O | 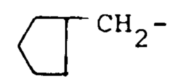 |
| 27. | $-CH_2CH=CH-CH_2-$ | S | $-(CH_2)_2-$ | $CH_3$ | $CH_3$ | 3 | O | $CH_3CH_2CH=CH-CH_2-$ |
| 28. | $-CH_2-CH=CH-$ | S | $-(CH_2)_3$ | H | H | 3 | O | $CH_3CH_2\equiv C-CH_2-$ |
| 29. | $-CH_2-(CH_2)_2-CH_2-$ | S | $(CH_2)_2-$ | $CH_3$ | $C_6H_5CH_2$ | 1 | O | $C_3H_7$ |
| 30. | $-CH_2-(CH_2)_2-$ | S | $-(CH_2)_3-$ | $CH_3$ | 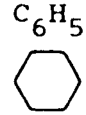 | 2 | O | $C_6H_5$ |

| Ex. No. | Q | X | $(CH_2)_n$ | R | $R^1$ | p | Y | $R^2$ |
|---|---|---|---|---|---|---|---|---|
| 31. | $-CH_2-(CH_2)_2-(CH_2)_2-$ | S | $-(CH_2)_4-$ | H | 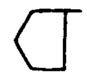 $CH_2$ | 4 | O | $C_6H_5-(CH_2)_2-$ |
| 32. | $-CH_2-(CH_2)_2-(CH_2)_4-$ | S | $-(CH_2)_3$ | $C_2H_5$ | $CH_3\overset{O}{\overset{\|}{C}}-$ | 2 | O | 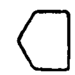 |
| 33. | $-CH_2-(CH_2)_3-(CH_2)_3-$ | S | $-(CH_2)_2-$ | $C_3H_7$ | $C_6H_5-\overset{O}{\overset{\|}{C}}$ | 3 | O | 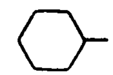 $CH_2-$ |
| 34. | $-CH_2-CH=CH-(CH_2)_2-$ | O | $(CH_2)_2$ | $CH_3$ | $CH_3$ | 1 | S | $C_6H_5$ |
| 35. | $-CH_2-CH=CH-$ | O | $-CH_2\overset{CH_3}{CH}-$ | $C_2H_5$ | H | 2 | S | $C_6H_5CH_2$ |
| 36. | $-CH_2-CH=CH-(CH_2)_3-$ | O | $-(CH_2)_3-$ | H | $C_2H_5$ | 2 | S | 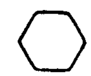 |
| 37. | $-CH_2-CH=CH-(CH_2)_4-$ | O | $-(CH_2)_4-$ | $CH_3$ | $C_6H_5$ | 1 | S | 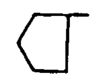 $CH_2-$ |
| 38. | $-CH_2CH=CH-CH_2-$ | O | $-(CH_2)_2-$ | $CH_3$ | $CH_3$ | 3 | S | $CH_3CH_2CH=CH-CH_2-$ |
| 39. | $-CH_2-CH=CH-$ | O | $-(CH_2)_3$ | H | H | 3 | S | $CH_3CH_2\equiv C-CH_2-$ |
| 40. | $-CH_2-(CH_2)_2-CH_2-$ | O | $(CH_2)_2-$ | $CH_3$ | $C_6H_5CH_2$ | 1 | S | $C_3H_7$ |
| 41. | $-CH_2-(CH_2)_2-$ | O | $-(CH_2)_3-$ | $CH_3$ | 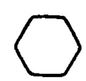 | 2 | S | $C_6H_5$ |

| Ex. No. | Q | X | $(CH_2)_n$ | R | $R^1$ | p | Y | $R^2$ |
|---|---|---|---|---|---|---|---|---|
| 42. | $-CH_2-(CH_2)_2-(CH_2)_2-$ | O | $-(CH_2)_4-$ | H | 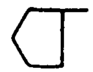 $CH_2$ | 4 | S | $C_6H_5-(CH_2)_2-$ |
| 43. | $-CH_2-(CH_2)_2-(CH_2)_4-$ | O | $-(CH_2)_3$ | $C_2H_5$ | $C_4H_9C(=O)$ | 2 | S | 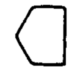 |
| 44. | $-CH_2-(CH_2)_3-(CH_2)_3-$ | O | $-(CH_2)_2-$ | $C_3H_7$ | $C_6H_5-C(=O)$ | 3 | S | 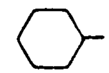 $CH_2-$ |
| 45. | $-CH_2-CH=CH-(CH_2)_2-$ | S | $(CH_2)_2$ | $CH_3$ | $CH_3$ | 1 | S | $C_6H_5$ |
| 46. | $-CH_2-CH=CH-$ | S | $-CH_2CH(CH_3)-$ | $C_2H_5$ | H | 2 | S | $C_6H_5CH_2$ |
| 47. | $-CH_2-CH=CH-(CH_2)_3-$ | S | $-(CH_2)_3-$ | H | $C_2H_5$ | 2 | S | 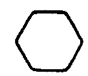 |
| 48. | $-CH_2-CH=CH-(CH_2)_4-$ | S | $-(CH_2)_4-$ | $CH_3$ | $C_6H_5$ | 1 | S | 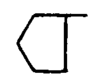 $CH_2-$ |
| 49. | $-CH_2CH=CH-CH_2-$ | S | $-(CH_2)_2-$ | $C_2H_5$ | $CH_3$ | 3 | S | $CH_3CH_2CH=CH-CH_2-$ |
| 50. | $-CH_2-CH=CH-$ | S | $-(CH_2)_3$ | H | H | 3 | S | $CH_3CH_2\equiv C-CH_2-$ |
| 51. | $-CH_2-(CH_2)_2-CH_2-$ | S | $(CH_2)_2-$ | $CH_3$ | $C_6H_5CH_2$ | 1 | S | $C_3H_7$ |
| 52. | $-CH_2-(CH_2)_2-$ | S | $-(CH_2)_3-$ | $CH_3$ | 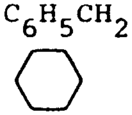 | 2 | S | $C_6H_5$ |

| Ex. No. | Q | X | $(CH_2)_n$ | R | $R^1$ | p | Y | $R^2$ |
|---|---|---|---|---|---|---|---|---|
| 53. | $-CH_2-(CH_2)_2-(CH_2)_2-$ | S | $-(CH_2)_4-$ | H | 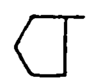 $CH_2$ | 4 | S | $C_6H_5-(CH_2)_2-$ |
| 54. | $-CH_2-(CH_2)_2-(CH_2)_4-$ | S | $-(CH_2)_3$ | $C_2H_5$ | $CH_3\overset{O}{\overset{\|}{C}}-$ | 2 | S | 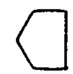 |
| 55. | $-CH_2-(CH_2)_3-(CH_2)_3-$ | S | $-(CH_2)_2-$ | $C_3H_7$ | $C_6H_5-\overset{O}{\overset{\|}{C}}$ | 3 | S | 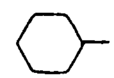 $CH_2-$ |

Example 56

[1R-(1α,2β,3β,4α)]-4-[[[3-[(Hexyloxy)methyl]-7-oxabicyclo[2.2.1]hept-2-yl]ethyl]thio]-N-hydroxy-N-methylbutanamide

A. (1α,2β,3β,4α)-2-[[(Chlorocarbonyl)oxy]methyl]-3-(hydroxy)methyl-7-oxabicyclo[2.2.1]heptane

To a solution of 10 g Example 6 Part A diol (63.2 mmole) in 40 ml dry THF at 0° C was added with stirring 55 ml of a 12.5% by weight solution of phosgene in toluene (63.2 mmole, 1 eq.) dropwise over a period of 30 minutes. Argon was then bubbled through the reaction mixture for 15 minutes. The mixture was concentrated to give title compound as a crude oil.

B. (1α,2β,3β,4α)-2,3-Bis(hydroxymethyl)-7-oxabicyclo[2.2.1]heptane, 2,3-cyclic carbonate

Part A oil was dissolved in 30 ml of dry $CH_2Cl_2$ and cooled to -50° C. To this solution was added dropwise a solution of 10 ml pyridine in 10 ml $CH_2Cl_2$. It was stirred for 10 minutes and quenched with $H_2O$. The mixture was extracted thoroughly with $CH_2Cl_2$. The organic extract was dried over $MgSO_4$ and concentrated to give the title cyclic carbonate as a crystalline solid (10.7 g).

C. (1α,2β,3β,4α)-2-Hydroxymethyl-3-[[[(1-methylethyl)oxy]carbonyl]oxy]methyl-7-oxabicyclo[2.2.1]heptane

A mixture of 10.7 g Part B cyclic carbonate (58.1 mmole) in 100 ml isopropanol was refluxed for 24 hours. Excess isopropanol was removed under reduced pressure to give 14.4 g title hydroxycarbonate as a viscous oil.

D. (1α,2β,3β,4α)-2-Hydroxymethyl-3-[[[(1-methylethyl)oxy]carbonyl]oxy]methyl-7-oxabicyclo[2.2.1]heptane, 2-[4-methyl(phenyl)]sulfonic acid ester

To a solution of 19.7 g Part C hydroxycarbonate (80 mmole) in 30 ml $CH_2Cl_2$ and 12.8 ml pyridine (160 mmole, 2 eq.) was added 18.5 g of p-toluenesulfonyl chloride (96 mmole, 1.2 eq.). The mixture was stirred at 25° C for 36 hours, then diluted with 200 ml ether, and washed with 100 ml brine. The organic layer was dried over $MgSO_4$ and concentrated to give 32.8 g of crude title tosylate as an oil.

E. (1α,2β,3β,4α)-2-Cyanomethyl-3-[[[(1-methylethyl)oxy]carbonyl]oxy]methyl-7-oxabicyclo[2.2.1]heptane

To a solution of 24.0 g crude Part D tosylate (60 mmole) in 20 ml DMSO was added with stirring 6.0 g powdered sodium cyanide (120 mmole, 2 eq.). The mixture was heated at 90°-95° C for 1.5 hours under an argon atmosphere. The cooled mixture was diluted with 50 ml water and extracted with five 100 ml portions of ether. The ethereal extracts were dried over anhydrous $MgSO_4$ and filtered through a bed of Florosil. The filtrate was concentrated, and the residue was recrystallised with ether/hexanes to give 8.4 g title cyanocarbonate as a light yellow crystalline solid.

F. [1R-(1α,2β,3β,4α)]-2-Cyanomethyl-3-hydroxymethyl-7-oxabicyclo[2.2.1]heptane

To 8.4 g Part E cyanocarbonate (33.2 mmole) was added 75 ml of a 1% solution of potassium carbonate in methanol-water (2:1). The reaction mixture was stirred at 25° C for 6 hours then acidified with 2N HCl solution, saturated with sodium chloride and extracted with six 100 ml portions of $CH_2Cl_2$. The combined organic layer was dried over anhydrous $MgSO_4$ and concentrated to give 5.5 g of crude title cyanoalcohol (compound II) as a light yellow oil.

G. [1R-(1α,2β,3β,4α)]-2-Cyanomethyl-3-[[[(1,1-dimethyl)ethyl]dimethyl]silyl]oxy-7-oxabicyclo[2.2.1]heptane

To a solution of 5.0 g Part F alcohol (30 mmole) in 50 ml of dry $CH_2Cl_2$ and 10 ml of triethylamine (70 mmole, 3.3 eq.) at 0° C was added with stirring 490 mg 4-dimethylaminopyridine (4 mmole) and 5.28 g t-butyldimethylsilylchloride (35 mmole, 1.16 eq.). The reaction mixture was slowly warmed to 25° C and stirred for 18 hours, then diluted with 200 ml ether, and filtered through a small bed of anhydrous $MgSO_4$. The filtrate was concentrated. Purification was done on a silica gel column, eluting with 15% ethyl acetate in hexanes to give 10.25 g of title silyl ether as a light yellow oil.

H. [1R-(1α,2β,3β,4α)]-2-[[[3-[(1,1-dimethyl)ethyl]dimethyl]silyl]oxy-7-oxabicyclo[2.2.1]hept-2-yl]acetaldehyde

To a solution of 10.0 g of Part G silyl ether (26.2 mmole) in 30 ml of dry toluene at -78 °C under an argon atmosphere was added dropwise 25 ml of a 25% by weight solution of diisobutylaluminumhydride (44 mmole) in toluene. The mixture was stirred at -78 °C for 4 hours, quenched at -78° with a saturated solution of ammonium chloride, warmed to 0 °C and acidified with 1N HCl solution, extracted with three 100 ml portions of $CH_2Cl_2$, dried over anhydrous $MgSO_4$ and concentrated to give 9.3 g of crude title aldehyde.

J. [1R-(1α,2β,3β,4α)]-2-[[[3-(3-(1,1-dimethyl)ethyl]dimethyl]silyl]oxy-7-oxabicyclo[2.2.1]hept-2-yl]ethanol

To 9.3 g crude Part H aldehyde (32.7 mmole) in 30 ml of dry THF at 0 °C under an argon atmosphere was added portionwise 1.0 g lithium aluminum hydride (26.0 mmole, 3.2 eq.) with stirring. The reaction mixture was stirred while being warmed to 25 °C over a period of 1 hour, quenched by slow addition of a saturated sodium sulfate solution at 0 °C, dried over anhydrous $MgSO_4$ and filtered. The solid was washed with $CH_2Cl_2$. The combined filtrate was concentrated to give a crude oil. This oil was purified on a silica gel column, eluting with 30% EtOAc in hexanes to give 8.55 g title alcohol as a colorless oil.

K. [1R-(1α,2β,3β,4α)]-2-[(Acetylthio)ethyl]-3-[[[(1,1-dimethyl]silyl]oxy-7-oxabicyclo[2.2.1]heptane

To a solution of 5.25 g triphenylphosphine (20 mmole, 2 eg.) in 60 ml dry THF at 0 °C was added dropwise 4.16 g diisopropylazodicarboxylate (20 mmole, 2 eg.) over a period of 15 minutes. The mixture was stirred at 0 °C for 30 minutes and a solution of 2.6 g Part J alcohol (10 mmole) and 1.45 ml of thiolacetic acid (20 mmole, 2 eq.) in 10 ml dry THF was add dropwise. The reaction mixture was stirred at 0 °C for 1 hour and at 25 °C for 3 hours and then concentrated. The residue was triturated with ether/hexane, filtered, and the filtrate was concentrated and purified on a silica gel column, eluting with 10% EtOAc in hexanes to give 2.3 g title thioacetate as a light yellow oil.

L. [1R-(1α,2β,3β,4α)]-2-[[3-(Acetylthio)ethyl]-7-oxabicyclo[2.2.1]hept-2-yl]methanol

To a solution of 2.3 g Part K thioacetate (6.7 mmole) in 20 ml dry THF at 0 °C was added 2.23 g tetra-n-butylammoniumfluoride trihydrate (7.07 mmole, 1.05 eq.) in 5 ml dry THF. The reaction mixture was warmed to 25 °C, stirred for 18 hours, diluted with 100 ml ether, washed with 30 ml saturated $NaHCO_3$ solution, dried over anhydrous $MgSO_4$ and concentrated to give a crude oil. Purification was done on a silica gel column, eluting with 20% EtOAc in hexane and 50% EtOAc in hexane to give 1.22 g of title alcohol thioacetate as a colorless oil.

M. [1R-(1α,2β,3β,4α)]-2-[[3-(Mercaptoethyl)-7-oxabicyclo[2.2.1]hept-2-yl]methanol

To a slurry of 200 mg of lithium aluminumhydride (5.27 mmole, 4 eq.) in 20 ml dry THF at 0 °C was added dropwise a solution of 1.22 g of Part L thioacetate (5.3 mmole) in 5 ml THF under an argon atmosphere. The reaction mixture was stirred at 0 °C for 1 hour and quenched with a saturated sodium sulfate solution, dried with anhydrous $MgSO_4$ and filtered. The filtrate was concentrated to give 900 mg of title thiol as a colorless oil.

N. [1R-[1α,2β,3β(1E,3R),4α]]-4-[[2-(3-Hydroxymethyl)-7-oxabicyclo[2.2.1]hept-2-yl]ethyl]thio]-2-butenoic acid, methyl ester

To a slurry of 1.38 g of dried and powdered potassium carbonate (10 mmole, 2.1 eq.) in 20 ml dry acetone at 0 °C was added a solution of 900 mg Part M thiol (4.8 mmole) in 5 ml acetone, followed by 1.75 ml of methyl-4-bromocrotonate (15 mmole, 3 eq.). The reaction mixture was stirred at 0 °C for 10 hours then diluted with 100 ml ether and filtered through a pad of anhydrous $MgSO_4$. The filtrate was concentrated. The residue was purified on a silica gel column, eluting with 20% EtOAc in hexane and 50% EtOAc in hexane to give 823 mg of title ester as a colorless oil.

O. [1R-(1α,2β,3β,4α)]-4-[[2-(3-Hydroxymethyl)-7-oxabicyclo[2.2.1]hept-2-yl]ethyl]thio]butanoic acid, methyl ester

A mixture of 570 mg of Part N olefin ester (2.0 mmole), and 600 mg of 10% palladium over carbon in 10 ml methanol was shaken in a Parr bottle under 40 psi hydrogen pressure, at 25°C for 18 hours and was filtered. The filtrate was concentrated to give 470 mg of the title ester as an oil.

P. [1R-(1α,2β,3β,4α)]-4-[[[3-[(Hexyloxy)methyl]-7-oxabicyclo[2.2.1]hept-2-yl]ethyl]thio]butanoic acid, hexyl ester

A suspension of 583 mg of powdered potassium hydroxide (9.7 mmole, 10 eg.) in 100 ml of dry xylene was heated to reflux and 50 ml of xylene was distilled off. To the solution was slowly added 1.7 g of hexyl mesylate (9.7 mmole, 10 eq.) and Part O alcohol (0.97 mmol) in xylene (20 ml). The mixture was refluxed for 2 hours, cooled to 25°C, diluted with 200 ml of ether and washed with two 50 ml portions of $H_2O$. The organic layer was dried over anhydrous $MgSO_4$ and concentrated. The residue was purified on a silica gel column, eluting with 20% EtOAc in hexane to give the title compound as an oil (contaminated with small amount of hexyl mesylate).

Q. [1R-(1α,2β,3β,4α)]-4-[[[3-[(Hexyloxy)methyl]-7-oxabicyclo[2.2.1]hept-2-yl]ethyl]thio]butanoic acid

To 200 mg of crude Part P ester (ca. 0.45 mmole) in 30 ml of THF and 20 ml of $H_2O$ saturated with argon at 0°C was added 4.5 ml of a 1M lithium hydroxide solution. The mixture was stirred at 25°C for 20 hours and concentrated. The residue was diluted with 10 ml of $H_2O$ and acidified to pH 3 with a saturated aqueous solution of oxalic acid. The aqueous solution was extracted with three 40 ml portions of ether. The combined organic layer was washed with two 40 ml portions of $H_2O$, dried over anhydrous $MgSO_4$ and concentrated. The residue was purified on a silica gel column, eluting with a gradient of pentane/ether to yield 148 mg of title acid.
TLC: Silica gel; MeOH/$CH_2Cl_2$ (7:93); $R_f$ ~ 0.55.

| | | | |
|---|---|---|---|
| Anal Calcd for $C_{19}H_{34}O_4S$: | C, 63.64; | H, 9.56; | S, 8.94 |
| Found: | C, 63.41; | H, 9.52; | S, 8.70 |

R. [1R-(1α,2β,3β,4α)]-4-[[[[3-(Hexyloxy)methyl]-7-oxabicyclo[2.2.1]hept-2-yl]methyl]thio]-N-hydroxy-N-methyl butanamide

A solution of Part Q acid (1.82 mmole) and oxalyl chloride (3.6 mmole) in dry benzene (10 ml) is cooled down to 0° (ice-water bath), treated with a solution of dimethylformamide (3 drops) in benzene and stirred at 0° for 30 minutes under nitrogen and at room temperature for one hour. The excess oxalyl chloride and solvent are blown off under a stream of nitrogen while heating the flask in a warm water bath and the residual oil is dried in vacuo (pump) for one hour. This acid chloride is dissolved in dry tetrahydrofuran (3.5 ml) and added dropwise under stirring into a cold solution (~0°, ice-water) of 98% methylhydroxylamine hydrochloride (318.7 mg, 3.74 mmole) and triethylamine (0.92 ml, 7.48 mmole) in tetrahydrofuran (4.6 ml) and water (4.6 ml). The mixture is stirred at 0° for 30 minutes, diluted with water (25 ml) and extracted twice with dichloromethane (125 ml). The combined organic extracts are washed with 1N HCl (25 ml), 5% $NaHCO_3$ (12 ml) and brine (20 ml), dried (anhydrous $MgSO_4$), filtered and evaporated to dryness giving an oil containing the desired product.

Example 56A

(1α,2β,3β,4α)-5-[[[3-[(Hexyloxymethyl]-7-oxabicyclo[2.2.1]hept-2-yl]methyl]thio]-N-hydroxy-N-methylpentanamide

A. (1α,2β,3β,4α)-cis-exo-[[3-Isopropyloxycarbonyloxymethyl-7-oxabicyclo[2.2.1]hept-2-yl]methyl]thioacetate

To a solution of 10.5 g of triphenylphosphine (40 mmole) in 100 ml of dry THF at 0°C was added dropwise 95% pure diisopropylazo- dicarboxylate (40 mmole) over a period of 15 minutes. After stirring for 30 minutes, a solution of 4.88 g of Example 56 title C alcohol carbonate (20 mmole) and 1.43 ml of distilled thiol acetic acid (20 mmole) in 10 ml of dry THE was added dropwise over a period of 20 minutes. The mixture was stirred at 0°C for 30 minutes and at 25°C for 1 hour, and then concentrated. The residue was triturated with ether/hexane, and then filtered. The filtrate was concentrated and purified on a silica gel column, eluting with 5% ethyl acetate in hexane followed by 10% ethyl acetate in hexane to give 5.12 g of title thioacetate as a colorless crystalline solid.

B. (1α,2β,3β,4α)-cis-exo-[3-(Hydroxymethyl)-7-oxabicyclo[2.2.1]hept-2-yl]-2-methanethiol

To a slurry of 400 mg of 95% pure lithium aluminum hydride (13 mmole) in 25 ml of dry THF at 0°C under an argon atmosphere was added dropwise a solution of 1.9 g of title A thioacetate (6 mmole) in 100 ml of dry THF. The mixture was stirred at 0°C for 30 minutes and at 25°C for 1 hour and then quenched with a saturated sodium sulfate solution. The mixture was dried with anhydrous $MgSO_4$ and filtered. The filtrate was concentrated to give the crude title thio-alcohol as an oil.

This oil was used in the next step without purification.

C. (1α,2β,3β,4α)-5-[[[3-(Hydroxymethyl)-7-oxabicyclo[2.2.1]hept-2-yl]methyl]thio]pentanoic acid, methyl and ethyl esters

To a slurry of 480 mg of 50% sodium hydride in mineral oil (10 mmole) in 20 ml of dry THF at 0°C was added dropwise a solution of 820 mg of title B thioalcohol (4.71 mmole) in 5 ml of dry THF under nitrogen. After starring for 20 minutes at 0°C, a solution of 3.17 ml of ethyl-5-bromo-valerate (20 mmole) was added dropwise. The reaction mixture was stirred at 0°C for 2 hours and then quenched with a saturated solution of ammonium chloride. The layers were separated. The aqueous layer was acidified with a 2N HCl solution and extracted several times with $CH_2Cl_2$. The combined organic layer was dried over anhydrous $MgSO_4$ and concentrated. The residue was diluted with 25 ml of ether and treated with an etheral solution of diazomethane.

Purification was done on a silica gel column, eluting with 10% EtOAc/hexane followed by 20% EtOAc/hexane to give 540 mg of a mixture of title methyl and ethyl esters as a colorless oil.

D. (1α,2β,3β,4α)-5-[[[3-[(Hexyloxy)methyl]-7-oxabicyclo[2.2.1]hept-2-yl]methyl]thio]pentanoic acid, hexyl ester

To a solution of 583 mg of powdered potassium hydroxide (9.7 mmole) in 50 ml of dry xylene was added a solution of 266.5 mg of title C alcohol (0.97 mmole) in 50 ml of dry xylene. The mixture was heated to reflux and 50 ml of xylene was distilled off. To the cooled remaining solution was added 1.7 g of hexyl mesylate (9.7 mmole, 10 eq.). The reaction was carried out under an atmosphere of nitrogen. The mixture was refluxed for 2 hours then cooled to 25°C, diluted with 200 ml of ether and washed with two 50 ml portions of $H_2O$. The organic layer was dried over anhydrous $MgSO_4$ and concentrated. The residue was purified on a silica gel column, eluting with 20% EtOAc in hexanes to give 200 mg of title oil (contaminated with a small amount of hexyl mesylate).

E. (1α,2β,3β,4α)-5-[[[3-[(Hexyloxy)methyl]-7-oxabicyclo[2.2.1]hept-2-yl]methyl]thio]pentanoic acid

To 200 mg of crude Part D compound (ca. 0.45 mmole) in 80 ml of THF and 20 ml of $H_2O$ saturated with argon at 0°C was added 4.5 ml of a 1M lithium hydroxide solution. The mixture was stirred at 25°C for 20 hours and concentrated *in vacuo*. The residue was diluted with 10 ml of $H_2O$ and acidified to pH 3 with a saturated aqueous solution of oxalic acid. The aqueous solution was extracted with three 40 ml portions of ether. The combined organic extracts were washed with two 40 ml portions of $H_2O$, dried over anhydrous

$MgSO_4$ and with two 40 ml portions of $H_2O$, dried over anhydrous $MgSO_4$ and concentrated. The residue was purified on a CC-7 silica gel column, eluting with a gradient of pentane/ether to yield the title compound.
TLC: silica gel; 7% MeOH in $CH_2Cl_2$; $R_f$~0.55.

| | | | |
|---|---|---|---|
| Anal Calcd for $C_{19}H_{34}O_4S$: | C, 63.64; | H, 9.56; | S, 8.94 |
| Found: | C, 63.41; | H, 9.52; | S, 8.70 |

F. (1α,2β,3β,4α)-5-[[[3-[(Hexyloxymethyl]-7-oxabicyclo[2.2.1]hept-2-yl]methyl]thio]-N-hydroxy-N-methylpentanamide

Following the procedure of Example 56 Part R, the title compound is obtained.

Example 57

(1α,2β,3β,4α)-4-[[2-[3-[(Phenoxy)methyl]-7-oxabicyclo[2.2.1]hept-2-yl]ethyl]thio]-N-hydroxy-N-methyl-4-butanamide

A. (1α,2β,3β,4α)-2-[3-[(Phenoxy)methyl]-7-oxabicyclo[2.2.1]hept-2-yl]ethanol

A solution of 1.44 g (5.66 mmol) of (1α,2β,3β,4α)-2-[3-[(phenoxy)methyl]-7-oxabicyclo[2.2.1]hept-2-yl-acetaldehyde in dry tetrahydrofuran (20 ml) was cooled down to 0° and treated with 173.4 mg (4.57 mmole) of lithium aluminum hydride under argon. The mixture was allowed to warm up to 25° over 1.5 hour and was then treated with 4 ml $Na_2SO_4$ and stirred for 30 minutes. The mixture was diluted with 50 ml $CH_2Cl_2$, stirred for 30 minutes and filtered. The precipitates were washed with another 50 ml $CH_2Cl_2$; the organic solutions were combined and dried over anhydrous $MgSO_4$. The solutions were filtered and stripped to dryness to yield 1.37 g of liquid (after evacuating for 4 hours). The crude product mixture was dissolved in 25 ml $CH_2Cl_2$ and flash chromatographed on a silica gel column (LPS-1) using ethyl acetate:hexane (1:3), and ethyl acetate:hexane (1:1). The fractions containing the desired product were collected and concentrated to give 1.22 g of the title compound.

B. (1α,2β,3β,4α)-[2-[3-[(Phenoxy)methyl]-7-oxabicyclo[2.2.1]hept-2-yl]ethyl]thioacetate

Triphenylphosphine (2.31 g, 8.72 mmole) was suspended and stirred in dry tetrahydrofuran (37 ml) under $N_2$ at 0°, and treated dropwise over a period of 15 minutes with diisopropylazodicarboxylate (1.8 ml, 8.89 mmole). After 30 minutes the suspension was treated with a solution of 1.12 g (4.37 mmole) of [1α,2β,3β,4α]-2-[3-[(hexyloxy)methyl]-7-oxabicyclo[2.2.1]hept-2-yl]ethanol and thiolacetic acid (0.63 ml, 8.81 mmole) in dry tetrahydrofuran (5 ml). The mixture was stirred at 0° for 1 hour, at room temperature for 4 hours and was concentrated to a syrup in vacuo. The syrup was triturated with $Et_2O$:Hexane (1:4, 100 ml) and the precipitates that formed were filtered off and washed with $Et_2O$:Hexane (1:4, 100 ml). The clear filtrate and washings were combined and were concentrated to give 4.64 g of a semi-solid containing the desired product.

The above product mixture was chromatographed (flash) on a silica gel column (LPS-1) eluting the column with $Et_2O$:Hexane (1:9; 4.6 liters). The fractions containing the desired title compound were combined and concentrated to give 1.5 g (100%) of an oil with consistent $^1H$ and $^{13}C$ spectral data.

C. (1α,2β,3β,4α)-[2-[3-[(Phenoxy)methyl]-7-oxabicyclo[2.2.1]hept-2-yl]ethanethiol

The Part B thioacetate (514 mg, 1.63 mmole) was dissolved in dry tetrahydrofuran (5 ml) and added to a suspension of 75 mg (1.98 mmole) of LAH in dry tetrahydrofuran (5 ml) at 0° under $N_2$. The mixture was stirred at 0° for 1.5 hours and was then quenched cautiously by successive additions of water (0.08 ml), 10% NaOH (0.12 ml) and water (0.24 ml). The mixture was stirred for 30 minutes, diluted with dichloromethane (25 ml) and filtered, washing the solids with $CH_2Cl_2$ (35 ml). The clear filtrate and washings were combined, dried (anhydrous $MgSO_4$) and concentrated to give 396 mg (89.2%) of title thiol compound as a homogeneous (tlc) oil with a consistent $^1H$ spectrum.

D. (1α,2β,3β,4α)-4-[[2-[3-[(Phenoxy)methyl]-7-oxabicyclo[2.2.1]hept-2-yl]ethyl]thio]butanoic acid, methyl ester

Part C thiol (500 mg, 5.8 mmole) and 1.0 g of powdered anhydrous $K_2CO_3$ were stirred in dry acetone (20 ml) under $N_2$ for a few minutes at room temperature and treated with a solution of 738 mg (5.4 mole) of 4-chloromethylbutyrate in dry acetone (5.0 ml). The mixture was stirred at room temperature for 48 hours, diluted with ether (100 ml) and filtered. The filtrate was dried (anhydrous $MgSO_4$), filtered and was concentrated to give an oil (1.47 g) containing the desired product and three manor components (tlc). The mixture was chromatographed (flash) on a silica gel column (LPS-1), eluting with $Et_2O$:Hexane (1:4, 3.5 liters) to give, after drying in vacuo, the title ester as an oil (496 mg, 74%) with consistent analytical, $H^1$-NMR, $C^{13}$-NMR, mass, and IR spectral data.

E. (1α,2β,3β,4α)-4-[[2-[3-[(Phenoxy)methyl]-7-oxabicyclo[2.2.1]hept-2-yl]ethyl]thio]butanoic acid

Following the procedure of Example 56 Part Q except substituting the above Part D ester for the Example 56 Part P ester, the title acid is obtained.

F. (1α,2β,3β,4α)-4-[[2-(3-[(Phenoxy)methyl]-7-oxabicyclo[2.2.1]hept-2-yl]ethyl]thio]-N-hydroxy-N-methyl-4-butanamide

Following the procedure of Example 56 Part R except substituting the above Part E acid for the Example 56 Part Q acid, the title compound is obtained.

Example 58

(1α,2β,3β,4α)-[[5-[[3-[(Hexyloxy)methyl]-7-oxabicyclo[2.2.1]hept-2-yl]-methyl]oxy]-N-hydroxy-N-methylpentanamide

A. (1α,2β,3β,4α)-Cis-exo-2-hydroxymethyl-3-benzyloxymethyl-7-oxabicyclo[2.2.1]heptane

To a suspension of 3.08 g of ether-washed sodium hydride (70 mmole, 50% oil dispersion) in 100 ml of dry DMF was added with stirring at 0 °C a solution of 10.0 g Example 56A diol (64 mmole) in 30 ml of DMF over a period of 15 minutes. The mixture was stirred for 30 minutes at 0 °C, 20 minutes at 25 °C, cooled to 0 °C and 12.0 g of benzyl bromide (70 mmole) was added dropwise. After stirring at 25 °C for 2 hours, the reaction was quenched with an aqueous ammonium chloride solution, extracted with ether, dried over anhydrous $MgSO_4$ and concentrated to a residue.

Purification of the residue was done on a silica gel column, eluting with 10-20% ethyl acetate in hexane to give 11.8 g of the title monobenzylether.

B. (1α,2β,3β,4α)-5-[[[3-Benzyloxymethyl-7-oxabicyclo[2.2.1]hept-2-yl]methy]oxy]pentanol, [[(1,1-dimethyl)-ethyl]dimethyl]silyl ether

To a mixture of 6.73 g powdered potassium hydroxide (121 mmole) in 20 ml of dry xylene was added a solution of 3.0 g of title A alcohol (12.1 mmole) in 10 ml of xylene. The mixture was heated to reflux and 15 ml of xylene was distilled off.

To the remaining solution was added a solution of 6.18 g of 5-tert-butyldimethylsilyloxy n-pentyl-mesylate in 10 ml of xylene. The resulting mixture was refluxed for 1 hour, cooled to 25 °C and diluted with 300 ml of ether. The ethereal solution was washed with two 50 ml portions of water, dried over anhydrous $MgSO_4$ and concentrated.

The residue was purified on a silica gel column, eluting with 20% ether in hexane to give 4.0 g of title compound as a yellow oil.

C. (1α,2β,3β,4α)-5-[[[3-Benzyloxymethyl-7-oxabicyclo[2.2.1]hept-2-yl]methyl]oxypentanol

To 536.5 mg of title B compound (1.19 mmole) in 2 ml of THF at 0 ° C was added 755.4 mg of tetra-n-butylammonium fluoride. The mixture was stirred at 0 ° C for 2 hours and at 25 ° C for 1 hour, then diluted with 50 ml of ether. The ethereal solution was washed with two 10 ml portions of $H_2O$, 10 ml of brine, dried over anhydrous $MgSO_4$ and concentrated to give crude title alcohol as an oil. This was used without further purification.

D. (1α,2β,3β,4α)-5-[[[3-Benzyloxymethyl-7-oxabicyclo[2.2.1]hept-2-yl]methyl]oxy]pentanoic acid,

and

E. (1α,2β,3β,4α)-5-[[3-Benzyloxymethyl-7-oxabicyclo[2.2.1]hept-2-yl]methyl]oxy]pentanoic acid, methyl ester

To crude title C alcohol in 10 ml of acetone at 0 ° C was added dropwise a solution of 2.67 M Jones reagent until the reaction mixture remained brown. The mixture was stirred for an additional 30 minutes at 0 ° C, quenched with isopropanol and diluted with 200 ml of ether. It was washed with 100 ml of saturated $NaHCO_3$ solution. The aqueous layer was acidified with concentrated HCl, saturated with solid NaCl and extracted with five 50 ml portions of $CH_2Cl_2$, dried over anhydrous $MgSO_4$ and concentrated to give 260 mg of title D acid, as an oil.

The above acid, dissolved in 10 ml of ether, was treated with an ethereal solution of diazomethane to give 260 mg of title E ester.

F. (1α,2β,3β,4α)-5-[[[3-Hydroxymethyl-7-oxabicyclo[2.2.1]hept-2-yl]methyl]oxy]pentanoic acid, methyl ester

A mixture of 260 mg title E ester (0.71 mmole) and 130 mg of 10% palladium over carbon in 5 ml of ethylacetate was shaken in a Parr bottle under 40 lbs of hydrogen pressure, at 25 ° C for 18 hours. The reaction mixture was filtered through a bed of Celite and the filtrate was concentratred to give 200 mg of title G alcohol as an oil.

G. (1α,2β,3β,4α)-5-[[[3-[(Hexyloxy)methyl]-7-oxabicyclo[2.2.1]hept-2-yl]methyl]oxy]pentanoic acid, hexyl ester

To a solution of 504 mg of powdered potassium hydroxide (8.4 mmole) in 40 ml of dry xylene was added a solution of 217 mg of title F alcohol (0.84 mmole) in 40 ml of xylene. The mixture was heated to reflux and 40 ml of xylene was distilled off. To the remaining solution was added 1.5 g of hexyl mesylate (8.4 mmole). The mixture was refluxed for 3 hours, cooled to 25 ° C, diluted with 200 ml of ether and washed with two 50 ml portions of $H_2O$. The organic layer was dried over anhydrous $MgSO_4$ and concentrated. The residue was purified on a silica gel column, eluting with 20% EtOAc in hexanes to give 200 mg of title oil (contaminated with some hexyl mesylate).

H. (1α,2β,3β,4α)-5-[[[3-[(Hexyloxy)methyl]-7-oxabicyclo[2.2.1]hept-2-yl]methyl]oxy]pentanoic acid

To 200 mg of crude Part G ester compound (ca. 0.46 mmole) in 80 ml of THF and 20 ml of $H_2O$ at 0 ° C was added 4.6 ml of 1M lithium hydroxide solution. The mixture was stirred at 25 ° C for 20 hours then concentrated. The residue was diluted with 10 ml of $H_2O$ and acidified with a saturated aqueous oxalic acid solution to pH 3. The aqueous solution was extracted with three 40 ml portions of ether. The combined organic layer was washed with two 40 ml portions of $H_2O$, dried over anhydrous $MgSO_4$ and concentrated. The residue was purified on a CC-7 silica gel column, eluting with a gradient of pentane/ether to yield 155 mg of title compound as a clear oil.
TLC: silica gel; 7% MeOH in $CH_2Cl_2$; $R_f$~0.4.

| | | |
|---|---|---|
| Anal Calcd for $C_{19}H_{34}O_5$: | C, 66.63; | H, 10.00 |
| Found: | C, 66.75; | H, 9.82 |

I. (1α,2β,3β,4α)-5-[[[3-[(Hexyloxy)methyl]-7-oxabicyclo[2.2.1]hept-2-yl]methyl]oxy]-N-hydroxy-N-methylpentanamide

Following the procedure of Example 56 Part R except substituting the Part I acid for the Example 56 Part Q acid, the title compound is obtained.

Example 59

(1α,2β,3β,4α)-5-[[[3-[(Hexylthio)methyl]-7-oxabicyclo[2.2.1]hept-2-yl]methyl]oxy]-N-hydroxy-N-methylpentanamide

A. (1α,2β,3β,4α)-5-[[3-(p-Toluenesulfonyloxymethyl)-7-oxabicyclo[2.2.1]hept-2-yl]methyl]oxy]pentanoic acid, methyl ester

To a solution of 544 mg (2.0 mmol) of (1α,2β,3β,4α)-5-[[3-(hydroxymethyl)-7-oxabicyclo[2.2.1]hept-2-yl]-methyl]oxy]pentanoic acid, methyl ester, prepared as described in Example 56 Part O, in 4 ml of dry pyridine is added 420 mg (2.2 mmol) of tosyl chloride. The mixture is stirred at room temperature under an argon atmosphere for 10 hours. The reaction mixture is diluted with 300 ml of ether, and washed with 1N aqueous HCl solution (3 x 100 ml). The ether layer is dried over anhydrous magnesium sulfate and concentrated in vacuo. Purification is effected by flash chromatography on 30 g of silica gel 60 using 50% hexane in ether as eluant to give 615 mg of title compound.

B. (1α,2β,3β,4α)-5-[[[3-[(Hexylthio)methyl]-7-oxabicyclo[2.2.1]hept-2-yl]methyl]oxy]pentanoic acid, methyl ester

To a solution of 132 mg (1.17 mmol) of potassium t-butoxide in 10 ml of dry THF under argon is added 378 mg (3.21 mmol) of 1-hexanethiol. To this mixture is added a solution of 425 mg (1.0 mmol) of Part A tosylate in 5 ml of THF. The reaction mixture is stirred at room temperature under argon for 2.5 hours and then heated to reflux for 5.5 hours. The cooled reaction is diluted with 300 ml of ether and poured into 100 ml of saturated $NaHCO_3$ solution. The aqueous layer is extracted with ether (2 x 100 ml). The combined ether extracts (500 ml) are washed with 0.5N aqueous sodium hydroxide (2 x 100 ml), brine (100 ml), and then dried ($MgSO_4$), filtered and concentrated in vacuo to give 675 g of crude oil. Purification is effected by chromatography on 25.2 g of silica gel 60 using 5:1 pet ether:ether as eluant to give 300 mg of title product as an oil.

C. (1α,2β,3β,4α)-5-[[[3-[(Hexylthio)methyl]-7-oxabicyclo[2.2.1]hept-2-yl]methyl]oxy]pentanoic acid

Following the procedure of Example 56 Part Q except substituting the above Part B ester for the Example 56 Part P ester, the title compound is obtained.

D. (1α,2β,3β,4α)-5-[[[3-[(Hexylthio)methyl]-7-oxabicyclo[2.2.1]hept-2-yl]methyl]oxy]-N-hydroxy-N-methylpentanamide

Following the procedure of Example 56 Part R except substituting the above Part C acid for the Example 56 Part Q acid, the title compound is obtained.

Example 60

(1α,2β,3β,4α)-5-[[[3-[(Hexylthio)methyl]-7-oxabicyclo[2.2.1]hept-2-yl]methyl]thio]-N-hydroxy-N-methylpentanamide

A. (1α,2β,3β,4α)-5-[[3-(p-Toluenesulfonyloxymethyl)-7-oxabicyclo[2.2.1]hept-2-yl]methyl]thio]pentanoic acid, methyl ester

To a solution of 576 mg (2.0 mmol) of (1α,2β,3β,4α)-5-[[[3-hydroxymethyl-7-oxabicyclo[2.2.1]hept-2-yl]-methyl]thio]pentanoic acid, methyl and ethyl esters prepared as described in Example 56A Part C in 4 ml of dry pyridine is added 420 mg (2.2 mmol) of tosyl chloride. The mixture is stirred at room temperature under an argon atmosphere for 10 hours. The reaction mixture is diluted with 300 ml of ether, washed with 1N

aqueous HCl solution (3 x 100 ml), and 0.5N aqueous NaOH solution (3 x 100 ml). The ether layer is dried over anhydrous magnesium sulfate and concentrated in vacuo. Purification is effected by flash chromatography on 30 g of silica gel 60 using 50% hexane in ether as eluant to give 860 mg of title compound.

B. (1α,2β,3β,4α)-5-[[[3-[(Hexylthio)methyl]-7-oxabicyclo[2.2.1]hept-2-yl]methyl]thio]pentanoic acid, hexyl ester

To a solution of 132 mg (1.17 mmol) of potassium t-butoxide in 10 ml of dry THF under argon is added 378 mg (3.21 mmol) of 1-hexanethiol. To this mixture is added a solution of 442 mg (1.0 mmol) of Part A tosylate in 5 ml of THF. The reaction mixture is stirred at room temperature under argon for 2.5 hours and then heated to reflux for 5.5 hours. The cooled reaction is diluted with 300 ml of ether and poured into 100 ml of saturated $NaHCO_3$ solution. The aqueous layer is extracted with ether (2 x 100 ml). The combined ether extracts (500 ml) are washed with 0.5N aqueous sodium hydroxide (2 x 100 ml), brine (100 ml), and then dried ($MgSO_4$), filtered and concentrated in vacuo to give 690 g of crude oil. Purification is effected by chromatography on 25.2 g of silica gel 60 using 5:1 pet ether:ether as eluant to give 310 mg of title product as an oil.

C. (1α,2β,3β,4α)-5-[[[3-[(Hexylthio)methyl]-7-oxabicyclo[2.2.1]hept-2-yl]methyl]thio]pentanoic acid

Following the procedure of Example 56 except substituting the above Part B ester for the Example 56 Part P ester, the title compound is obtained.

D. (1α,2β,3β,4α)-5-[[[3-(Hexylthio)methyl]-7-oxabicyclo[2.2.1]hept-2-yl]methyl]thio]-N-hydroxy-N-methylpentanamide

Following the procedure of Example 56 Part R except substituting the above Part C acid for the Example 56 Part Q acid, the title compound is obtained.

Example 61

(1α,2β,3β,4α)-5-[[[3-[(Hexyloxy)methyl]-7-oxabicyclo[2.2.1]hept-2-yl]ethyl]thio]-N-hydroxy-N-methyl-5-pentanamide

A. (1α,2β,3β,4α)-cis-exo-3-Isopropyloxycarbonyloxymethyl-2-hydroxymethyl-7-oxabicyclo[2.2.1]heptane

To a suspension of 11.4 g of lithium aluminum hydride (300 mmole) in 400 ml of dry THF at 0°C was added dropwise a solution of 32 g of (1α,2β,3β,4α)-cis-exo-[7-oxabicyclo[2.2.1]hept-2-y1α-2,3-dicarboxylic acid anhydride (mesoanhydride) (190 mmole) in 400 ml of dry THF over a period of 1 hour. The reaction mixture was stirred at 25°C for 18 hours, cooled to 0°C and quenched by slow addition of a saturated $Na_2SO_4$ solution, and filtered. The solid was washed with three 100 ml portions of $CH_2Cl_2$. The combined organic layer was dried over $MgSO_4$ and concentrated to give 32 g of (1α,2β,3β,4α)-cis-exo-7-oxabicyclo-[2.2.1]heptane-2,3-dimethanol (meso-diol) as a colorless solid.

To a solution of 10 g (63.2 mmole) of meso-diol in 40 ml dry THF at 0°C was added with stirring 55 ml of a 12.5% by weight solution of phosgene in toluene (63.2 mmole) dropwise over a period of 30 minutes. Argon was then bubbled through the reaction mixture for 15 minutes. The mixture was concentrated to give a crude oil of (1α,2β,3β,4α)-cis-exo-3-chlorocarbonyloxy-2-hydroxymethyl-7-oxabicyclo[2.2.1]heptane.

This oil was dissolved in 30 ml of dry $CH_2Cl_2$ and cooled to -50°C. To this solution was added dropwise a solution of 10 ml pyridine in 10 ml $CH_2Cl_2$. It was stirred for 10 minutes and quenched with $H_2O$. The mixture was extracted thoroughly with $CH_2Cl_2$. The organic extract was dried over $MgSO_4$ and concentrated to give (1α,2β,3β,4α)-7-oxabicyclo[2.2.1]heptane 2,3-dimethanol carbonate (cyclic carbonate) as a crystalline solid (10.7 g).

A mixture of 10.7 g of (1α,2β,3β,4α)-cis-exo-7-oxabicyclo[2.2.1]heptane 2,3-dimethanol carbonate (cyclic carbonate) (58.1 mmole) in 100 ml isopropanol was refluxed for 24 hours. Excess isopropanol was removed under reduced pressure to give 14.4 g title A compound (hydroxycarbonate) as a viscous oil.

B. (1α,2β,3β,4α)-cis-exo-3-Isopropyloxycarbonyloxymethyl-2-p-toluenesulfonyloxymethyl-7-oxabicyclo-[2.2.1]heptane

To a solution of 19.7 g of title A alcohol (80 mmole) in 30 ml $CH_2Cl_2$ and 12.8 ml pyridine (160 mmole, 2 eq.) was added 18.5 g p-toluenesulfonyl chloride (96 mmole). The mixture was stirred at 25°C for 36 hours then diluted with 200 ml ether, and washed with 100 ml brine.

The organic layer was dried over $MgSO_4$ and concentrated to give 32.8 g of title crude tosylate as an oil.

C. (1α,2β,3β,4α)-cis-exo-3-Isopropyloxycarbonyloxymethyl-2-cyanomethyl-7-oxabicyclo[2.2.1]heptane

To a solution of 24.0 g of title B crude tosylate (60 mmole) in 20 ml DMSO was added with stirring 6.0 g powdered sodium cyanide (120 mmole). The mixture was heated at 90°-95°C for 1.5 hours under an argon atmosphre. The cooled mixture was diluted with 50 ml water and extracted with five 100 ml portions of ether. The ethereal extracts were dried over anhydrous $MgSO_4$ and filtered though a bed of Florosil®. The filtrate was concentrated, and the residue was recrystallized with ether/hexanes to give 8.4 g of title cyanocarbonate as a light yellow crystalline solid.

D. (1α,2β,3β,4α)-cis-exo-3-Hydroxymethyl-2-cyanomethyl-7-oxabicyclo[2.2.1]heptane

To 8.4 g of title C cyanocarbonate (33.2 mmole) was added 75 ml of a 1% solution of potassium carbonate in methanol-water (2:1). The reaction mixture was stirred at 25°C for 6 hours, then acidified with 2N HCl solution, saturated with sodium chloride and extracted with six 100 ml portions of $CH_2Cl_2$. The combined organic layer was dried over anhydrous $MgSO_4$ and concentrated to give 5.5 g of crude title cyanoalcohol as a light yellow oil.

E. (1α,2β,3β,4α)-cis-exo-3-t-Butyldimethylsilyloxymethyl-2-cyanomethyl-7-oxabicyclo[2.2.1]heptane

To a solution of 5.0 g title D alcohol (30 mmole) in 50 ml of dry $CH_2Cl_2$ and 10 ml of triethylamine (70 mmole, 3.3 eq.) at 0°C was added with stirring 490 mg of 4-dimethylaminopyridine (4 mmole) and 5.29 g t-butyldimethylsilyl chloride (35 mmole). The reaction mixture was slowly warmed to 25°C and stirred for 18 hours, then diluted with 200 ml ether and filtered through a small bed of anhydrous $MgSO_4$. The filtrate was concentrated. Purification was done on a silica gel column, eluting with 15% ethyl acetate in hexanes to give 10.25 g of title silyl ether as a light yellow oil.

F. (1α,2β,3β,4α)-cis-exo-3-t-Butyldimethylsilyloxymethyl-2-formylmethyl-7-oxabicyclo[2.2.1]heptane

To a solution of 10.0 g of title E silyl ether (26.2 mmole) in 30 ml of dry toluene at -78°C under an argon atmosphere was added dropwise 25 ml of a 25% by weight solution of diisobutylaluminum hydride (44 mmole) in toluene. The mixture was stirred at -78°C for 4 hours, quenched at -78°C with a saturated solution of ammonium chloride, warmed to 0°C, acidified with 1N HCl solution, extracted with three 100 ml portions of $CH_2Cl_2$, dried over anhydrous $MgSO_4$ and concentrated to give 9.3 g of crude title aldehyde.

G. (1α,2β,3β,4α)-cis-exo-2-[3-t-Butyldimethylsilyloxymethyl-7-oxabicyclo[2.2.1]hept-2-yl]ethanol

To 9.3 g crude title F aldehyde (32.7 mmole) in 30 ml of dry THF at 0°C under an argon atmosphere was added portionwise 1.0 g lithium aluminum hydride (26.0 mmole) with stirring. The reaction mixture was stirred while being warmed to 25°C over a period of 1 hour, quenched by slow addition of a saturated sodium sulfate at 0°C, dried over anhydrous $MgSO_4$ and filtered. The solid was washed with $CH_2Cl_2$. The combined filtrate was concentrated to give a crude oil. This oil was purified on a silica gel column, eluting with 30% EtOAc in hexanes to give 8.55 g title alcohol as a colorless oil.

H. (1α,2β,3β,4α)-2-[2-[3-t-Butyldimethylsilyloxymethyl-7-oxabicyclo[2.2.1]hept-2-yl]ethyl]thioacetate

To a solution of 5.25 g triphenylphosphine (20 mmole) in 60 ml dry THF at 0°C was added dropwise 4.16 g diisopropylazodicarboxylate (20 mmole) over a period of 15 minutes. The mixture was stirred at 0°C for 30 minutes, and a solution of 2.6 g of title G alcohol (10 mmole) and 1.45 ml of thiolacetic acid (20 mmole) in 10 ml dry THF was added dropwise. The reaction mixture was stirred at 0°C for 1 hour, 25°C

for 3 hours, and was concentrated. The residue was triturated with ether/hexane, filtered, and the filtrate was concentrated and purified on a silica gel column, eluting with 10% EtOAc in hexane to give 2.3 g title thioacetate as a light yellow oil.

I. (1α,2β,3β,4α)-2-[2-[3-Hydroxymethyl-7-oxabicyclo[2.2.1]hept-2-yl]ethyl]thioacetate

To a solution of 2.3 g title H thioacetate (6.7 mmole) in 20 ml dry THF at 0°C was added 2.23 g of tetra-n-butylammoniumfluoride trihydrate (7.07 mmole) in 5 ml dry THF. The mixture was warmed at 25°C and stirred for 18 hours, diluted with 100 ml ether, washed with 30 ml of a saturated $NaHCO_3$ solution, dried over anhydrous $MgSO_4$ and concentrated to give a crude oil.

Purification was done on a silica gel column, eluting with 20% EtOAc in hexanes and 50% EtOAc in hexanes to give 1.22 g of title alcohol - thioacetate as a colorless oil.

J. (1α,2β,3β,4α)-2-[3-Hydroxymethyl-7-oxabicyclo[2.2.1]hept-2-yl]ethanethiol

To a slurry of 200 mg lithium aluminum hydride (5.27 mmole) in 20 ml dry THF at 0°C was added a solution of 1.22 g title I thioacetate (5.3 mmole) in 5 ml THF dropwise under an argon atmosphere. The reaction mixture was stirred at 0°C for 1 hour, quenched with a saturated sodium sulfate solution, dried with anhydrous $MgSO_4$, and was filtered. The filtrate was concentrated to give 900 mg of the title thiol as a colorless oil.

K. [1α,2β,3β,4α]-5-[[2-[3-Hydroxymethyl-7-oxabicyclo[2.2.1]hept-2-yl]ethyl]thio]pentanoic acid, ethyl ester

To a slurry of 1.38 g of dried and powdered sodium hydride (5.75 mmole) in 20 ml dry tetrahydrofuran at 0°C is added a solution of 900 mg title J thiol (4.8 mmole) in 5 ml THF followed by 1.75 ml of ethyl-5-bromoyalerate (11.05 mmole, 2.3 eq.). The reaction mixture is stirred at 0°C for 10 hours, diluted with 100 ml ether and filtered through a pad of anhydrous $MgSO_4$. The filtrate is then concentrated. The residue is purified on a silica gel column, eluting with 20% EtOAc in hexanes and 50% EtOAc in hexanes to give 1.22 g of title alcohol as a colorless oil.

L. (1α,2β,3β,4α)-5-[[2-[3-[(Hexyloxy)methyl]-7-oxabicyclo[2.2.1]hept-2-yl]ethyl]thio]pentanoic acid, hexyl ester

Following the procedure of Example 58 Part G except substituting the above Part K alcohol for the Example 58 Part F alcohol, the title compound is obtained.

M. (1α,2β,3β,4α)-5-[[2-[3-[(Hexyloxy)methyl]-7-oxabicyclo[2.2.1]hept-2-yl]ethyl]thio]pentanoic acid

Following the procedure of Example 56 Part Q except substituting the Part L hexyl ester for the Example 56 Part P ester, the title compound is obtained.

N. (1α,2β,3β,4α)-5-[[[(3-Hexyloxy)methyl]-7-oxabicyclo[2.2.1]hept-2-yl]ethyl]thio]N-hydroxy-N-methylpentanamide

Following the procedure of Example 56 Part R except substituting the above Part M acid for the Example 56 Part Q acid, the title compound is obtained.

Example 62

[1R-(1α,2β,3β,4α)]-4-[2-[3-[(Hexyloxy)methyl]-7-oxablcyclo[2.2.1]hept-2-yl]ethoxy]-N-hydroxy-N-methylbutanamide

A. [1R-(1α,2β,3β,4α)]-2-(Benzyloxy)methyl-3-cyanomethyl-7-oxabicyclo[2.2.1]heptane

To a slurry of 1.1 g of sodium hydride (21 mmole, 50% oil dispersion) in 25 ml of dry DMF at 0°C was added a solution of 3.34 g of Example 56 Part F cyanoalcohol (20 mmole) in 10 ml of DMF over a period of 10 minutes, After stirring for an additional 15 minutes, 3.6 g of benzyl bromide was added dropwise. The reaction mixture was stirred for 30 minutes at 0°C and 3 hours at 25°C then quenched with a saturated

ammonium chloride solution, and diluted with ether. The organic layer was washed with brine. The combined aqueous layer was re-extracted with ether. The combined organic layer was dried over anhydrous $MgSO_4$ and concentrated to leave an oil. The crude oil was chromatographed on a silica gel column, eluting with 10-20% ethyl acetate in hexanes to give 4.43 g of the title A benzyl ether.

B. [1R-(1α,2β,3β,4α)]-2-[[3-(Benzyloxy)methyl]-7-oxabicyclo[2.2.1]hept-2-yl]acetaldehyde

and

C. [1R-(1α,2β,3β,4α)]-2-[[3-(Benzyloxy)methyl]-7-oxabicyclo[2.2.1]hept-2-yl]ethanol

To a solution of 4.43 g of title A nitrile (17:24 mmole) in 20 ml of dry toluene at -78°C was added dropwise 20 ml of a 25% by weight solution of diisobutylaluminum hydride in toluene (35 mmole). After stirring at -78°C for 4 hours the reaction was quenched with a saturated ammonium chloride solution. The mixture was warmed to 25°C and 50 ml of a 1N aqueous hydrochloric acid solution was added. The organic layer was separated and the aqueous layer was extracted several times with ether. The combined organic extract was dried over anhydrous $MgSO_4$ and concentrated to give 4.55 g of crude title B aldehyde.

To the above crude title B aldehyde (17.24 mmole) in 30 ml of dry THF at 0°C was added 380 mg of lithium aluminum hydride (10 mmole) portionwise. After stirring while warming to 25°C over a period of 1 hour, the reaction was quenched with a saturated sodium sulfate solution. Solid anhydrous $MgSO_4$ was added and the mixture was filtered. The filtrate was concentrated to give 4.25 g of title C alcohol as a colorless oil.

D. [1R-(1α,2β,3β,4α)]-4-[2-[[3-(Benzyloxy)methyl]-7-oxabicyclo[2.2.1]hept-2-yl]ethoxy]butanol, t-butyldimethylsilyl ether

To a mixture of 4.5 g of powdered potassium hydroxide (82.6 mmole) in 20 ml of dry xylene was added a solution of 2.0 g of title C alcohol (8.26 mmole) in 10 ml of xylene. The mixture was heated to reflux and 15 ml of xylene was distilled off. To the remaining solution was added a solution of 4.0 g of 4-tert-butyldimethylsilyloxy n-butylmesylate in 10 ml of xylene. The resulting mixture was refluxed for 1 hour, cooled to 25°C and diluted with 300 ml of ether. The ethereal solution was washed with two 50 ml portions of water, dried over anhydrous $MgSO_4$ and concentrated. The residue was purified on a silica gel column, eluting with 20% ether in hexanes to give 1.4 g of title D compound as a yellow oil.

E. [1R-(1α,2β,3β,4α)]-4-[2-[[3-(Benzyloxy)methyl]-7-oxabicyclo[2.2.1]hept-2-yl]ethoxy]butanol

To 1.2 g of title D compound (2.68 mmole) in 5 ml of THF at 0°C was added 1.1 g of tetra-n-butylammonium fluoride (3.46 mmole). The mixture was stirred at 0°C for 1 hour and at 25°C for 1 hour and was diluted with 50 ml of ether. The ethereal solution was washed with two 10 ml portions of $H_2O$, 10 ml of brine, dried over anhydrous $MgSO_4$ and concentrated to give crude title E alcohol as an oil. This was used without purification.

F. [1R-(1α,2β,3β,4α)]-4-[[2-[3-(Benzyloxy)methyl]-7-oxabicyclo[2.2.1]hept-2-yl]ethoxy]butanoic acid

and

G. [1R-(1α,2β,3β,4α)]-4-[2-[[3-(Benzyloxy)methyl]-7-oxabicyclo[2.2.1]hept-2-yl]ethoxy]butanoic acid, methyl ester

To crude title E alcohol in 30 ml of acetone at 0°C was added dropwise a solution of 2.6 M Jones' reagent until the reaction mixture remained brown in color. The mixture was stirred for an additional 30 minutes at 0°C, quenched with isopropanol and diluted with 200 ml of ether. Anhydrous sodium acetate along with anhydrous magnesium sulfate was added. The mixture was stirred for 15 minutes at 25°C and filtered through a bed of Florosil®. The filtrates were concentrated. The residue was treated with 200 ml of a saturated $NaHCO_3$ solution and extracted with two 50 ml portions of ether. The aqueous layer was acidified with concentrated HCl, saturated with solid NaCl and extracted with five 100 ml portions of $CH_2Cl_2$, dried over anhydrous $MgSO_4$ and concentrated to give title F acid as an oil.

The above title F acid, dissolved in 30 ml of ether, was treated with an ethereal solution of diazomethane to give an oil which was purified on a silica gel column, eluting with 20% EtOAc in hexanes to yield 500 mg of pure title G ester.

H. [1R-(1α,2β,3β,4α)]-4-[[2-[3-(Hydroxymethyl)]-7-oxabicyclo[2.2.1]hept-2-yl]ethoxy]butanoic acid, methyl ester

A mixture of 500 mg of title G ester (1.38 mmole), 250 mg of 10% palladium over carbon in 10 ml of ethyl acetate and 1 ml of glacial acetic acid was shaken in a Parr bottle under 40 lbs. of hydrogen pressure at 25 ° C for 18 hours. The mixture was filtered through a bed of Celite and concentrated to give 242 mg of title H alcohol as an oil.

J. [1R-(1α,2β,3β,4α)]-4-[2[3-[(Hexyloxy)methyl]-7-oxaoicyclo[2.2.1]hept-2-yl]ethoxy]butanoic acid, hexyl ester

Following the procedure of Example 58 Part G except substituting the above Part H alcohol for the Example 58 Part F alcohol, the title compound is obtained.

K. [1R-(1α,2β,3β,4α)]-4-[2-[3-[(Hexyloxy)methyl]-7-oxabicyclo[2.2.1]hept-2-yl]ethoxy]butanoic acid

Following the procedure of Example 56 Part Q except substituting the Part J hexyl ester for the Example 56 Part P hexyl ester, the title compound is obtained.

L. [1R-(1α,2β,3β,4α)]-4-[2-[3-[(Hexyloxy)methyl]-7-oxabicyclo[2.2.1]hept-2-yl]ethoxy]-N-hydroxy-N-methylbutanamide

Following the procedure of Example 56 Part R except substituting the above Part K acid for the Example 56 Part Q acid, the title compound is obtained.

Example 63

(1α,2β,3β,4α)-5-[[2-[3-[(Hexylthio)methyl]-7-oxabicyclo[2.2.1]hept-2-yl]ethyl]thio]-N-hydroxy-N-methylpentanamide

Following the procedure of Example 60 except substituting (1α,2β,3β,4α)-5-[[2-[3-hydroxymethyl-7-oxabicyclo[2.2.1]hept-2-yl]ethyl]thio]pentanoic acid, methyl ester (prepared in Example 61, Part K) for the Example 56A Part C alcohol, the title compound is obtained.

Example 64

(1α,2β,3β,4α)-4-[[2-[3-[(Hexylthio)methyl]-7-oxabicyclo[2.2.1]hept-2-yl]ethoxy]-N-hydroxy-N-methylbutanamide

Following the procedure of Example 59 except substituting (1α,2β,3β,4α)-4-[2-[3-hydroxymethyl-7-oxabicyclo[2.2.1]hept-2-yl]ethoxy]butanoic acid, methyl ester (prepared in Example 62, Part I) for the Example 58 Part F alcohol, the title compound is obtained.

Example 65

(1α,2β,3β,4α)-5-[[3-[2-(Hexyloxy)ethyl]-7-oxabicyclo[2.2.1]hept-2-yl]methoxy]-N-hydroxy-N-methylpentanamide

A. (1α,2β,3β,4α)-5-[[(3-Formyl)-7-oxabicyclo[2.2.1]hept-2-yl]methoxy]pentanoic acid, methyl ester

To 5.44 g of (1α,2β,3β,4α)-5-[[3-(hydroxymethyl)-7-oxabicyclo[2.2.1]hept-2-yl]methoxy]pentanoic acid, methyl ester prepared as described in Example 58, Part F (20 mmol) in 65 ml of dry $CH_2Cl_2$ at 25 ° C is added 13.0 g Celite, 1.7 g NaOAc (6.15 mmole, 30 mole %) and 12.94 g pyridinium chlorochromate (60

mmole, 3 eq.). The mixture is stirred at 25 ° C for 2 hours, diluted with 100 ml ether and filtered through a bed of Florosil®. The filtrate is concentrated to give 5.25 g of title aldehyde as a clear oil which is used in the next reaction without further purification.

B. (1α,2β,3β,4α)-5-[[3-(2-Oxo)ethyl]-7-oxabicyclo[2.2.1]hept-2-yl]methoxy]pentanoic acid, methyl ester

Into a dry 100 ml round bottom 3-necked flask containing a stir bar is added dried methoxymethyl-triphenylphosphonium chloride ($(C_6H_5)_3P^+-CH_2OCH_3Cl^-$) (3.25 g, 9.54 mmole) and 30 ml of distilled toluene (stored over molecular sieves). The resulting suspension is stirred in an ice-bath, under argon until cold and a 1.4 M solution of 5.73 ml (8.01 mmol) of potassium t-amylate in toluene is added dropwise. A bright red solution is formed which is stirred at 0 ° C for an additional 35 minutes. Thereafter, a solution of 1.04 g (3.84 mmol) of (1α,2β,3β,4α)-5-[[(3-formyl)-7-oxabicyclo[2.2.1]hept-2-yl]methoxy]pentanoic acid, methyl ester in 10 ml toluene is added by means of a dropping funnel over a 35 minute period with the ice-bath still in place. The reaction is then quenched by addition of 2.3 g (39 mmol) of acetic acid in 5 ml ether. The reaction mixture is immediately poured into 200 ml of saturated $NH_4Cl$, and extracted with ether (4 x 200 ml). The combined ether phases are washed with a saturated NaCl solution, dried ($MgSO_4$) and concentrated to yield an oil in a white crystalline solid (phosphine oxide). The white solid is removed after trituration with EtOAc and the mother liquor is purified by chromatography on an LPS-1 silica column to obtain the enol-ether. The enol-ether is dissolved in 20 ml of THF and then treated with 10 ml of a 20% aqueous trifluoroacetic acid solution. After 1 hour at room temperature, sodium bicarbonate is carefully added. The mixture is then extracted several times with methylene chloride. The combined methylene chloride extract is dried over anhydrous magnesium sulfate and concentrated under reduced pressure. Chromatography on a LPS-1 silica gel column and elution with 15-30% ethylacetate in hexane gives 980 mg of title B aldehyde.

C. (1α,2β,3β,4α)-5-[[3-[2-(Hydroxy)ethyl]-7-oxabicyclo[2.2.1]hept-2-yl]methoxy]pentanoic acid ester

The aldehyde (980 g, 3.45 mmol) from part B in methanol (50 ml) is treated with $NaBH_4$ (0.19 g, 5 mmol) in an argon atmosphere at 0 ° C. After stirring at 0 ° C for 1 hour, the reaction is quenched by addition of 2N HCl (to pH 2). The methanol is removed in vacuo and the reaction mixture is taken up in ether. The ether solution is washed with saturated $KHCO_3$, saturated NaCl and dried ($MgSO_4$, anhydrous). The ether is evaporated to yield the title C compound.

D. (1α,2β,3β,4α)-5-[[3-[2-(Hexyloxy)ethyl]-7-oxabicyclo[2.2.1]hept-2-yl]methoxy]pentanoic acid

Following the procedure of Example 56, Part P except substituting the above part C alcohol for the Example 56 Part O alcohol used in Example 56 Part P, the title compound is obtained.

E. (1α,2β,3β,4α)-5-[[3-[2-(Hexyloxy)ethyl]-7-oxabicyclo[2.2.1]hept-2-yl]methoxy]-N-hydroxy-N-methylpentanamide

Following the procedure of Example 56 Part R except substituting the above Part D acid for the Example 56 Part Q acid, the title compound is obtained.

Example 66

(1α,2β,3β,4α)-5-[[[3-[2-(Hexyloxy)ethyl]-7-oxabicyclo[2.2.1]hept-2-yl]methyl]thio]-N-hydroxy-N-methylpentanamide

A. (1α,2β,3β,4α)-5-[[[3-[2-(Hexyloxy)ethyl]-7-oxabicyclo[2.2.1]hept-2-yl]methyl]thio]pentanoic acid

Following the procedure of Example 56 Part P except substituting (1α,2β,3β,4α)-5-[[3-(2-hydroxy)ethyl]-7-oxaoicyclo[2.2.1]hept-2-yl]methyl]thio]pentanoic acid, methyl ester (prepared as described in Example 68 Parts A to C) for the alcohol used in Example 56 Part P, the title compound is obtained.

B. (1α,2β,3β,4α)-5-[[[3-(2-Hexyloxy)ethyl]-7-oxabicyclo[2.2.1]hept-2-yl]methyl]thio]-N-hydroxy-N-methylpentanamide

Following the procedure of Example 56 Part R except substituting the above Part A acid for the Example 56 Part Q acid, the title compound is obtained.

Example 67

(1α,2β,3β,4α)-5-[[3-[(2-Hexylthio)ethyl]-7-oxabicyclo[2.2.1]hept-2-yl]methoxy]-N-hydroxy-N-methylpentanamide

Following the procedure of Example 59 except substituting (1α,2β,3β,4α)-5-[[3-(2-hydroxyethyl)-7-oxabicyclo[2.2.1]hept-2-yl]methoxy]pentanoic acid, methyl ester (prepared as described in Example 65 Part C) for the Example 56 Part O alcohol, the title compound is obtained.

Example 68

(1α,2β,3β,4α)-5-[[[3-[2-(Hexylthio)ethyl]-7-oxabicyclo[2.2.1]hept-2-yl]methyl]thio]-N-hydroxy-N-methylpentanamide

A. (1α,2β,3β,4α)-5-[[[(3-Formyl)-7-oxabicyclo[2.2.1]hept-2-yl]methyl]thio]pentanoic acid, methyl ester

To a solution of 4 ml of oxalyl chloride (35 mmole) in 10 ml of dry methylene chloride at -60 °C is added dropwise 6.5 ml of dry dimethylsulfoxide (90 mmole) over a period of 15 minutes. After stirring for an additional 30 minutes, a solution of 2.51 g of ((1α,2β,3β,4α)-5-[[[3-hydroxymethyl-7-oxabicyclo[2.2.1]hept-2-yl]methyl]thio pentanoic acid, methyl ester (prepared as described for Examples 61 Part K or 66 Part A) (8.7 mmole) in 10 ml of dry methylene chloride is added dropwise over a period of 15 minutes. The mixture is stirred at -60 °C for 30 minutes and then 10 ml of distilled triethylamine (~70 mmole) is added. The reaction is then warmed to room temperature and water is added. It is then stirred at room temperature for additional 30 minutes, extracted with methylene chloride and washed with a saturated bicarbonate solution. The organic layer is dried over anhydrous magnesium sulfate and concentrated under reduced pressure.

Purification of the crude residue on a LPS-1 silica gel column and elution with 10-30% ethyl acetate in hexane gives 1.72 g of title A aldehyde.

B. (1α,2β,3β,4α)-5-[[[[3-(2-Oxo)ethyl]-7-oxabicyclo[2.2.1]hept-2-yl]methyl]thio]pentanoic acid, methyl ester

Into a dry 100 ml round bottom 3-necked flask containing a stir bar is added dried 3.27 g (9.54 mmoles) methoxymethyltriphenylphosphonium chloride ($(C_6H_5)_3P^+-CH_2OCH_3Cl^-$) and 30 ml distilled toluene (stored over molecular sieves) under argon. The resulting suspension is stirred in an ice-bath until cold and then a 1.4 M solution of 5.73 ml (8.01 mmol) of potassium t-amylate in toluene is added dropwise. A bright red solution forms which is stirred at 0 °C for an additional 35 minutes. Thereafter, a solution of 1.08 g (3.84 mmol) of (1α,2β,3β,4α)-5-[[[(3-formyl)-7-oxabicyclo[2.2.1]hept-2-yl]methyl]thio]pentanoic acid, methyl ester in 10 ml toluene is added by means of a dropping funnel over a 35 minute period with the ice-bath still in place. The reaction is then quenched by addition of 2.3 g (39 mmol) acetic acid in 5 ml ether. The mixture is immediately poured into 200 ml saturated $NH_4Cl$, and extracted with ether (4 x 200 ml). The combined ether phases are washed with NaCl saturated solution, dried ($MgSO_4$ anhydrous) and concentrated to yield an oil in a white crystalline solid (phosphine oxide). The white solid is triturated with EtOAc, removed by filtration, and the mother liquor is purified by chromatography on an LPS-1 silica column to obtain the enol-ether. The enol ether is dissolved in 20 ml of THF and is then treated with 10 ml of a 20% aqueous trifluoro acetic acid solution. After 1 hour at room temperature, the trifluoroacetic acid is neutralized by addition of solid sodium bicarbonate. The mixture is then extracted with methylene chloride. The methylene chloride extract is dried over anhydrous magnesium sulfate and concentrated under reduced pressure. Chromatography of the crude residue on a LPS-1 silica gel column and elution with 15-30% ethyl acetate in hexane gives 1.02 g of title B aldehyde.

C. (1α,2β,3β,4α)-5-[[[3-(2-Hydroxyethyl)-7-oxabicyclo[2.2.1]hept-2-yl]methyl]thio]pentanoic acid methyl ester

The aldehyde (1.02 g, 3.45 mmol) from part B in methanol (50 ml) is treated with $NaBH_4$ (0.19 g, 5 mmol) in an argon atmosphere at 0° C. After stirring at 0° C for 1 hour, the reaction is quenched by addition of 2N HCl (to pH 2). The methanol is removed in vacuo and the mixture is taken up in ether. The ether solution is washed with a saturated $KNCO_3$ solution, saturated NaCl solution and dried ($MgSO_4$ anhydrous). The ether is evaporated to yield the title C compound.

D. (1α,2β,3β,4α)-5-[[[3-[2-(Hexyloxy)ethyl]-7-oxabicyclo[2.2.1]hept-2-yl]methyl]thio]pentanoic acid

Following the procedure of Example 56A, Part D except substituting the above part C alcohol for the Example 56A Part C alcohol, the title compound is obtained.

E. (1α,2β,3β,4α)-5-[[[3-[2-(Hexylthio)methyl]-7-oxabicyclo[2.2.1]hept-2-yl]methyl]thio]-N-hydroxy-N-methylpentanamide

Following the procedure of Example 56 Part R except substituting the above Part D acid for Example 56 Part Q acid, the title compound is obtained.

Example 69

(1α,2β,3β,4α)-4-[2-[3-[2-(Hexyloxy)ethyl]-7-oxabicyclo[2.2.1]hept-2-yl]ethoxy]-N-hydroxy-N-methylbutanamide

A. (1α,2β,3β,4α)-4-[2-[3-Formyl-7-oxabicyclo[2.2.1]hept-2-yl]ethoxy]butanoic acid, methyl ester

Following the procedure of Example 65 Part A except substituting (1α,2β,3β,4α)-4-[2-[3-hydroxymethyl-7-oxabicyclo[2.2.1]hept-2-yl]ethoxy]butanoic acid, methyl ester (prepared as described in Example 62 Part H) for the Example 58 Part F alcohol, the title alcohol is obtained.

B. (1α,2β,3β,4α)-4-[[2-[3-(2-Oxo)ethyl]-7-oxabicyclo[2.2.1]hept-2-yl]ethoxy]butanoic acid, methyl ester

Following the procedure of Example 65 Part B except substituting the above Part A aldehyde for the Example 65 Part A aldehyde, the title compound is obtained.

C. (1α,2β,3β,4α)-4-[2-[[3-(2-Hydroxy)ethyl]-7-oxabicyclo[2.2.1]hept-2-yl]ethoxy]butanoic acid, methyl ester

Following the procedure of Example 65 Part C except substituting the above Part B aldehyde for the Example 65 Part B aldehyde, the title compound is obtained.

D. (1α,2β,3β,4α)-4-[2-[3-[2-(Hexyloxy)ethyl]-7-oxabicyclo[2.2.1]hept-2-yl]ethoxy]butanoic acid

Following the procedure of Example 58 Parts G and H except substituting the above Part C alcohol for the Example 58 Part F alcohol used in Example 56 Part G, the title compound is obtained.

E. (1α,2β,3β,4α)-4-[2-[3-[2-(Hexyloxy)methyl]-7-oxabicyclo[2.2.1]hept-2-yl]ethoxy]-N-hydroxy-N-methylbutanamide

Following the procedure of Example 56 Part R except substituting the above Part D acid for the Example 56 Part Q acid, the title compound is obtained.

Example 70

(1α,2β,3β,4α)-5-[[2-[3-[2-(Hexyloxy)ethyl]-7-oxabicyclo[2.2.1]hept-2-yl]ethyl]thio]-N-hydroxy-N-methylpentanamide

A. (1α,2β,3β,4α)-5-[[[2-(3-Formyl)-7-oxabicyclo[2.2.1]hept-2-yl]ethyl]thio]pentanoic acid, methyl ester

Following the procedure of Example 65 Part A except substituting (1α,2β,3β,4α)-4-[[2-[(hydroxy)methyl-7-oxabicyclo[2.2.1]hept-2-yl]ethyl]thio] pentanoic acid, methy and ethyl esters (prepared as described in Example 61 Part K) for the Example 58 Part F alcohol, the title compound is obtained.

B. (1α,2β,3β,4α)-5-[[2-[3-(2-Oxo)ethyl]-7-oxabicyclo[2.2.1]hept-2-yl]ethyl]thio]pentanoic acid, methyl and ethyl esters

Following the procedure of Example 65 Part B except substituting the above Part A aldehyde for the Example 65 Part A aldehyde, the title compound is obtained.

C. (1α,2β,3β,4α)-5-[[2-[3-[(2-(Hydroxy)ethyl]-7-oxabicyclo[2.2.1]hept-2-yl]ethyl]thio]pentanoic acid, methyl and ethyl esters

Following the procedure of Example 65 Part C except substituting the above Part B aldehyde for the Example 65 Part B aldehyde, the title compound is obtained.

D. (1α,2β,3β,4α)-5-[[2-[3-[2-(Hexyloxy)ethyl]-7-oxabicyclo[2.2.1]hept-2-yl]ethyl]thio]pentanoic acid

Following the procedure of Example 56A Parts D and E except substituting the above Part C alcohol for the Example 56A Part C alcohol, the title compound is obtained.

E. (1α,2β,3β,4α)-5-[[2-[3-[2-(Hexyloxy)ethyl]-7-oxabicyclo[2.2.1]hept-2-yl]ethyl]thio]-N-hydroxy-N-methylpentanamide

Following the procedure of Example 56 Part R except substituting the above Part D acid for the Example 56 Part Q acid, the title compound is obtained.

Example 71

(1α,2β,3β,4α)-4-[2-[3-[2-(Hexylthio)ethyl]-7-oxabicyclo[2.2.1]hept-2-yl]ethoxy]-N-hydroxy-N-methylbutanamide

A. (1α,2β,3β,4α)-4-[2-[3-[(2-Hydroxy)ethyl]-7-oxabicyclo[2.2.1]hept-2-yl]ethoxy]butanoic acid, methyl ester

Following the procedure of Example 65 Parts A, B and C except substituting (1α,2β,3β,4α)-4-[2-[3-hydroxymethyl)-7-oxabicyclo[2.2.1]hept-2-yl]ethoxy]butanoic acid, methyl ester (prepared as described in Example 62 Part H) for the Example 58 Part F alcohol, the title compound is obtained.

B. (1α,2β,3β,4α)-4-[2-[3-[2-(Hexylthio)ethyl]-7-oxabicyclo[2.2.1]hept-2-yl]ethoxy]butanoic acid

Following the procedure of Example 67 except substituting (1α,2β,3β,4α)-4-[2-[3-[2-(hydroxy)ethyl]-7-oxabicyclo[2.2.1]hept-2-yl]ethoxy]butanoic acid, methyl ester for the Example 65 Part C alcohol, the title compound is obtained.

C. (1α,2β,3β,4α)-4-[2-[3-[2-(Hexylthio)ethyl]-7-oxabicyclo[2.2.1]hept-2-yl]ethoxy]-N-hydroxy-N-methylbutanamide

Following the procedure of Example 56 Part R except substituting the above Part B acid for the Example 56 Part Q acid, the title compound is obtained.

Example 72

(1α,2β,3β,4α)-5-[[2-[3-[2-(Hexylthio)ethyl]-7-oxabicyclo[2.2.1]hept-2-yl]ethyl]thio]-N-hydroxy-N-methylpentanamide

A. (1α,2β,3β,4α)-5-[[2-[3-[2-(Hydroxy)ethyl]-7-oxabicyclo[2.2.1]hept-2-yl]ethyl]thio]pentanoic acid, methyl ester

Following the procedure of Example 66 Part A except substituting (1α,2β,3β,4α)-5-[2-[3-(hydroxymethyl)-7-oxabicyclo[2.2.1]hept-2-yl]ethyl]thio]pentanoic acid, methyl and ethyl esters (prepared as described in Example.61 Part K) for the Example 56A Part C alcohol, the title compound is obtained.

B. (1α,2β,3β,4α)-5-[[2-[3-[2-(Hexylthio)ethyl]-7-oxabicyclo[2.2.1]hept-2-yl]ethyl]thio]pentanoic acid

Following the procedure of Example 68 except substituting the above title A alcohol for the Example 66 Part A alcohol, the title compound is obtained.

C. (1α,2β,3β,4α)-5-[[2-[3-[2-(Hexylthio)ethyl]-7-oxabicyclo[2.2.1]hept-2-yl]ethyl]thio]-N-hydroxy-N-methylpentanamide

Following the procedure of Example 56 Part R except substituting the above Part B acid for the Example 56 Part Q acid, the title compound is obtained.

Example 73

(1α,2β,3β,4α)-5-[[[3-(Methoxymethyl)-7-oxabicyclo[2.2.1]hept-2-yl]methoxy]-N-hydroxy-N-methylpentanamide

Following the procedure of Example 58 except substituting methyl mesylate for hexyl mesylate, the title compound is obtained.

Example 74

(1α,2β,3β,4α)-5-[[3-[(2-Propenyloxy)methyl]-7-oxabicyclo[2.2.1]hept-2-yl]methoxy]-N-hydroxy-N-methylpentanamide

Following the procedure of Example 58 except substituting 2-propenyl mesylate for hexyl mesylate, the title compound is obtained.

Example 75

(1α,2β,3β,4α)-5-[[3-(2-Butenyloxy)methyl]-7-oxabicyclo[2.2.1]hept-2-yl]methoxy]-N-hydroxy-N-methylpentanamide

Following the procedure of Example 58 except substituting 2-butenyl mesylate for hexyl mesylate, the title compound is obtained.

Example 76

(1α,2β,3β,4α)-5-[[3-[(Benzyloxy)methyl]-7-oxabicyclo[2.2.1]hept-2-yl]methoxy]-N-hydroxy-N-methylpentanamide

Following the procedure of Example 58 except substituting benzyl mesylate for hexyl mesylate, the title compound is obtained.

Example 77

(1α,2β,3β,4α)-5-[[3-[(Phenyloxy)methyl]-7-oxabicyclo[2.2.1]hept-2-yl]methoxy]-N-hydroxy-N-methylpentanamide

(a) Phenol (1 mmol) is added to a solution of triphenylphosphine (1 mmol), diethylazodicarboxylate (1 mmol) and title F alcohol from Example 58 (1 mmol) in 25 ml THF and is stirred under an argon atmosphere for 48 hours at 23 °C. The reaction mixture is concentrated in vacuo. The residue is triturated with ether and the solids are removed. The filtrate is concentrated in vacuo and chromatographed on silica gel to give (1α,2β,3β,4α)-5-[[3-[(phenyloxy)methyl]-7-oxabicyclo[2.2.1]hept-2-yl]-methoxy]pentanoic acid, methyl ester.

(b) Following the procedure as set out in Example 58, the ester from part (a) is converted to the title compound.

Example 78

(1α,2β,3β,4α)-5-[[3-[(Cyclohexyloxy)methyl]-7-oxabicyclo[2.2.1]hept-2-yl]methoxy]-N-hydroxy-N-methylpentanamide

Following the procedure of Example 58 except substituting cyclohexylmesylate for hexyl mesylate, the title compound is obtained.

Example 79

(1α,2β,3β,4α)-5-[[3-[(Cyclopentylmethoxy)methyl]-7-oxabicyclo[2.2.1]hept-2-yl]methoxy]-N-hydroxy-N-methylpentanamide

Following the procedure of Example 58 except substituting cyclopentylmethyl mesylate for hexyl mesylate, the title compound is obtained.

Example 80

(1α,2β,3β,4α)-5-[[[3-[(Benzyloxy)methyl]-7-oxabicyclo[2.2.1]hept-2-yl]methyl]thio]-N-hydroxy-N-methylpentanamide

Following the procedure of Example 56A except substituting benzyl mesylate for hexyl mesylate, the title compound is obtained.

Example 81

(1α,2β,3β,4α)-5-[[[3-[(2-Butenyloxy)methyl]-7-oxabicyclo[2.2.1]hept-2-yl]methyl]thio]-N-hydroxy-N-methylpentanamide

Following the procedure of Example 56A except substituting 2-butenyl mesylate for hexyl mesylate, the title compound is obtained.

Example 82

(1α,2β,3β,4α)-5-[[[3-[(Cyclohexyloxy)methyl]-7-oxabicyclo[2.2.1]hept-2-yl]methyl]thio]-N-hydroxy-N-methylpentanamide

Following the procedure of Example 56A except substituting cyclohexyl mesylate for hexyl mesylate, the title compound is obtained.

Example 83

(1α,2β,3β,4α)-5-[[[3-[(Cyclopentylmethyloxy)methyl]-7-oxabicyclo[2.2.1]hept-2-yl]methyl]thio]-N-hydroxy-N-methylpentanamide

Following the procedure of Example 56A except substituting cyclopentylmethyl mesylate for hexyl mesylate, the title compound is obtained.

Example 84

(1α,2β,3β,4α)-5-[[[3-[(Phenyloxy)methyl]-7-oxabicyclo[2.2.1]hept-2-yl]methyl]thio]-N-hydroxy-N-methylpentanamide

Following the procedure of Examples 77 and 56A except substituting the Example 56A Part C alcohol for the Example 77 Part A alcohol, the title compound is obtained.

Example 85

(1α,2β,3β,4α)-5-[[3-[(2-Pentylthio)methyl]-7-oxabicyclo[2.2.1]hept-2-yl]methoxy]-N-hydroxy-N-methylpentanamide

Following the procedure of Example 59 except substituting 2-pentanethiol for 1-hexanethiol, the title compound is obtained.

Example 86

(1α,2β,3β,4α)-5-[[3-[(Benzylthio)methyl]-7-oxabicyclo[2.2.1]hept-2-yl]methoxy]-N-hydroxy-N-methylpentanamide

Following the procedure of Example 59 except substituting benzylmercaptan for 1-hexanethiol, the title compound is obtained.

Example 87

(1α,2β,3β,4α)-5-[[3-[(Cyclohexylthio)methyl]-7-oxabicyclo[2.2.1]hept-2-yl]methoxy]-N-hydroxy-N-methylpentanamide

Following the procedure of Example 59 except substituting cyclohexanethiol for 1-hexanethiol, the title compound is obtained.

Example 88

(1α,2β,3β,4α)-5-[[3-[(Phenylthio)methyl]-7-oxabicyclo[2.2.1]hept-2-yl]methoxy]-N-hydroxy-N-methylpentanamide

Following the procedure of Example 59 except substituting phenylmercaptan for 1-hexanethiol, the title compound is obtained.

Example 89

(1α,2β,3β,4α)-5-[[[3-[(Cyclopentylthio)methyl]-7-oxabicyclo[2.2.1]hept-2-yl]methyl]thio]-N-hydroxy-N-methylpentanamide

Following the procedure of Example 60 except substituting cyclopentylmercaptan for 1-hexanethiol, the title compound is obtained.

Example 90

(1α,2β,3β,4α)-5-[[[3-[(Cyclohexylmethylthio)methyl]-7-oxabicyclo[2.2.1]hept-2-yl]methyl] thio]-N-hydroxy-N-methylpentanamide

Following the procedure of Example 60 except substituting cyclohexylmethylmercaptan for 1-hexanethiol, the title compound is obtained.

Example 91

(1α,2β,3β,4α)-5-[[[3-[(Phenylthio)methyl]-7-oxabicyclo[2.2.1]hept-2-yl]methyl]thio]-N-hydroxy-N-methylpentanamide

Following the procedure of Example 60 except substituting phenylmercaptan for 1-hexanethiol, the title compound is obtained.

Example 92

(1α,2β,3β,4α)-5-[[[3-[(Benzylthio)methyl]-7-oxabicyclo[2.2.1]hept-2-yl]methyl]thio]-N-hydroxy-N-methylpentanamide

Following the procedure of Example 60 except substituting benzylmercaptan for 1-hexanethiol, the title compound is obtained.

Example 93

(1α,2β,3β,4α)-5-[[[3-[(3-Pentenylthio)methyl]-7-oxabicyclo[2.2.1]hept-2-yl]methyl]thio]-N-hydroxy-N-methylpentanamide

Following the procedure of Example 60 except substituting 1-(3-pentenyl)mercaptan for 1-hexanethiol, the title compound is obtained.

Example 94

(1α,2β,3β,4α)-5-[[[3-[(Cyclohexyloxy)methyl]-7-oxabicyclo[2.2.1]hept-2-yl]ethyl]thio]-N-hydroxy-N-methylpentanamide

Following the procedure of Example 61 except substituting cyclohexyl mesylate for hexyl mesylate, the title compound is obtained.

Example 95

(1α,2β,3β,4α)-5-[[2-[3-[[(Cyclopentylmethyl)oxy]methyl]-7-oxabicyclo[2.2.1]hept-2-yl]ethyl]thio]-N-hydroxy-N-methylpentanamide

Following the procedure of Example 61 except substituting cyclopentylmethyl mesylate for hexyl mesylate, the title compound is obtained.

Example 96

(1α,2β,3β,4α)-5-[[2-[3-[(2-Butenyloxy)methyl]-7-oxabicyclo[2.2.1]hept-2-yl]ethyl]thio]-N-hydroxy-N-methylpentanamide

Following the procedure of Example 61 except substituting 2-butenyl mesylate for hexyl mesylate, the title compound is obtained.

Example 97

(1α,2β,3β,4α)-5-[[2-[3-[(Phenyloxy)methyl]-7-oxabicyclo[2.2.1]hept-2-yl]ethyl]thio]-N-hydroxy-N-methylpentanamide

Following the procedure of Examples 77 and 56A except substituting the Example 61 Part K alcohol for the Example 58 Part F alcohol, the title compound is obtained.

Example 98

(1α,2β,3β,4α)-4-[2-[3-[(Cyclohexyloxy)methyl]-7-oxabicyclo[2.2.1]hept-2-yl]ethoxy]-N-hydroxy-N-methylbutanamide

Following the procedure of Example 62 except substituting cyclohexyl mesylate for hexyl mesylate, the title compound is obtained.

Example 99

(1α,2β,3β,4α)-4-[2-[3-[(2-Pentenyloxy)methyl]-7-oxabicyclo[2.2.1]hept-2-yl]ethoxy]-N-hydroxy-N-methylbutanamide

Following the procedure of Example 62 except substituting 2-pentenyl mesylate for hexyl mesylate, the title compound is obtained.

Example 100

(1α,2β,3β,4α)-4-[2-[3-[(Benzyloxy)methyl]-7-oxabicyclo[2.2.1]hept-2-yl]ethoxy]-N-hydroxy-N-methylbutanamide

Following the procedure of Example 62 except substituting benzyl mesylate for hexyl mesylate, the title compound is obtained.

Example 101

(1α,2β,3β,4α)-4-[2-[3-[(Phenyloxy)methyl]-7-oxabicyclo[2.2.1]hept-2-yl]ethoxy]-N-hydroxy-N-methylbutanamide

Following the procedure of Examples 77 and 62 except substituting the Example 62 Part H alcohol for the Example 58 Part F alcohol, the title compound is obtained.

Example 102

(1α,2β,3β,4α)-4-[2-[3-[(Cyclopentylmethyloxy)methyl]-7-oxabicyclo[2.2.1]hept-2-yl]ethoxy]-N-hydroxy-N-methylbutanamide

Following the procedure of Example 62 except substituting cyclopentylmethyl mesylate for hexyl mesylate, the title compound is obtained.

Example 103

(1α,2β,3β,4α)-5-[[2-[3-[(Benzylthio)methyl]-7-oxabicyclo[2.2.1]hept-2-yl]ethyl]thio]-N-hydroxy-N-methylpentanamide

Following the procedure of Example 63 except substituting benzylmercaptan for 1-hexanethiol, the title compound is obtained.

Example 104

(1α,2β,3β,4α)-5-[[2-[3-[(Phenylthio)methyl]-7-oxabicyclo[2.2.1]hept-2-yl]ethyl]thio]-N-hydroxy-N-methylpentanamide

Following the procedure of Example 63 except substituting phenylmercaptan for 1-hexanethiol, the title compound is obtained.

Example 105

(1α,2β,3β,4α)-5-[[2-[3-[(Cycloheptylthio)methyl]-7-oxabicyclo[2.2.1]hept-2-yl]ethyl]thio]-N-hydroxy-N-methylpentanamide

Following the procedure of Example 63 except substituting cycloheptylmercaptan for 1-hexanethiol, the title compound is obtained.

Example 106

(1α,2β,3β,4α)-5-[[2-[3-[(Cyclohexylmethylthio)methyl]-7-oxabicyclo[2.2.1]hept-2-yl]ethyl]thio]-N-hydroxy-N-methylpentanamide

Following the procedure of Example 63 except substituting cyclohexylmethylmercaptan for 1-hexanethiol, the title compound is obtained.

Example 107

(1α,2β,3β,4α)-5-[[2-[3-[(2-Propenylthio)methyl]-7-oxabicyclo[2.2.1]hept-2-yl]ethyl]thio]-N-hydroxy-N-methylpentanamide

Following the procedure of Example 63 except substituting 2-propenylthiol for 1-hexanethiol, the title compound is obtained.

Example 108

(1α,2β,3β,4α)-4-[2-[3-[(Benzylthio)methyl]-7-oxabicyclo[2.2.1]hept-2-yl]ethoxy]-N-hydroxy-N-methylbutanamide

Following the procedure of Example 64 except substituting benzylmercaptan for 1-hexanethiol, the title compound is obtained.

Example 109

(1α,2β,3β,4α)-4-[2-[3-[(Phenylthio)methyl]-7-oxabicyclo[2.2.1]hept-2-yl]ethoxy]-N-hydroxy-N-methylbutanamide

Following the procedure of Example 64 except substituting phenylmercaptan for 1-hexanethiol, the title compound is obtained.

Example 110

(1α,2β,3β,4α)-4-[2-[3-[(3-Pentenylthio)methyl]-7-oxabicyclo[2.2.1]hept-2-yl]ethoxy]-N-hydroxy-N-methylbutanamide

Following the procedure of Example 64 except substituting 3-pentenylthiol for 1-hexanethiol, the title compound is obtained.

Example 111

(1α,2β,3β,4α)-4-[2-[3-[(Cyclohexylthio)methyl]-7-oxabicyclo[2.2.1]hept-2-yl]ethoxy]-N-hydroxy-N-methylbutanamide

Following the procedure of Example 64 except substituting cyclohexylmercaptan for 1-hexanethiol, the title compound is obtained.

Example 112

(1α,2β,3β,4α)-5-[[3-[2-(Benzyloxy)ethyl]-7-oxabicyclo[2.2.1]hept-2-yl]methoxy]-N-hydroxy-N-methylpentanamide

Following the procedure of Example 65 except substituting benzyl mesylate for hexyl mesylate, the title compound is obtained.

Example 113

(1α,2β,3β,4α)-5-[[3-[2-(Phenyloxy)ethyl]-7-oxabicyclo[2.2.1]hept-2-yl]methoxy]-N-hydroxy-N-methylpentanamide

Following the procedure of Example 65 except substituting phenyl mesylate for hexyl mesylate, the title compound is obtained.

Example 114

(1α,2β,3β,4α)-5-[[3-[2-(3-Butenyloxy)ethyl]-7-oxabicyclo[2.2.1]hept-2-yl]methoxy]-N-hydroxy-N-methylpentanamide

Following the procedure of Example 65 except substituting 1-butenyl mesylate for hexyl mesylate, the title compound is obtained.

Example 115

(1α,2β,3β,4α)-5-[[3-[2-(Cyclohexyloxy)ethyl]-7-oxabicyclo[2.2.1]hept-2-yl]methoxy]-N-hydroxy-N-methylpentanamide

Following the procedure of Example 65 except substituting cyclohexyl mesylate for hexyl mesylate, the title compound is obtained.

Example 116

(1α,2β,3β,4α)-5-[[3-[2-(Propyloxy)ethyl]-7-oxabicyclo[2.2.1]hept-2-yl]methoxy]-N-hydroxy-N-methylpentanamide

Following the procedure of Example 65 except substituting n-propyl mesylate for hexyl mesylate, the title compound is obtained.

Example 117

(1α,2β,3β,4α)-5-[[[3-[2-(Benzyloxy)ethyl]-7-oxabicyclo[2.2.1]hept-2-yl]methyl]thio]-N-hydroxy-N-methylpentanamide

Following the procedure of Example 66 except substituting benzyl mesylate for hexyl mesylate, the title compound is obtained.

Example 118

(1α,2β,3β,4α)-5-[[[3-[2-(Phenyloxy)ethyl]-7-oxabicyclo[2.2.1]hept-2-yl]methyl]thio]-N-hydroxy-N-methylpentanamide

Following the procedure of Example 77 except substituting the Example 68 Part A alcohol for the Example 58 Part F alcohol, the title compound is obtained.

Example 119

(1α,2β,3β,4α)-5-[[[3-[2-(Cyclohexyloxy)ethyl]-7-oxabicyclo[2.2.1]hept-2-yl]methyl]thio]-N-hydroxy-N-methylpentanamide

Following the procedure of Example 66 except substituting cyclohexyl mesylate for hexyl mesylate, the title compound is obtained.

Example 120

(1α,2β,3β,4α)-5-[[[3-[2-(Cyclopentylmethyloxy)ethyl]-7-oxabicyclo[2.2.1]hept-2-yl]methyl]thio]-N-hydroxy-N-methylpentanamide

Following the procedure of Example 66 except substituting cyclopentylmethyl mesylate for hexyl mesylate, the title compound is obtained.

Example 121

(1α,2β,3β,4α)-5-[[[3-[2-(2-Pentenyloxy)ethyl]-7-oxabicyclo[2.2.1]hept-2-yl]methyl]thio]-N-hydroxy-N-methylpentanamide

Following the procedure of Example 66 except substituting 2-pentenyl mesylate for hexyl mesylate, the title compound is obtained.

Example 122

(1α,2β,3β,4α)-5-[[3-[2-(Pentylthio)ethyl]-7-oxabicyclo[2.2.1]hept-2-yl]methoxy]-N-hydroxy-N-methylpentanamide

Following the procedure of Example 67 except substituting 1-pentanethiol for 1-hexanethiol, the title compound is obtained.

Example 123

(1α,2β,3β,4α)-5-[[3-[2-(Benzylthio)ethyl]-7-oxabicyclo[2.2.1]hept-2-yl]methoxy]-N-hydroxy-N-methylpentanamide

Following the procedure of Example 67 except substituting benzylthiol for 1-hexanethiol, the title compound is obtained.

Example 124

(1α,2β,3β,4α)-5-[[3-[2-(Phenylthio)ethyl]-7-oxabicyclo[2.2.1]hept-2-yl]methoxy]-N-hydroxy-N-methylpentanamide

Following the procedure of Example 67 except substituting phenylmercaptan for 1-hexanethiol, the title compound is obtained.

Example 125

(1α,2β,3β,4α)-5-[[3-[2-(Cyclohexylthio)ethyl-7-oxabicyclo[2.2.1]hept-2-yl]methoxy]-N-hydroxy-N-methylpentanamide Following the procedure of Example 67 except substituting cyclohexylmercaptan for 1-hexanethiol, the title compound is obtained.

Example 126

(1α,2β,3β,4α)-5-[[[3-[2-(Benzylthio)ethyl]-7-oxabicyclo[2.2.1]hept-2-yl]methyl]thio]-N-hydroxy-N-methylpentanamide

Following the procedure of Example 68 except substituting benzylmercaptan for 1-hexanethiol, the title compound is obtained.

Example 127

(1α,2β,3β,4α)-5-[[[3-[2-(Cycloheptylthio)ethyl]-7-oxabicyclo[2.2.1]hept-2-yl]methyl]thio]-N-hydroxy-N-methylpentanamide

Following the procedure of Example 68 except substituting cycloheptylmercaptan for 1-hexanethiol, the title compound is obtained.

Example 128

(1α,2β,3β,4α)-5-[[[3-[2-(Phenylthio)ethyl]-7-oxabicyclo[2.2.1]hept-2-yl]methyl]thio]-N-hydroxy-N-methylpentanamide

Following the procedure of Example 68 except substituting phenylmercaptan for 1-hexanethiol, the title compound is obtained.

Example 129

(1α,2β,3β,4α)-5-[[[3-[2-(Cyclohexylmethylthio)ethyl]-7-oxabicyclo[2.2.1]hept-2-yl]methyl]thio]-N-hydroxy-N-methylpentanamide

Following the procedure of Example 68 except substituting cyclohexylmethylmercaptan for 1-hexanethiol, the title compound is obtained.

Example 130

(1α,2β,3β,4α)-5-[[[3-[2-(2-Propenylthio)ethyl]-7-oxabicyclo[2.2.1]hept-2-yl]methyl]thio]-N-hydroxy-N-methylpentanamide

Following the procedure of Example 68 except substituting 1-(2-propenyl)thiol for 1-hexanethiol, the title compound is obtained.

Example 131

(1α,2β,3β,4α)-4-[2-[3-[2-(Heptyloxy)ethyl]-7-oxabicyclo[2.2.1]hept-2-yl]ethoxy]-N-hydroxy-N-methylbutanamide

Following the procedure of Example 69 except substituting heptyl mesylate for hexyl mesylate, the title compound is obtained.

Example 132

(1α,2β,3β,4α)-4-[2-[3-[2-(Benzyloxy)ethyl]-7-oxabicyclo[2.2.1]hept-2-yl]ethoxy]-N-hydroxy-N-methylbutanamide

Following the procedure of Example 69 except substituting benzyl mesylate for hexyl mesylate, the title compound is obtained.

Example 133

(1α,2β,3β,4α)-4-[2-[3-[2-(Phenyloxy)ethyl]-7-oxabicyclo[2.2-1]hept-2-yl]ethoxy]-N-hydroxy-N-methylbutanamide

Following the procedure of Example 69 except substituting phenyl mesylate for hexyl mesylate, the title compound is obtained.

Example 134

(1α,2β,3β,4α)-4-[2-[3-[2-(Cyclohexyloxy)ethyl]-7-oxabicyclo[2.2.1]hept-2-yl]ethoxy]-N-hydroxy-N-methylbutanamide

Following the procedure of Example 69 except substituting cyclohexyl mesylate for hexyl mesylate, the title compound is obtained.

Example 135

(1α,2β,3β,4α)-4-[2-[3-[2-(2-Cyclopentylethyloxy)ethyl]-7-oxabicyclo[2.2.1]hept-2-yl]ethoxy]-N-hydroxy-N-methylbutanamide

Following the procedure of Example 69 except substituting cyclopentylethyl mesylate for hexyl mesylate, the title compound is obtained.

Example 136

(1α,2β,3β,4α)-5-[[2-[3-[2-(Benzyloxy)ethyl]-7-oxabicyclo[2.2.1]hept-2-yl]ethyl]thio]-N-hydroxy-N-methylpentanamide

Following the procedure of Example 70 except substituting benzyl mesylate for hexyl mesylate, the title compound is obtained.

Example 137

(1α,2β,3β,4α)-5-[[2-[3-[2-(Phenyloxy)ethyl]-7-oxabicyclo[2.2.1]hept-2-yl]ethyl]thio]-N-hydroxy-N-methylpentanamide

Following the procedure of Example 77 except substituting the Example 70 Part C alcohol for the Example 56 Part F alcohol, the title compound is obtained.

Example 138

(1α,2β,3β,4α)-5-[[2-[3-[2-(Cyclopentyloxy)ethyl]-7-oxabicyclo[2.2.1]hept-2-yl]ethyl]thio]-N-hydroxy-N-methylpentanamide

Following the procedure of Example 70 except substituting cyclopentyl mesylate for hexyl mesylate, the title compound is obtained.

Example 139

(1α,2β,3β,4α)-4-[[2-[3-[2-(3-Hexenyloxy)ethyl]-7-oxabicyclo[2.2.1]hept-2-yl]ethyl]thio]-N-hydroxy-N-methylpentanamide

Following the procedure of Example 70 except substituting 3-hexenyl mesylate for hexyl mesylate, the title compound is obtained.

Example 140

(1α,2β,3β,4α)-4-[[2-[3-[2-(Cyclopropylmethyloxy)ethyl]-7-oxabicyclo[2.2.1]hept-2-yl]ethyl]thio]-N-hydroxy-N-methylpentanamide

Following the procedure of Example 70 except substituting cyclopropylmethyl mesylate for hexyl mesylate, the title compound is obtained.

Example 141

(1α,2β,3β,4α)-4-[2-[3-[2-(Benzylthio)ethyl]-7-oxabicyclo[2.2.1]hept-2-yl]ethoxy]-N-hydroxy-N-methylbutanamide

Following the procedure of Example 71 except substituting benzylmercaptan for 1-hexanethiol, the title compound is obtained.

Example 142

(1α,2β,3β,4α)-4-[2-[3-[2-(Phenylthio)ethyl]-7-oxabicyclo[2.2.1]hept-2-yl]ethoxy]-N-hydroxy-N-methylbutanamide

Following the procedure of Example 71 except substituting phenylmercaptan for 1-hexanethiol, the title compound is obtained.

Example 143

(1α,2β,3β,4α)-4-[2-[3-[2-(Cyclohexylthio)ethyl]-7-oxabicyclo[2.2.1]hept-2-yl]ethoxy]-N-hydroxy-N-methylbutanamide

Following the procedure of Example 71 except substituting cyclohexylmercaptan for 1-hexanethiol, the title compound is obtained.

Example 144

(1α,2β,3β,4α)-4-[2-[3-[2-(2-Heptenylthio)ethyl]-7-oxabicyclo[2.2.1]hept-2-yl]ethoxy]-N-hydroxy-N-methylbutanamide

Following the procedure of Example 71 except substituting 1-(2-heptenyl)thiol for 1-hexanethiol, the title compound is obtained.

Example 145

(1α,2β,3β,4α)-4-[2-[3-[2-(Cyclopentylmethylthio)ethyl]-7-oxabicyclo[2.2.1]hept-2-yl]ethoxy]-N-hydroxy-N-methylbutanamide

Following the procedure of Example 71 except substituting cyclopentylmethylmercaptan for 1-hexanethiol, the title compound is obtained.

Example 146

(1α,2β,3β,4α)-5-[[2-[3-[2-(Benzylthio)ethyl]-7-oxabicyclo[2.2.1]hept-2-yl]ethyl]thio]-N-hydroxy-N-methylpentanamide

Following the procedure of Example 72 except substituting benzylmercaptan for 1-hexanethiol, the title compound is obtained.

Example 147

(1α,2β,3β,4α)-5-[[2-[3-[2-(Phenylthio)ethyl]-7-oxabicyclo[2.2.1]hept-2-yl]ethyl]thio]-N-hydroxy-N-methylpentanamide

Following the procedure of Example 72 except substituting phenylmercaptan for 1-hexanethiol, the title compound is obtained.

Example 148

(1α,2β,3β,4α)-5-[[2-[3-[2-(Cyclohexylthio)ethyl]-7-oxabicyclo[2.2.1]hept-2-yl]ethyl]thio]-N-hydroxy-N-methylpentanamide

Following the procedure of Example 72 except substituting cyclohexylmercaptan for 1-hexanethiol, the title compound is obtained.

Example 149

(1α,2β,3β,4α)-5-[[2-[3-[2-(2-Hexenylthio)ethyl]-7-oxabicyclo[2.2.1]hept-2-yl]ethyl]thio]-N-hydroxy-N-methylpentanamide

Following the procedure of Example 72 except substituting 1-(2-hexenyl)thiol for 1-hexanethiol, the title compound is obtained.

Example 150

(1α,2β,3β,4α)-5-[[2-[3-[2-(Butylthio)ethyl]-7-oxabicyclo[2.2.1]hept-2-yl]ethyl]thio]-N-hydroxy-N-methylpentanamide

Following the procedure of Example 72 except substituting 1-butanethiol for 1-hexanethiol, the title compound is obtained.

Example 151

(1α,2β,3β,4α)-5-[[3-[4-(Hexyloxy)butyl]-7-oxabicyclo[2.2.1]hept-2-yl]methoxy]-N-hydroxy-N-methylpentanamide

A. (1α,2β,3β,4α)-5-[3-(3-Oxo)propyl]-7-oxabicyclo[2.2.1]hept-2-yl]methoxy]pentanoic acid, methyl ester

Following the procedure of Example 65 Part B except substituting (1α,2β,3β,4α)-5-[3-(2-oxo)ethyl-7-oxabicyclo[2.2.1]hept-2-yl]methoxy]pentanoic acid, methyl ester for (1α,2β,3β,4α)-7-[3-formyl-7-oxabicyclo-[2.2.1]hept-2-yl]methoxy]pentanoic acid, methyl ester, the title A compound is obtained.

B. (1α,2β,3β,4α)-5-[[3-(4-Oxo)butyl-7-oxabicyclo[2.2.1]hept-2-yl]methoxy]pentanoic acid, methyl ester

Following the procedure of Example 70 Part B except substituting the aldehyde from Part A above for (1α,2β,3β,4α)-5-[3-formyl-7-oxabicyclo[2.2.1]-hept-2-yl]methoxy]pentanoic acid, methyl ester, the title B aldehyde is obtained.

C. (1α,2β,3β,4α)-5-[[3-(4-Hydroxybutyl)-7-oxabicyclo[2.2.1]hept-2-yl]methoxy]pentanoic acid, methyl ester

Following the procedure of Example 65 Part C except substituting the title B aldehyde for (1α,2β,3β,4α)-5-[3-(2-oxo)ethyl-7-oxabicyclo-[2.2.1]hept-2-yl]methoxy]pentanoic acid, methyl ester, the title C alcohol is obtained.

D. (1α,2β,3β,4α)-5-[[3-[4-(Hexyloxy)butyl-7-oxabicyclo[2.2.1]hept-2-yl]methoxy]pentanoic acid

Following the procedure of Example 58 except substituting the above part C alcohol for the alcohol used in Example 58 Part G, the title compound is obtained.

E. (1α,2β,3β,4α)-5-[[3-[4-(Hexyloxy)butyl]-7-oxabicyclo[2.2.1]hept-2-yl]methoxy]-N-hydroxy-N-methylpentanamide

Following the procedure of Example 56 Part R except substituting the above Part D acid for the Example 56 Part Q acid, the title compound is obtained.

Example 152

(1α,2β,3β,4α)-5-[[[3-[4-(Benzyloxy)butyl]-7-oxabicyclo[2.2.1]hept-2-yl]methyl]thio]-N-hydroxy-N-methylpentanamide

Following the procedure of Examples 151 and 66 except substituting the Example 151 Part C alcohol for the Example 66 Part A alcohol and substituting benzyl mesylate for hexyl mesylate, the title compound is obtained.

Example 153

(1α,2β,3β,4α)-5-[[3-[4-(Cyclohexylthio)butyl]-7-oxabicyclo[2.2.1]hept-2-yl]methoxy]-N-hydroxy-N-methylpentanamide

Following the procedure of Examples 151 and 66 except substituting the Example 151 Part C alcohol for the Example 65 Part C alcohol and substituting cyclohexylmercaptan for 1-hexanethiol, the title compound is obtained.

Example 154

(1α,2β,3β,4α)-5-[[[3-[4-(Hexylthio)butyl]-7-oxabicyclo[2.2.1]hept-2-yl]methyl]thio]-N-hydroxy-N-methylpentanamide

A. (1α,2β,3β,4α)-5-[[[3-(3-Oxo)propyl-7-oxabicyclo[2.2.1]hept-2-yl]methyl]thio]pentanoic acid, methyl ester

Following the procedure of Example 68 Part B except substituting (1α,2β,3β,4α)-5-[[[3-(2-oxo)ethyl-7-oxabicyclo[2.2.1]hept-2-yl]methyl]thio]pentanoic acid, methyl ester for (1α,2β,3β,4α)-5-[[[3-formyl-7-oxabicyclo[2.2.1]hept-2-yl]methyl]thio]pentanoic acid, methyl ester, the title A compound is obtained.

B. (1α,2β,3β,4α)-5-[[[3-(4-Oxo)butyl-7-oxabicyclo[2.2.1]hept-2-yl]methyl]thio]pentanoic acid, methyl ester

Following the procedure of Example 68 Part B except substituting the aldehyde from Part A above for (1α,2β,3β,4α)-5-[[[3-formyl-7-oxabicyclo[2.2.1]hept-2-yl]methyl]thio]pentanoic acid, methyl ester, the title B aldehyde is obtained.

C. (1α,2β,3β,4α)-5-[[[3-(4-Hydroxybutyl)-7-oxabicyclo[2.2.1]hept-2-yl]methyl]thio]pentanoic acid, methyl ester

Following the procedure of Example 68 Part C except substituting the title B aldehyde for (1α,2β,3β,4α)-5-[[[3-(2-oxo)ethyl-7-oxabicyclo-[2.2.1]hept-2-yl]methyl]thio]pentanoic acid, methyl ester, the title C alcohol is obtained.

D. (1α,2β,3β,4α)-5-[[[3-[4-(Hexylthio)butyl]-7-oxabicyclo[2.2.1]hept-2-yl]methyl]thio]pentanoic acid

Following the procedure of Example 56A except substituting the above Part C alcohol for the alcohol used in Example 56A, the title compound is obtained.

E. (1α,2β,3β,4α)-5-[[[3-[4-(Hexylthio)butyl]-7-oxabicyclo[2.2.1]hept-2-yl]methyl]thio]-N-hydroxy-N-methylpentanamide

Following the procedure of Example 56 Part R except substituting the above Part D acid for the Example 56 Part Q acid, the title compound is obtained.

Example 155

(1α,2β,3β,4α)-4-[2-[3-[4-(Hexyloxy)butyl]-7-oxabicyclo[2.2.1]hept-2-yl]ethoxy]-N-hydroxy-N-methylbutanamide

Following the procedure of Examples 151 and 69 except substituting the Example 65 Part B aldehyde for the aldehyde used in Example 151 Part A, the title compound is obtained.

Example 156

(1α,2β,3β,4α)-5-[[[3-[(4-Hexylthio)butyl]-7-oxabicyclo[2-2-1]hept-2-yl]methyl]thio]-N-hydroxy-N-phenylpentanamide

Following the procedure of Example 56 Part R except substituting the Example 154 Part D acid for the Example 56 Part Q acid and N-phenylhydroxylamine for N-methylhydroxylamine, the title compound is obtained.

Example 157

(1α,2β,3β,4α)-5-[[[3-[(4-Hexylthio)butyl]-7-oxabicyclo[2.2.1]hept-2-yl]methyl]thio]-N-hydroxy-N-benzylpentanamide

Following the procedure of Example 56 Part R except substituting the Example 154 Part D acid for the Example 56 Part Q acid and N-benzylhydroxylamine for N-methylhydroxylamine, the title compound is obtained.

Example 158

(1α,2β,3β,4α)-5-[[[3-[(4-Hexylthio)butyl]-7-oxabicyclo[2.2.1]hept-2-yl]methyl]thio]-N-hydroxy-N-cyclohexylpentanamide

Following the procedure of Example 56 Part R except substituting the Example 154 Part D acid for the Example 56 Part Q acid and N-cyclohexylhydroxylamine for N-methylhydroxylamine, the title compound is obtained.

Example 159

(1α,2β,3β,4α)-5-[[2-[3-[4-(Hexyloxy)butyl]-7-oxabicyclo[2.2.1]hept-2-yl]ethyl]thio]-N-hydroxy-N-methylpentanamide

Following the procedure of Examples 151 and 70 except substituting the Example 70 Part B aldehyde for the aldehyde used in Example 151 Part A, the title compound is obtained.

Example 160

(1α,2β,3β,4α)-4-[[2-[3-[4-(Hexylthio)butyl]-7-oxabicyclo[2-2-1]hept-2-yl]ethyl]thio]-N-hydroxy-N-methylpentanamide

Following the procedure of Examples 154 and 72 except substituting the Example 72 Part A aldehyde for the aldehyde used in Example 154 Part A, the title compound is obtained.

Example 161

(1α,2β,3β,4α)-5-[[3-[(Hexylsulfinyl)methyl]-7-oxabicyclo[2.2.1]hept-2-yl]methoxy]-N-hydroxy-N-methylpentanamide

A. (1α,2β,3β,4α)-5-[[3-[(Hexylsulfinyl)methyl]-7-oxabicyclo[2.2.1]hept-2-yl]methoxy]pentanoic acid, methyl ester

and

(1α,2β,3β,4α)-5-[[3-(Hexylsulfonyl)methyl-7-oxabicyclo[2.2.1]hept-2-yl]methoxy]pentanoic acid, methyl ester

To a solution of 668 mg (1.72 mmol) of (1α,2β,3β,4α)-5-[[3-[(hexylthio)methyl]-7-oxabicyclo[2.2.1]hept-2-yl]methoxy]pentanoic acid, methyl ester (prepared as described in Example 59) in 6.78 ml of methanol at 0°C is added dropwise over 4 minutes 8.37 ml of 0.5M aqueous sodium periodate solution. Tetrahydrofuran (2 ml) is then added and the resulting reaction mixture is stirred at room temperature for 15 hours. A white precipitate is removed by filtration and washed with ether (3 x 50 ml). The filtrate is washed with 60 ml of a saturated aqueous $NaHCO_3$ solution and dried over anhydrous magnesium sulfate. Concentration in vacuo affords 648 mg of an oily crude product. This is chromatographed on 50 g of silica gel 60 using 0.5-1.0% $CH_3OH$ in ether to give the title compounds.

B. (1α,2β,3β,4α)-5-[[3-[(Hexylsulfonyl)methyl]-7-oxabicyclo[2.2.1]hept-2-yl]methoxy]pentanoic acid

To a stirred solution of 165 mg (0.39 mmole) of the Part A sulfonyl compound in 20.3 ml of THF and 3.09 ml of $H_2O$ under argon is added 3.90 ml of 1N aqueous lithium hydroxide solution. This mixture is purged with argon vigorously for 10 minutes and stirred at room temperature for 6 hours. The reaction mixture is acidified to pH 4 by addition of 1N aqueous HCl solution and poured into 30 ml of saturated NaCl solution. The resulting solution is saturated with solid NaCl and extracted with EtOAc (4 x 50 ml). The combined EtOAc extracts are dried ($MgSO_4$ andhydrous), filtered and concentrated in vacuo to give 165 mg of crude acid which is purified by flash chromatography to obtain 145 mg of pure acid.

C. (1α,2β,3β,4α)-5-[[3-[(Hexylsulfinyl)methyl]-7-oxabicyclo[2.2.1]hept-2-yl]methoxy]-N-hydroxy-N-methylpentanamide

Following the procedure of Example 56 Part R except substituting the Part B acid for the Example 56 Part Q acid, the title compound is obtained.

Example 162

(1α,2β,3β,4α)-5-[[3-[(Hexylsulfinyl)methyl]-7-oxabicyclo[2.2.1]hept-2-yl]methoxy]-N-hydroxy-N-methylpentanamide

To a stirred solution of 142 mg (0.35 mmol) of Example 161 ester and sulfinyl ester in 27.0 ml of THF and 4.11 ml of $H_2O$ under argon is added 5.19 ml of 1N aqueous lithium hydroxide solution. This mixture is purged with argon vigorously for 10 minutes and stirred at room temperature for 6 hours. The reaction mixture is acidified to pH 4 by addition of 1N aqueous HCl solution and poured into 50 ml of saturated NaCl solution. The resulting solution is saturated with solid NaCl and extracted with EtOAc (4 x 100 ml). The combined EtOAc extracts are dried ($MgSO_4$ anhydrous), filtered and concentrated in vacuo to give crude acid which is purified by flash chromatography.

The acid is then treated as described in Example 56 Part R to form the title compound.

Examples 163 to 172

Following the procedures as outlined in the specification and as described in the working Examples, the following compounds may be prepared.

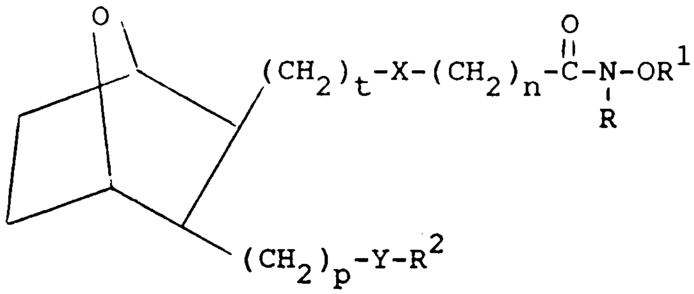

| Ex. No. | t | X | n | p | Y | $R^2$ | R | $R^1$ |
|---|---|---|---|---|---|---|---|---|
| 163. | 1 | O | 2 | 1 | S | $C_3H_7$ | H | $C_4H_9$ |
| 164. | 2 | S | 1 | 2 | S | $C_6H_5$ | $C_3H_7$ | H |
| 165. | 1 | S | 4 | 3 | O | $C_6H_4(CH_2)_2$- | $C_2H_5$ | $C_6H_5$ |
| 166. | 2 | O | 5 | 2 | O | 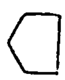 | H | 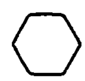 |
| 167. | 1 | O | 3 | 4 | $\underset{\underset{O}{\|}}{S}$ | 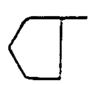 $CH_2$- | $C_4H_9$- 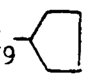 | $CH_3\overset{\overset{O}{\|}}{C}$ |
| 168. | 2 | S | 6 | 5 | O | 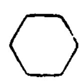 | $C_6H_5$ | $C_6H_5\overset{\overset{O}{\|}}{C}$ |
| 169. | 1 | $\underset{\underset{O}{\|}}{\overset{\overset{O}{\|}}{S}}$ | 7 | 3 | $\underset{\underset{O}{\|}}{\overset{\overset{O}{\|}}{S}}$ | $CH_2$=CH-$CH_2$- | 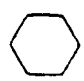 | 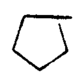 $CH_2$ |
| 170. | 2 | S | 8 | 2 | O | $CH_3CH_2CHCH_2$- | $C_3H_7$ | $C_2H_5$ |
| 171. | 1 | $\overset{\overset{O}{\|}}{S}$ | 6 | 1 | $\overset{\overset{O}{\|}}{S}$ | $C_6H_{13}$ | 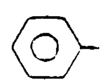-$CH_2$ | H |
| 172. | 2 | O | 5 | 2 | S | $C_4H_9$ | H | H |

## Claims

### Claims for the following Contracting States : BE, CH, DE, FR, GB, IT, LI, LU, NL, SE

**1.** Hydroxamic acids of the general formula I

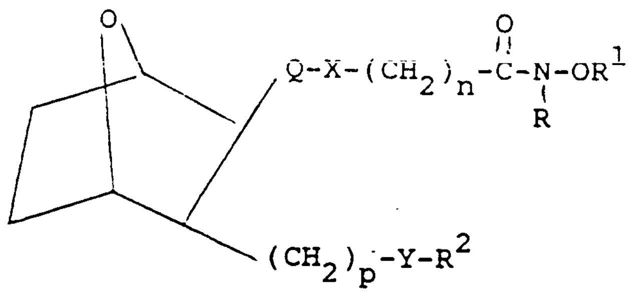

(I)

including all stereoisomers thereof, wherein Q is $-CH_2-A-(CH_2)_m-$ wherein A is -CH=CH- or $-(CH_2)_2-$, m is 1 to 6 wherein A is CH=CH and m is 0 to 6 wherein A is $(CH_2)_2$, or Q is $(CH_2)_t$ wherein t is 1 or 2; X is

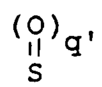

or O wherein q' is 0, 1 or 2; n is 1 to 8, R is H, lower alkyl, aryl, aralkyl or cycloalkyl; $R^1$ is H, lower alkyl, aryl, aralkyl, cycloalkyl, alkanoyl or aroyl; p is 1 to 5; Y is O or

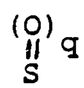

,

wherein q is 0, 1 or 2, and wherein q is 0, 1 or 2, when X is O and q is 0 (zero) when X is $S(O)_{q'}$; and $R^2$ is lower alkyl, aryl, arylalkyl, cycloalkyl, cycloalkylalkyl, lower alkenyl or lower alkynyl containing 2 to 12 carbons, wherein lower alkyl or alkyl alone or as part of another group contains 1 to 12 carbons and is unsubstituted or is substituted with halo, $CF_3$, alkoxy, aryl, alkyl-aryl, haloaryl, cycloalkyl, or alkycycloalkyl;

aryl alone or as part of another group is phenyl or naphthyl which is unsubstituted or is substituted with 1 or 2 lower alkyl groups, 1 or 2 halogens and/or 1 or 2 lower alkoxy groups;

cycloalkyl alone or as part of another group contains 3 to 12 carbons and is unsubstituted or is substituted with 1 or 2 halogens, 1 or 2 lower alkyl groups and/or 1 or 2 lower alkoxy groups; and

$(CH_2)_m$, $(CH_2)_n$ ns $(CH_2)_p$ may independently contain 1 or 2 lower alkyl and/or halo substituents.

**2.** The compound as defined in Claim 1 wherein X is O.

**3.** The compound as defined in Claim 1 wherein X is S.

**4.** The compound as defined in Claim 1 wherein p is 1.

**5.** The compound as defined in Claim 1 wherein Q is $CH_2-A-(CH_2)_m$.

**6.** The compound as defined in Claim 5 wherein A is CH=CH.

**7.** The compound as defined in Claim 5 wherein A is $(CH_2)_2$.

**8.** The compound as defined in Claim 7 wherein m is 0.

9. The compound as defined in Claim 1 wherein m is 1 to 3 and n is 1 to 3.

10. The compound as defined in Claim 1 wherein Y is O.

11. The compound as defined in Claim 1 wherein Y is S.

12. The compound as defined in Claim 6 wherein A is $CH_2$-$CH_2$ or CH = CH, m is 1 to 3, n is 1 to 3, X is O, $R^1$ is H, R is lower alkyl, p is 1, Y is O or S and $R^2$ is lower alkyl, phenyl or benzyl.

13. The compound as defined in Claim 1 wherein $R^2$ is butyl, pentyl, hexyl or heptyl including all isomers thereof.

14. The compound as defined in Claim 1 wherein Q is $(CH_2)_2$ and p is 1.

15. The compound as defined in Claim 1 wherein Q is $CH_2$ and p is 1.

16. The compound as defined in Claim 15 wherein n is 3 to 5.

17. The compound as defined in Claim 1 wherein Q is $CH_2$, X is O or S, Y is O, p is 1, n is 3 to 5, R is H or lower alkyl, $R^1$ is H or lower alkyl and $R^2$ is lower alkyl.

18. The compound as defined in Claim 1 having the name (1α,2β,3β,4α)-5-[[[3-[(hexyloxy)methyl]-7-oxabicyclo[2.2.1]hept-2-yl]methyl]thio]-N-hydroxy-N-methylpentanamide, including all stereoisomers thereof.

19. The compound as defined in Claim 1 having the name (1α,2β,3β,4α)-5-[[2-[3-[(hexyloxy)methyl]-7-oxabicyclo[2.2.1]hept-2-yl]ethyl]thio]-N-hydroxy-N-methyl-4-butanamide, including all stereoisomers thereof.

20. The compound as defined in Claim 1 having the name [1α,2β(2Z),3β,4α]-[[4-[3-[(hexyloxy)methyl-7-oxabicyclo[2.2.1]hept-2-yl]-2-butenyl]oxy]-N-hydroxy-N-methylacetamide including all stereoisomers thereof.

21. The compound as defined in Claim 1 having the name [1α,2β,3β,4α]-2-[[4-[3-[(hexylthio)methyl]-7-oxabicyclo[2.2.1]hept-2-yl]butyl]oxy]-N-hydroxy-N-methylacetamide including all stereoisomers thereof.

22. The compound as defined in Claim 1 having the name [1α,2β,3β,4α]-2-[[4-[3-[(hexyloxy)methyl]-7-oxabicyclo[2.2.1]hept-2-yl]butyl]oxy]-N-hydroxy-N,2,2-trimethylacetamide including all stereoisomers.

23. The compound as defined in Claim 1 having the name [1α,2β(2Z),3β,4α]-2-[[4-[3-(hexyloxy)methyl]-7-oxabicyclo[2.2.1]hept-2-yl]-2-butenyl]oxy]-N-hydroxy-N,2,2-trimethyl acetamide including all stereoisomers thereof.

24. A composition for inhibiting allergic conditions in a mammalian species, comprising an effective amount of a compound as defined in Claim 1 or a pharmaceutically acceptable salt thereof, and a pharmaceutically acceptable carrier thereof.

25. A compound as defined in Claim 1 for use for inhibiting $\Delta^5$-lipoxygenase.

26. A compound as defined in Claim 1 for use for treating asthma in a mammalian species.

27. A compound as defined in Claim 1 for use for inhibiting or reducing inflammation

28. A compound as defined in Claim 1 for use for inhibiting or treating psoriasis.

**Claim for the following Contracting States : ES, GR**

1. Process for preparing hydroxamic acids of the general formula I

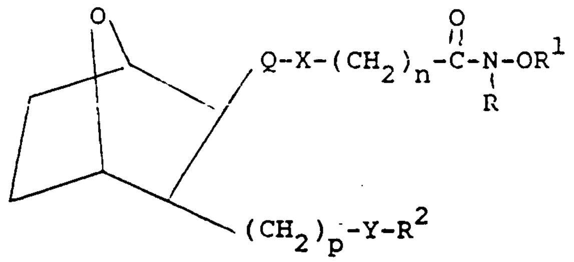

(I)

including all stereoisomers thereof, wherein Q is $-CH_2-A-(CH_2)_m-$ wherein A is -CH=CH- or $-(CH_2)_2-$, m is 1 to 6 wherein A is CH=CH and m is 0 to 6 wherein A is $(CH_2)_2$, or Q is $(CH_2)_t$ wherein t is 1 or 2; X is

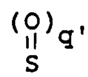

or O wherein q' is 0, 1 or 2; n is 1 to 8, R is H, lower alkyl, aryl, aralkyl or cycloalkyl; $R^1$ is H, lower alkyl, aryl, aralkyl, cycloalkyl, alkanoyl or aroyl; p is 1 to 5; Y is O or

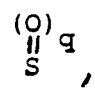

wherein q is 0, 1 or 2, and wherein q is 0, 1 or 2, when X is O and q is 0 (zero) when X is $S(O)_{q'}$; and $R^2$ is lower alkyl, aryl, arylalkyl, cycloalkyl, cycloalkylalkyl, lower alkenyl or lower alkynyl containing 2 to 12 carbons, wherein lower alkyl or alkyl alone or as part of another group contains 1 to 12 carbons and is unsubstituted or is substituted with halo, $CF_3$, alkoxy, aryl, alkyl-aryl, haloaryl, cycloalkyl, or alkycycloalkyl;

aryl alone or as part of another group is phenyl or naphtyl which is unsubstituted or is substituted with 1 or 2 lower alkyl groups, 1 or 2 halogens and/or 1 or 2 lower alkoxy groups;

cycloalkyl alone or as part of another group contains 3 to 12 carbons and is unsubstituted or is substituted with 1 or 2 halogens, 1 or 2 lower alkyl groups and/or 1 or 2 lower alkoxy groups; and

$(CH_2)_m$, $(CH_2)_n$ and $(CH_2)_p$ may be independently contain 1 or 2 lower alkyl and/or halo subsitutents characterized in that an acid of the general formula

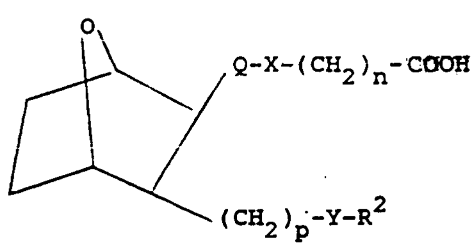

wherein Q, X, Y, $R^2$, n and p have the above meaning is converted to the corresponding hydroxamic acid.

## Revendications

**Revendications pour les Etats contractants suivants : BE, CH, DE, FR, GB, IT, LI, LU, NL, SE**

**1.** Acides hydroxamiques de formule générale I

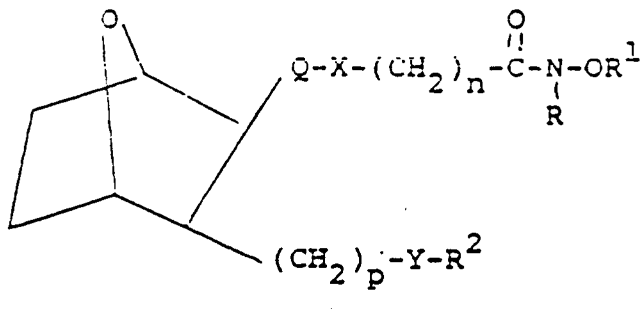

(I)

y compris tous leurs stéréo-isomères, formule dans laquelle Q est $-CH_2-A-(CH_2)_m-$ où A est -CH = CH- ou $-(CH_2)_2-$, m est un nombre de 1 à 6 lorsque A est CH = CH et m est un nombre de 0 à 6 lorsque A est $(CH_2)_2$, ou bien Q est $(CH_2)_t$ où t est égal à 1 ou 2;

X est 0 ou

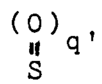

où q' est égal à 0, 1 ou 2; n est un nombre de 1 à 8, R est H ou un radical alkyle inférieur, aryle, aralkyle ou cycloalkyle; $R^1$ est H ou un radical alkyle inférieur , aryle, aralkyle, cycloalkyle, alcanoyle ou aroyle; p est un nombre de 1 à 5; Y est O ou

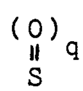

où q est égal à 0, 1 ou 2, q étant égal à 0, 1 ou 2 lorsque X est O et q étant égal à 0 (zéro) lorsque X est $S(O)_{q'}$;et $R^2$ est un radical alkyle inférieur, aryle, arylalkyle, cycloalkyle, cycloalkylalkyle, alcényle inférieur ou alcynyle inférieur contenant 2 à 12 atomes de carbone,

le radical alkyle inférieur, ou alkyle, seul ou en tant que partie d'un autre groupement, contenant 1 à 12 atomes de carbone et n'étant pas substitué, ou étant substitué par halo, $CF_3$, alcoxy, aryle, alkylaryle, haloaryle, cycloalkyle ou alkylcycloalkyle;

le radical aryle, seul ou en tant que partie d'un autre groupement, étant un radical phényle ou naphtyle qui n'est pas substitué ou qui est substitué par 1 ou 2 radicaux alkyle inférieur, 1 ou 2 atomes d'halogène et/ou 1 ou 2 radicaux alcoxy inférieur;

le radical cycloalkyle, seul ou en tant que partie d'un autre groupement, contenant 3 à 12 atomes de carbone et n'étant pas substitué ou étant substitué par 1 ou 2 atomes d'halogène, 1 ou 2 radicaux alkyle inférieur et/ou 1 ou 2 radicaux alcoxy inférieur; et

$(CH_2)_m$, $(CH_2)_n$ et $(CH_2)_p$ peuvent porter, indépendamment, 1 ou 2 substituants alkyle inférieur et/ou halogène.

**2.** Composé selon la revendication 1, dans la formule duquel X est O.

**3.** Composé selon la revendication 1, dans la formule duquel X est S.

**4.** Composé selon la revendication 1, dans la formule duquel p est égal à 1.

**5.** Composé selon la revendication 1, dans la formule duquel Q est $CH_2-A-(CH_2)_m$.

6. Composé selon la revendication 5, dans la formule duquel A est CH = CH.

7. Composé selon la revendication 5, dans la formule duquel A est $(CH_2)_2$.

8. Composé selon la revendication 7, dans la formule duquel m est égal à 0.

9. Composé selon la revendication 1, dans la formule duquel m est un nombre de 1 à 3 et n est un nombre de 1 à 3.

10. Composé selon la revendication 1, dans la formule duquel Y est O.

11. Composé selon la revendication 1, dans la formule duquel Y est S.

12. Composé selon la revendication 6, dans la formule duquel A est $CH_2$-$CH_2$ ou CH = CH, m est un nombre de 1 à 3, n est un nombre de 1 à 3, X est O, $R^1$ est H, R est un radical alkyle inférieur, p est égal à 1, Y est O ou S et $R^2$ est un radical alkyle inférieur, phényle ou benzyle.

13. Composé selon la revendication 1, dans la formule duquel $R^2$ est un radical butyle, pentyle, hexyle ou heptyle, y compris tous ses isomères.

14. Composé selon la revendication 1, dans la formule duquel Q est $(CH_2)_2$ et p est égal à 1.

15. Composé selon la revendication 1, dans la formule duquel Q est $CH_2$ et p est égal à 1.

16. Composé selon la revendication 15, dans la formule duquel n est un nombre de 3 à 5.

17. Composé selon la revendication 1, dans la formule duquel Q est $CH_2$, X est O ou S, Y est O, p est égal à 1, n est un nombre de 3 à 5, R est H ou un radical alkyle inférieur, $R^1$ est H ou un radical alkyle inférieur et $R^2$ est un radical alkyle inférieur.

18. Composé selon la revendication 1, qui est le (1α,2β,3β,4α)-5-[[[3-[(hexyloxy) méthyl]-7-oxabicyclo-[2.2.1]hept-2-yl]méthyl]thio]-N-hydroxy-N-méthylpentanamide, y compris tous ses stéréo-isomères.

19. Composé selon la revendication 1, qui est le (1α,2β,3β,4α)-5-[[2-[3-[(hexyloxy)-méthyl]-7-oxabicyclo-[2.2.1]hept-2-yl]éthyl]thio]-N-hydroxy-N-méthyl-4-butanamide, y compris tous ses stéréo-isomères.

20. Composé selon la revendication 1, qui est le [1α,2β(2Z),3β,4α]-[[4-[3-[(hexyloxy)méthyl-7-oxabicyclo-[2.2.1]hept-2-yl]-2-butényl]oxy]-N-hydroxy-N-méthylacétamide, y compris tous ses stéréo-isomères.

21. Composé selon la revendication 1, qui est le [1α,2β,3β,4α]-2-[[4-[3-[(hexylthio)méthyl]-7-oxabicyclo-[2.2.1]-hept-2-yl]butyl]oxy]-N-hydroxy-N-méthylacétamide, y compris tous ses stéréo-isomères.

22. Composé selon la revendication 1, qui est le [1α,2β,3β,4α]-2-[[4-[3-[(hexyloxy)méthyl]-7-oxabicyclo-[2.2.1]hept-2-yl]butyl]oxy]-N-hydroxy-N,2,2 -triméthylacétamide, y compris tous ses stéréo-isomères.

23. Composé selon la revendication 1, qui est le [1α,2β(2Z),3β,4α]-2-[[4-[3-(hexyloxy)méthyl]-7-oxabicyclo-[2.2.1]hept-2-yl]-2-buténykl]oxy]-N-hydroxy-N,2,2-triméthylacétamide, y compris tous ses stéréo-isomères.

24. Composition permettant d'inhiber les états allergiques chez une espèce mammifère, comprenant une quantité efficace d'un composé selon la revendication 1 ou d'un sel pharmaceutiquement acceptable de celui-ci, et un porteur pharmaceutiquement acceptable.

25. Composé selon la revendication 1, servant à inhiber la $\Delta^5$-lipoxygénase.

26. Composé selon la revendication 1, servant à traiter l'asthme chez une espèce mammifère.

27. Composé selon la revendication 1, servant à inhiber ou réduire l'inflammation.

**28.** Composé selon la revendication 1, servant à inhiber ou à traiter le psoriasis.

**Revendication pour les Etats contractants suivants : ES, GR**

**1.** Procédé de préparation d'acides hydroxamiques de formule générale I

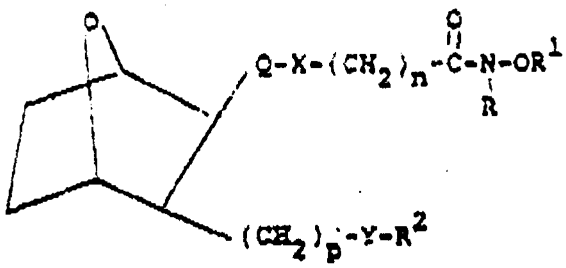

(I)

y compris tous leurs stéréo-isomères, formule dans laquelle Q est $-CH_2-A-(CH_2)_m-$ où A est -CH=CH- ou $-(CH_2)_2$, m est un nombre de 1 à 6 lorsque A est CH=CH et m est un nombre de 0 à 6 lorsque A est $(CH_2)_2$, ou bien Q est $(CH_2)_t$ où t est égal à 1 ou 2;

X est O ou

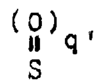

où q' est égal à 0, 1 ou 2; n est un nombre de 1 à 8, R est H ou un radical alkyle inférieur, aryle, aralkyle ou cycloalkyle; $R^1$ est H ou un radical alkyle inférieur, aryle, aralkyle, cycloalkyle, alcanoyle ou aroyle; p est un nombre de 1 à 5; Y est O ou

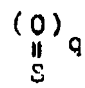

où q est égal à 0, 1 ou 2, q étant égal à 0, 1 ou 2 lorsque X est O et q étant égal à 0 (zéro) lorsque X est $S(O)_q$; et $R^2$ est un radical alkyle inférieur, aryle, arylalkyle, cycloalkyle, cycloalkylalkyle, alcényle inférieur ou alcynyle inférieur contenant 2 à 12 atomes de carbone,

le radical alkyle inférieur, ou alkyle, seul ou en tant que partie d'un autre groupement, contenant 1 à 12 atomes de carbone et n'étant pas substitué, ou étant substitué par halo, $CF_3$, alcoxy, aryle, alkylaryle, haloaryle, cycloalkyle ou alkylcycloalkyle;

le radical aryle, seul ou en tant que partie d'un autre groupement, étant un radical phényle ou naphtyle qui n'est pas substitué ou qui est substitué par 1 ou 2 radicaux alkyle inférieur, 1 ou 2 atomes d'halogène et/ou 1 où 2 radicaux alcoxy inférieur;

le radical cycloalkyle, seul ou en tant que partie d'un autre groupement, contenant 3 à 12 atomes de carbone et n'étant pas substitué ou étant substitué par 1 ou 2 atomes d'halogène, 1 ou 2 radicaux alkyle inférieur et/ou 1 ou 2 radicaux alcoxy inférieur; et

$(CH_2)_m$, $(CH_2)_n$ et $(CH_2)_p$ peuvent porter, indépendamment, 1 ou 2 substituants alkyle inférieur et/ou halogène,

caractérisé en ce qu'on transforme un acide de formule générale

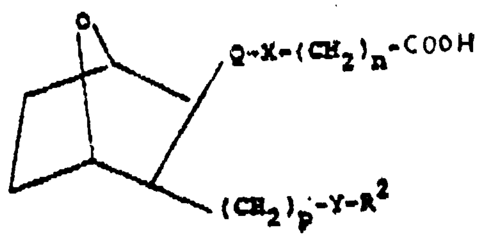

dans laquelle Q, X, Y, $R^2$, n et p ont les significations précédentes, en l'acide hydroxamique correspondant.

## Patentansprüche

### Patentansprüche für folgende Vertragsstaaten : BE, CH, DE, FR, GB, IT LI, LU, NL, SE

**1.** Hydroxamsäuren der allgemeinen Formel I

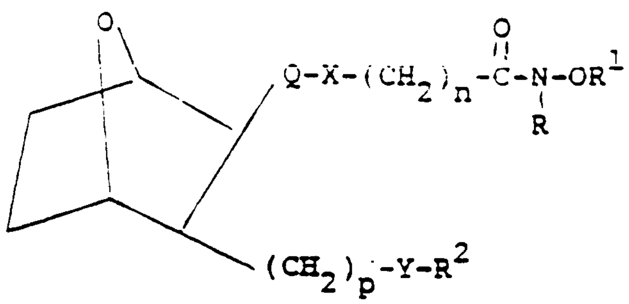

(I)

einschließlich aller Stereoisomeren davon, in der Q ein Rest der Formel $-CH_2-A-(CH_2)_m-$, wobei A eine der Gruppen -CH=CH- oder $-(CH_2)_2-$ ist, m einen Wert von 1 bis 6 aufweist, wobei A die Gruppe CH=CH ist und m einen Wert von 0 bis 6 aufweist, wobei A die Gruppe $(CH_2)_2$ ist oder Q ein Rest der Formel $(CH_2)_t$ ist, wobei t den Wert 1 oder 2 aufweist; X ein Rest der Formel $S=(O)_{q'}$ oder ein O-Atom ist, wobei q' den Wert 0, 1 oder 2 aufweist; n einen Wert von 1 bis 8 aufweist; R ein H-Atom, ein Niederalkyl-, Aryl-, Aralkyl- oder Cycloalkylrest ist; $R^1$ ein H-Atom, ein Niederalkyl-, Aryl-, Aralkyl-, Cycloalkyl-, Alkanoyl- oder Aroylrest ist; p einen Wert von 1 bis 5 aufweist; Y ein O-Atom oder ein Rest der Formel $S=(O)_q$ ist, wobei q den Wert 0, 1 oder 2 aufweist und q, wenn X ein O-Atom ist, den Wert 0, 1 oder 2 aufweist und q, wenn X ein Rest der Formel $S(O)_{q'}$ ist, den Wert 0 aufweist; und $R^2$ ein Niederalkyl-, Aryl-, Arylalkyl-, Cycloalkyl-, Cycloalkylalkyl-, Niederalkenyl- oder Niederalkynylrest mit 2 bis 12 Kohlenstoffatomen ist, wobei der Niederalkyl- oder Alkylrest allein oder als Teil einer anderer Gruppe 1 bis 12 Kohlenstoffatome enthält und unsubstituiert oder mit Halogenatomen, $CF_3$-Gruppen, Alkoxy-, Aryl-, Alkylaryl-, Haloaryl-, Cycloalkyl- oder Alkylcycloalkylresten substituiert ist; der Arylrest allein oder als Teil einer anderer Gruppe eine Phenyl- oder Naphthylgruppe ist, die unsubstituiert oder mit 1 oder 2 Niederalkylresten, 1 oder 2 Halogenatomen und/oder 1 oder 2 Niederalkoxyresten substituiert ist; der Cycloalkylrest allein oder als Teil einer anderen Gruppe 3 bis 12 Kohlenstoffatome enthält und unsubstituiert oder mit 1 oder 2 Halogenatomen, 1 oder 2 Niederalkylresten und/oder 1 oder 2 Niederalkoxyresten substituiert ist, und die Reste der Formeln $(CH_2)_m$, $(CH_2)_n$ und $(CH_2)_p$ unabhängig voneinander 1 oder 2 Niederalkyl- und/oder Halogensubstituenten enthalten können.

**2.** Verbindung nach Anspruch 1, in der X ein O-Atom ist.

**3.** Verbindung nach Anspruch 1, in der X ein S-Atom ist.

**4.** Verbindung nach Anspruch 1, in der p den Wert 1 aufweist.

**5.** Verbindung nach Anspruch 1, in der Q ein Rest der Formel $CH_2-A-(CH_2)_m$ ist.

6. Verbindung nach Anspruch 5, in der A die Gruppe CH = CH ist.

7. Verbindung nach Anspruch 5, in der A die Gruppe $(CH_2)_2$ ist.

8. Verbindung nach Anspruch 7, in der m den Wert 0 aufweist.

9. Verbindung nach Anspruch 1, in der m einen Wert von 1 bis 3 und n einen Wert von 1 bis 3 aufweisen.

10. Verbindung nach Anspruch 1, in der Y ein O-Atom ist.

11. Verbindung nach Anspruch 1, in der Y ein S-Atom ist.

12. Verbindung nach Anspruch 6, in der A eine der Gruppen $CH_2$-$CH_2$ oder CH = CH ist, m einen Wert von 1 bis 3 aufweist, n einen Wert von 1 bis 3 aufweist, X ein O-Atom ist, $R^1$ ein H-Atom ist, R ein Niederalkylrest ist, p den Wert 1 aufweist, Y ein O- oder S-Atom ist und $R^2$ ein Niederalkylrest, eine Phenyl- oder Benzylgruppe ist.

13. Verbindung nach Anspruch 1, in der $R^2$ eine Butyl-, Pentyl-, Hexyl- oder Heptylgruppe ist, einschließlich aller Isomeren davon.

14. Verbindung nach Anspruch 1, in der Q die Gruppe $(CH_2)_2$ ist und p den Wert 1 aufweist.

15. Verbindung nach Anspruch 1, in der Q die Gruppe $CH_2$ ist und p den Wert 1 aufweist.

16. Verbindung nach Anspruch 15, in der n einen Wert von 3 bis 5 aufweist.

17. Verbindung nach Anspruch 1, in der Q die Gruppe $CH_2$ ist, X ein O- oder S-Atom ist, Y ein O-Atom ist, p den Wert 1 aufweist, n einen Wert von 3 bis 5 aufweist, R ein H-Atom oder Niederalkylrest ist, $R^1$ ein H-Atom oder ein Niederalkylrest ist und $R^2$ ein Niederalkylrest ist.

18. Verbindung nach Anspruch 1 mit der Bezeichnung (1α,2β,3β,4α)-5-[[[3-[(Hexyloxy)-methyl]-7-oxabicyclo [2.2.1]hept-2-yl]methyl]thio]-N-hydroxy-N-methylpentanamid einschließlich aller Stereoisomeren davon.

19. Verbindung nach Anspruch 1 mit der Bezeichnung (1α,2β,3β,4α)-5-[[2-[3-[(Hexyloxy)-methyl]-7-oxabicyclo [2.2.1]hept-2-yl]ethyl]thio]-N-hydroxy-N-methyl-4-butanamid einschließlich aller Stereoisomeren davon.

20. Verbindung nach Anspruch 1 mit der Bezeichnung [1α,2β(2Z),3β,4α]-[[4-[3-[(Hexyloxy)-methyl-7-oxabicyclo [2.2.1]hept-2-yl]-2-butenyl]oxy]-N-hydroxy-N-methylacetamid einschließlich aller Stereoisomeren davon.

21. Verbindung nach Anspruch 1 mit der Bezeichnung [1α,2β,3β,4α]-2-[[4-[3-[(Hexylthio)-methyl]-7-oxabicyclo [2.2.1]hept-2-yl]butyl]oxy]-N-hydroxy-N-methylacetamid, einschließlich aller Stereoisomeren davon.

22. Verbindung nach Anspruch 1 mit der Bezeichnung [1α,2β,3β,4α]-2-[[4-[3-[(Hexyloxy)-methyl]-7-oxabicyclo [2.2.1]hept-2-yl]butyl]oxy]-N-hydroxy-N,2,2-trimethylacetamid einschließlich aller Stereoisomeren davon.

23. Verbindung nach Anspruch 1 mit der Bezeichnung [1α,2β(2Z),3β,4α]-2-[[4-[3-[(Hexyloxy)-methyl]-7-oxabicyclo[2.2.1]hept-2-yl]-2-butenyl]oxy]-N-hydroxy-N,2,2-trimethylacetamid einschließlich aller Stereoisomeren davon.

24. Zusammensetzung zur Hemmung allergischer Erkrankungen bei einem Säuger, die eine wirksame Menge einer Verbindung nach Anspruch 1 oder ein pharmazeutisch verträgliches Salz davon und einen pharmazeutisch verträglichen Träger davon umfaßt.

25. Verbindung nach Anspruch 1 zur Verwendung für die Hemmung der $\Delta^5$-Lipoxygenase.

26. Verbindung nach Anspruch 1 zur Verwendung für die Behandlung von Asthma bei einem Säuger.

27. Verbindung nach Anspruch 1 zur Verwendung für die Hemmung oder Eindämmung von Entzündungen.

28. Verbindung nach Anspruch 1 zur Verwendung für die Hemmung oder Behandlung von Schuppenflechte.

**Patentanspruch für folgende Vertragsstaaten : ES, GR**

1. Verfahren zur Herstellung von Hydroxamsäuren der allgemeinen Formel I

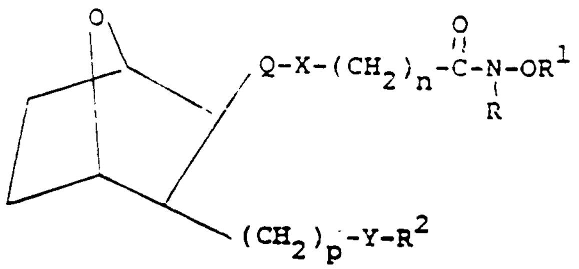

(I)

einschließlich aller Stereoisomeren davon, in der Q ein Rest der Formel $-CH_2-A-(CH_2)_m-$, wobei A eine der Gruppen $-CH=CH-$ oder $-(CH_2)_2-$ ist, m einen Wert von 1 bis 6 aufweist, wobei A die Gruppe $CH=CH$ ist und m einen Wert von 0 bis 6 aufweist, wobei A die Gruppe $(CH_2)_2$ ist oder Q ein Rest der Formel $(CH_2)_t$ ist, wobei t den Wert 1 oder 2 aufweist; X ein Rest der Formel $S=(O)_{q'}$ oder ein O-Atom ist, wobei q' den Wert 0, 1 oder 2 aufweist; n einen Wert von 1 bis 8 aufweist; R ein H-Atom, ein Niederalkyl-, Aryl-, Aralkyl- oder Cycloalkylrest ist; $R^1$ ein H-Atom, ein Niederalkyl-, Aryl-, Aralkyl-, Cycloalkyl-, Alkanoyl- oder Aroylrest ist; p einen Wert von 1 bis 5 aufweist; Y ein O-Atom oder ein Rest der Formel $S=(O)_q$ ist, wobei q den Wert 0, 1 oder 2 aufweist und q, wenn X ein O-Atom ist, den Wert 0, 1 oder 2 aufweist und q, wenn X ein Rest der Formel $S(O)_{q'}$ ist, den Wert 0 aufweist; und $R^2$ ein Niederalkyl-, Aryl-, Arylalkyl-, Cycloalkyl-, Cycloalkylalkyl-, Niederalkenyl- oder Niederalkynylrest mit 2 bis 12 Kohlenstoffatomen ist, wobei der Niederalkyl- oder Alkylrest allein oder als Teil einer anderer Gruppe 1 bis 12 Kohlenstoffatome enthält und unsubstituiert oder mit Halogenatomen, $CF_3$-Gruppen, Alkoxy-, Aryl-, Alkylaryl-, Haloaryl-, Cycloalkyl- oder Alkylcycloalkylresten substituiert ist; der Arylrest allein oder als Teil einer anderer Gruppe eine Phenyl- oder Naphthylgruppe ist, die unsubstituiert oder mit 1 oder 2 Niederalkylresten, 1 oder 2 Halogenatomen und/oder 1 oder 2 Niederalkoxyresten substituiert ist; der Cycloalkylrest allein oder als Teil einer anderen Gruppe 3 bis 12 Kohlenstoffatome enthält und unsubstituiert oder mit 1 oder 2 Halogenatomen, 1 oder 2 Niederalkylresten und/oder 1 oder 2 Niederalkoxyresten substituiert ist, und die Reste der Formeln $(CH_2)_m$, $(CH_2)_n$ und $(CH_2)_p$ unabhängig voneinander 1 oder 2 Niederalkyl- und/oder Halogensubstituenten enthalten können. dadurch gekennzeichnet, daß eine Säure der allgemeinen Formel

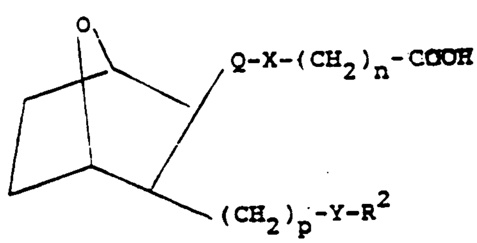

in der Q, X, Y, $R^2$, n und p die vorstehende Bedeutung haben, in die korrespondierende Hydroxamsäure überführt wird.